# EUROPEAN PATENT APPLICATION

(11) **EP 4 338 745 A1**
(43) Date of publication of application: **20.03.2024**
(21) Application number: 22195618.8
(22) Date of filing: 14.09.2022
(51) Int. Cl.: A61K 35/15, C07K 14/74, C12N 5/078, A61P 35/00

(54) **ALLOGENEIC HUMAN MACROPHAGES FOR CELL THERAPY**

(71) Applicant: Technische Universität Dresden, 01069 Dresden (DE)
(72) Inventor: ENGLMEIER, Ludwig, 83080 Oberaudorf (DE); SIEWEKE, Michael, 01069 Dresden (DE)
(74) Representative: Maikowski & Ninnemann Patentanwälte Partnerschaft mbB

(57) **Abstract**

The present invention relates to an allogeneic myeloid cell, such as a macrophage, for use in therapy, such as the treatment of cancer in a human patient, wherein the allogeneic myeloid cell, such as the allogeneic macrophage, comprises at least one MHC-II complex which is identical with any one MHC-II complex of said human patient. This has the advantage that an antigen-specific response of a CD4+ T-cell of said human patient, for example against a tumor neoantigen, can be triggered. The invention also relates to an allogeneic myeloid cell, such as an allogeneic macrophage or allogeneic monocyte, for use in the treatment of cancer in a human patient, wherein the allogeneic macrophage or allogeneic mononcyte comprises at least one protein complex selected from the group consisting of HLA-DR, HLA-DP and HLA-DQ, which protein complex is identical with any corresponding HLA complex of said human patient, preferably wherein the allogeneic macrophage comprises two protein complexes selected from said groups or even wherein the allogeneic macrophages comprises three complexes selected from said group.

## Description

### BACKGROUND OF THE INVENTION

Macrophages are plastic cells, and their phenotype depends on the environmental stimuli they encounter. When macrophages are exposed to bacterial components or inflammatory cytokines such as IFNγ, macrophages enhance their microbicidal and tumoricidal capacity and produce high levels of proinflammatory cytokines; this activation state has been called M1 or classically activated macrophages (O'Shea and Murray, 2008; Gordon, 2003). In contrast, when macrophages are stimulated with immunosuppressive cytokines such as IL-13, IL-4 or M-CSF, they exhibit an anti-inflammatory phenotype, typically including production of IL-10 and promoting tissue remodeling and repair as well as angiogenesis (Murray and Wynn, 2011; Mantovani et al., 2002); this activation state has been called M2 or alternatively activated macrophages.

Interestingly, macrophages are the most abundant cells in the tumor microenvironment. Clinical and experimental evidences showed that tumor associated macrophages (TAMs) promote tumor progression and malignancy (Mantovani et al., 1992; Condeelis and Pollard, 2006; Mantovani et al., 2006), and their presence is correlated with poor survival (Qian and Pollard, 2010). Indeed, TAMs support cancer development by promoting tumor angiogenesis, tumor cell invasion and metastasis and by suppressing anti-tumor immune response (Condeelis and Pollard, 2006; Pollard, 2004). It has been reported that several cancers and their cellular infiltrates provide a cytokine cocktail in the milieu which leads to an M2 macrophage polarization (Nevala et al., 2009). Thus, TAMs apparently resemble an M2 phenotype (Balkwill et al., 2005).

Bart et al., 2021, review the approaches towards macrophage reprogramming in various disorders and discuss the potential implications and challenges for macrophage-targeted approaches in human disease. In particular, the various attempts to re-program tumor associated macrophages away from the M2-like phenotype by way of small molecules and cytokines, nanovectors, antibodies, nucleic acids or viral vectors are discussed.

Macrophages which have been polarized towards an M1-phenotype prior to administration have been tested for the treatment for diseases such as cancer. However, the M1-phenotype is not stable and the administered macrophages can get re-polarized by the tumor microenvironment to an M2 like phenotype.

We have described highly desirable human macrophages which can resist tumor-induced repolarization in patent application PCT/EP2022/056819.

Understanding the mechanistic features for optimal activity of myeloid cells would be highly desirable for a most efficacious medical use of M1-polarized myeloid cells, such as M1 macrophages.

### SUMMARY OF THE INVENTION

The present invention provides an insight into the features of M1-polarized myeloid cells that allow their optimal antitumoral activity. We have observed in the mouse that lymphoid cells, in particular T-cells, and/or NK cells are involved in an aspect of the antitumor activity of myeloid cells, such as macrophages. We have also observed a dramatic upregulation of HLA class II genes in the myeloid cells which are resistant to tumor-induced repolarization and which have antitumor activity. Based on these observations, but without wishing to be bound by any theory, we suggest that optimal antitumor activity involves the interaction of myeloid cells, such as macrophages, with T-cells, such as CD4+ T-cells, via the interaction of the MHC II complex on myeloid cells, such as macrophages, with the T-cell receptor on T-cells. This has important implications for improved anticancer cell therapies with allogeneic macrophages, monocytes or dendritic cells, and in particular anticancer cell therapies with macrophages strictu sensu.

Because of MHC restriction those T-cells, which would be specific for tumor-derived neoantigens, should respond much better to a tumor-derived peptide-antigen bound to a matched MHC II complex than to the same tumor-derived peptide-antigen bound to an unmatched MHC II complex. It is therefore important to consider that the allogeneic myeloid cell, such as the allogeneic macrophages, carries at least one version of an HLA-DP, HLA-DQ and/or HLA-DR complex, which is also present in the CD4 T cells of the individual to be treated. In other words, there should be at least a partial match between the patient's lymphoid immune cells and the allogeneic macrophages at the level of the MHC-II complex, so that, for example, the antigen-specific recognition of a tumor-derived neoantigen by a neoantigen-specific patient's CD4+ T-cell is significantly improved when said neoantigen is presented on an allogeneic antigen presenting cell, such as a macrophage.

Thus, the present invention relates to an allogeneic myeloid cell, such as a macrophage, for use in therapy, such as the treatment of cancer in a human patient, wherein the allogeneic myeloid cell, such as the allogeneic macrophage, comprises at least one MHC-II complex which is identical with any one MHC-II complex of said human patient. This has the advantage that an antigen-specific response of a CD4+ T-cell of said human patient, for example against a tumor neoantigen, can be triggered. The invention also relates to an allogeneic myeloid cell, such as an allogeneic macrophage or allogeneic monocyte, for use in the treatment of cancer in a human patient, wherein the allogeneic macrophage or allogeneic mononcyte comprises at least one protein complex selected from the group consisting of HLA-DR, HLA-DP and HLA-DQ, which protein complex is identical with any corresponding HLA complex of said human patient, preferably wherein the allogeneic macrophage comprises two protein complexes selected from said groups or even wherein the allogeneic macrophages comprises three complexes selected from said group.

The partial, and preferably complete, MHC II match between the allogeneic myeloid cell, such as a macrophage, and the patient to be treated can also be described at the genetic level. The present invention therefore also relates to an allogeneic myeloid cell, such as a macrophage, for use in the treatment of cancer in a patient, wherein the allogeneic myeloid cell and the patient to be treated have at least one pair of alleles in common selected from the group consisting of the following pairs: HLA-DRA/ HLA-DRB1, HLA-DRA/ HLA-DRB3, HLA-DRA/ HLA-DRB4, HLA-DRA/ HLA-DRB5, HLA-DPA1 / HLA-DPB1 and HLA-DQA1 /HLA-DQB1, preferably wherein the allogeneic myeloid cell and the patient to be treated have two, three, four, five or even all six pairs of alleles in common. It should be noted that for heterozygous patients this means an overlap of one allele pair with one allele pair of the allogeneic myeloid cell, but not necessarily a match of both allele pairs of the patient with both allele pairs of the allogeneic myeloid cell (but such a match of both allele pairs is, of course, included).

### FIGURES

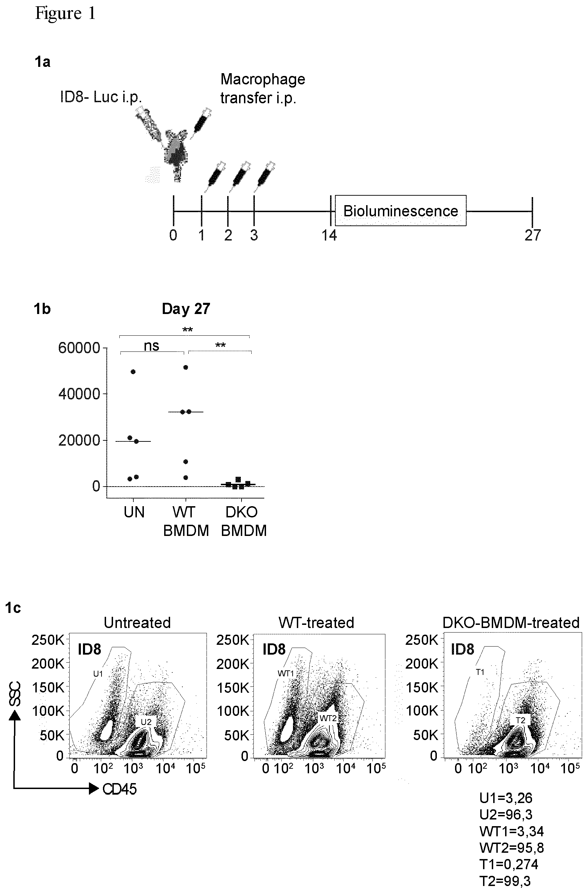

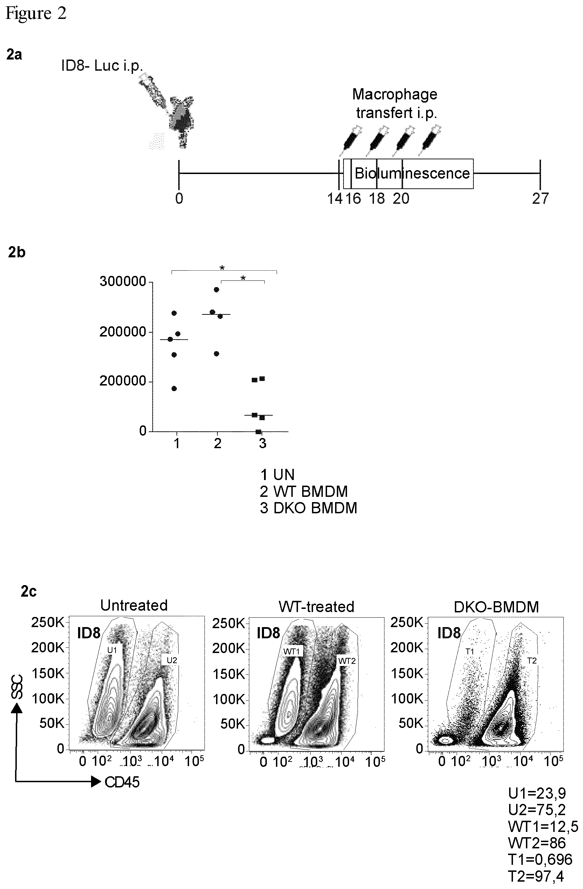

Figure 1. Adoptive transfer of mouse Maf-DKO macrophages inhibits tumor growth in vivo.
   (a) Representative scheme of the experimental procedure.
   (b) Quantification of bioluminescence (luciferase activity) from primary tumors obtained on day 27.
   (c) Quantification of ID8 tumor cells in the peritoneal cavity.
   (**: p≤0,007). UN: untreated mice ; WT: mice treated with wild type derived bone marrow macrophages ; BM-DKO: mice treated with MafB and c-Maf double deficient macrophages derived from the bone marrow
Figure 2. Adoptive transfer of mouse Maf-DKO macrophages reverses established tumor growth in vivo.
   (a) Representative scheme of the experimental procedure.
   (b) Quantification of bioluminescence (luciferase activity) from primary tumors obtained on day 27.
   (c) Gating strategy and quantification of ID8 tumor cells.
   (*: p≤0,04). UN: untreated mice; WT-BMDM: mice treated with wild type derived bone marrow macrophages; BM-DKO: mice treated with bone marrow derived MafB and c-Maf double deficient macrophages.

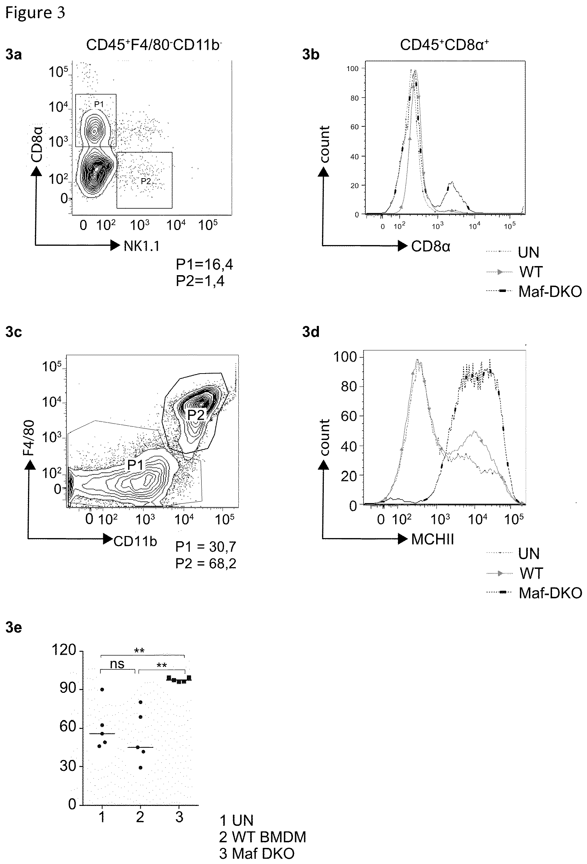

Figure 3. Cellular contents in the peritoneal ascites.
   (a) Gating strategy on CD8 T-cells and NK cells from the peritoneal cells of tumor bearing mice.
   (b) Histogram showing CD8α expression by peritoneal cells of untreated (dotted, small circles) WT-BMDM (solid, triangles) treated and Maf-DKO (dashed, rectangles) treated mice in the peritoneal cavity at day 27 after tumor initiation.
   (c) Gating strategy of peritoneal macrophages.
   (d) MHC class II expression in peritoneal macrophages.
   (e) Mean fluorescence intensity of MHCII expression by peritoneal macrophages.
   (**: p≤0,007). UN: untreated mice; WT: mice treated with wildtype derived bone marrow macrophages; Maf-DKO: mice treated with MafB and c-Maf double deficient macrophages.

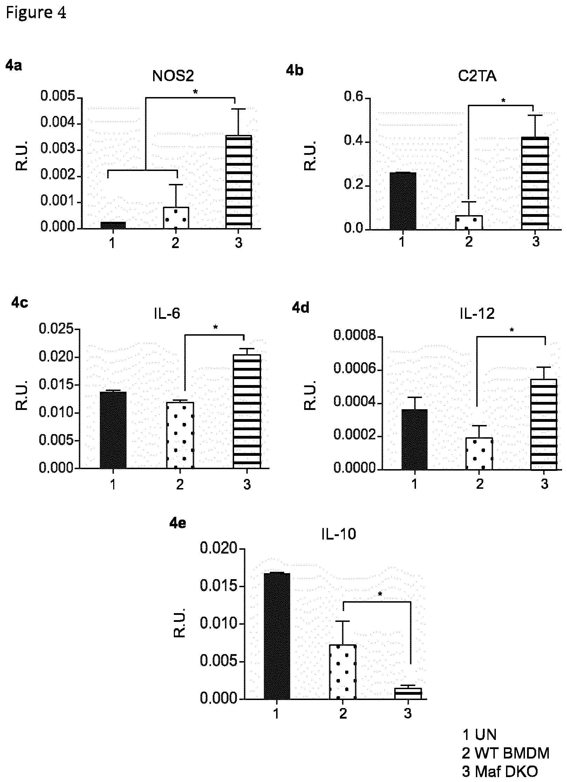

Figure 4. Sorted macrophages from Maf-DKO treated mice resemble M1 activated macrophages in vivo.
   Total mRNA was isolated from sorted CD11b+ peritoneal cells of tumor-bearing mice, for realtime PCR analysis of (a) NOS2, (b) C2TA, (c) IL-6, (d) IL-12 and (e) IL-10.
   (*: p≤0.04). Data are shown as mean of n=5. UN: untreated mice; WT: mice treated with wildtype derived bone marrow macrophages; Maf-DKO: mice treated with MafB and c-Maf double doubleknock out macrophages.
Figure 5. Mouse Maf-DKO macrophages are more sensitive to M1 stimuli and more resistant to M2 stimuli than WT macrophages. Total mRNA was isolated from cultured macrophages, for realtime PCR analysis of (a) IL-6, (b) IL-10, (c) C2TA, (d) arginase and (e) NOS2, and upon stimulation with compounds A, B, C and D. Maf-DKO macrophages are represented by the dotted bars, and WT macrophages by the dashed bars.
Figure 6. Mouse Maf-DKO macrophages are not re-educated by tumors in vitro. Cell supernatant was collected to analyze the production of (a) IL-6, and (b) TNFα upon stimulation with LPS in the absence (black bars) or in the presence (checkered bars) of ID8 tumor cell supernatant. SN=Supernatant
Figure 7. Mouse Maf-DKO macrophages reverse established melanoma growth in vivo. Experimental metastasis of B16 melanoma cells in C57BI6 mice.
   (a) Representative scheme of the experimental procedure. Macrophages were injected after 7 days of tumor development.
   (b) Lungs were removed and photographs were taken.
   (c) The number of surface visible metastatic colonies of the lungs were counted with a magnifying glass and the median was used for analysis). Results were obtained from 5 mice in each group.
   (*: p≤0,04 ; **: p≤0,007; *** :p≤0,0005). UN: untreated mice; WT: mice treated with wildtype derived bone marrow macrophages; Maf-DKO: mice treated with MAFB and c-Maf double deficient macrophages.

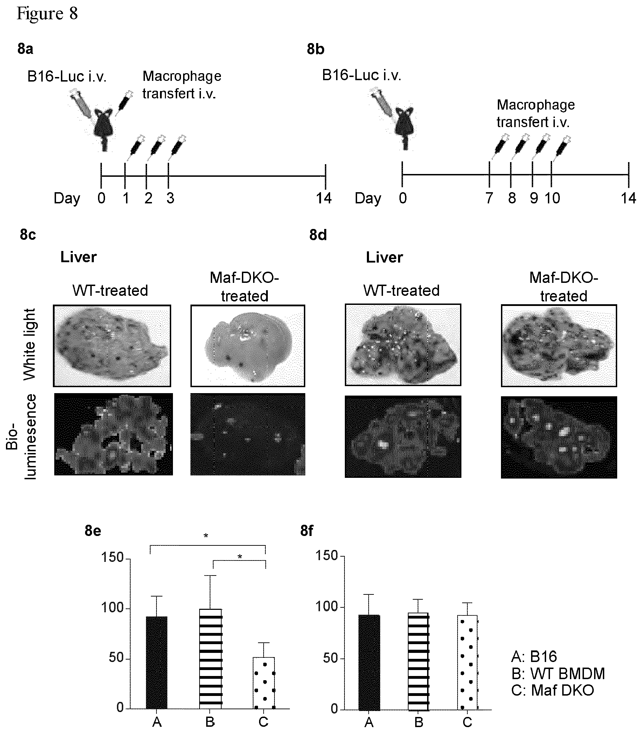

Figure 8. Mouse MAF DKO cells recruit also other leucocytes in the protection against B16 melanoma.
   Experimental metastasis of B16 melanoma cells in Rag2yc knock-out mice. (a,b) Representative scheme of the experimental procedure, in (a) mice were treated with macrophages immediately after tumor-initiation (results see 8c and 8e) and in (b) mice were injected with macrophages after tumor establishment (results see 8d and 8f). (c,d) Livers were removed and photographs were taken and bioluminescence was also recorded (pictures are from representative livers; white light not identical with bioluminescence). (e,f) The number of surface visible metastatic colonies from the mice treated in (a) and (b) were counted with a magnifying glass and the mediane was used for analysis. Results were obtained from 5 mice in each group. (*: p≤0.04). B16: untreated mice; WT: mice treated with wildtype derived bone marrow macrophages; Maf-DKO: mice treated with DKO macrophages.

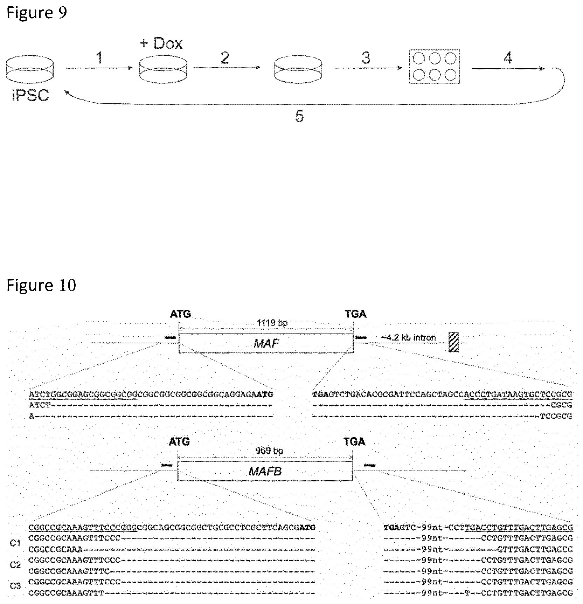

Figure 9: Workflow for CRISPR/Cas9-mediated deletion of MAF and MAFB in a doxycycline-inducible Cas9-expressing human iPSC line
   Step 1: Lipofectamine transfection with a vector expressing MAF-targeting sgRNAs
   Step 2: Cas9 induction upon doxycycline (Dox) treatment
   Step 3: Isolation of reporter-positive, sgRNA expressing cells by cell sorting to derive single-cell colonies
   Step 4: Selection of MAF KO clones
   Step 5: Repeat Steps 1 to 4 using the selected MAF KO iPSC clone with MAFB-targeting sgRNAs to knock out MAFB subsequently
Figure 10: Gene structure of human MAF and MAFB indicating position of CRISPR/Cas9 target sites in the 5' and 3' UTR of both genes, and indels obtained.
   The human MAF gene has a short and a long isoform generated by alternative splicing, the significance of the long isoform is unknown. We knocked out exon 1 of the MAF gene. Human MAFB is a single-exon gene. The first line of each sequence block refers to the wild-type locus, subsequent lines the indels generated by CRISPR/Cas9 editing. For MAF, only one clone was isolated (indels on both alleles are shown), for MAFB, the indels for 3 isolated clones are shown (C1, C2, C3: clone 1, 2, 3). In the sequence block, start and stop codons are bold, genomic target sequences are underlined. The protospacer positions are shown as a black line close to the start or stop codon.

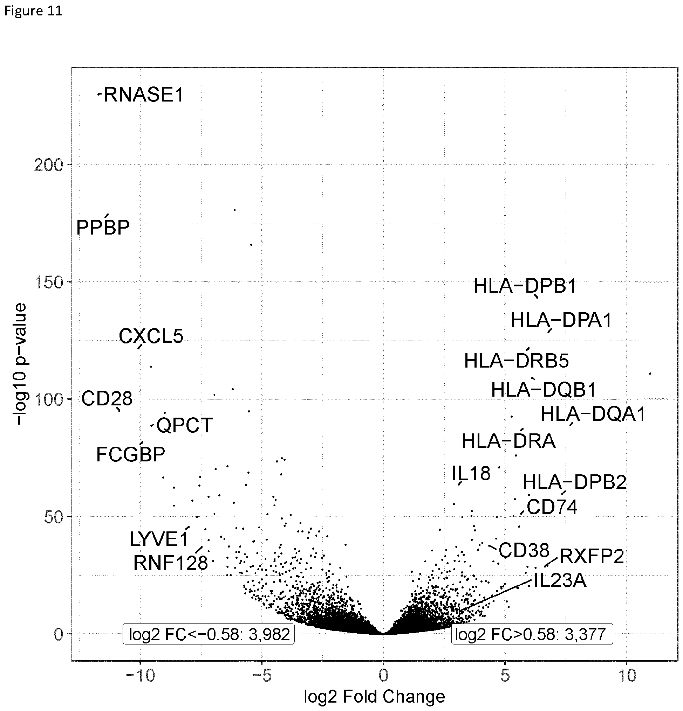

Figure 11: Volcano plot of differentially expressed genes between human wt- and DKOmacrophages. iPS cell derived macrophages were cultured for 7 days in M-CSF and GM-CSF and then shifted to M-CSF only for 2 hours. Differentially expressed genes as identified by deep sequencing are shown. X-axis shows the degree of change, γ-axis the statistical significance.

### REFERENCES

Asano, K., Nabeyama, A., Miyake, Y., Qiu, C.H., Kurita, A., Tomura, M., Kanagawa, O., Fujii, S., and Tanaka, M. (2011). CD169-positive macrophages dominate antitumor immunity by crosspresenting dead cell-associated antigens. Immunity 34, 85-95. Aziz, A., Soucie, E., Sarrazin, S., and Sieweke, M.H. (2009). MafB/c-Maf deficiency enables selfrenewal of differentiated functional macrophages. Science 326, 867-871. Bakri et al. (2005) Blood 105(7):2707-16 Balkwill, F., Charles, K.A., and Mantovani, A. (2005). Smoldering and polarized inflammation in the initiation and promotion of malignant disease. Cancer cell 7, 211-217. Balkwill, F., and Mantovani, A. (2001). Inflammation and cancer: back to Virchow? Lancet 357, 539-545. Bart, V et al. (2021). Immunology 163(2):128-144. Buchrieser et al. (2017) Stem Cell Reports 8(2):334-345 Chu et al. (2016) Proc Natl Acad Sci U S A 113(44):12514-12519 Condeelis, J., and Pollard, J.W. (2006). Macrophages: obligate partners for tumor cell migration, invasion, and metastasis. Cell 124, 263-266. Cordes S. and Barsh (1994) Cell 79(6):1025-34 Eichmann A. et al. (1997) Mech Dev 65(1-2):111-22. Freund et al. (2020) J. Exp. Med. 217(7): "Efficient gene knockout in primary human and murine myeloid cells by non-viral delivery of CRISPR-Cas9" Fusaki et al. (2009) Proc Jpn Acad Ser B Phys Biol Sci 85(8):348-62 Gemelli et al. (2006) Cell Death Differ 13(10):1686-96 Gonzalez et al. (2014) Cell Stem Cell 15(2):215-226 Gordon, S. (2003). Alternative activation of macrophages. Nature reviews Immunology 3, 23-35. Hagemann, T., Wilson, J., Burke, F., Kulbe, H., Li, N.F., Pluddemann, A., Charles, K., Gordon, S., and Balkwill, F.R. (2006). Ovarian cancer cells polarize macrophages toward a tumorassociated phenotype. J Immunol 176, 5023-5032. Hegde et al. (1999) Blood 94(5):1578-89. Ho et al. (1996). Cell 85(7):973-83 Isogai et al. (2018) Cell Reprogram 20(6):347-355 Karpinski et al (2016) Nat Biotechnol 34(4):401-9 Kataoka, K. et al. (1994) Mol. Cell. Biol. 14:700-712 Kelly L. et al. (2000) EMBO J 19(9):1987-97 Kerkar, S.P., and Restifo, N.P. (2012). Cellular constituents of immune escape within the tumor microenvironment. Cancer research 72, 3125-3130. Kerppola, T. K. and Curran, T. (1994) Oncogene 9:675-684 Kim et al. (1999) Immunity 10(6):745-51 Klichinsky et al. (2020) Nat Biotechnol. 38(8):947-953 Lansing et al. (2020) Nucleic Acids Res 48(1):472-485 Mantovani, A., Bottazzi, B., Colotta, F., Sozzani, S., and Ruco, L. (1992). The origin and function of tumor-associated macrophages. Immunology today 13, 265-270. Mantovani, A., Schioppa, T., Porta, C., Allavena, P., and Sica, A. (2006). Role of tumor associated macrophages in tumor progression and invasion. Cancer metastasis reviews 25, 315-322. Mantovani, A., Sozzani, S., Locati, M., Allavena, P., and Sica, A. (2002). Macrophage polarization: tumor-associated macrophages as a paradigm for polarized M2 mononuclear phagocytes. Trends in immunology 23, 549-555. Murray, P.J., and Wynn, T.A. (2011). Protective and pathogenic functions of macrophage subsets. Nature reviews Immunology 11, 723-737. Nevala, W.K., Vachon, C.M., Leontovich, A.A., Scott, C.G., Thompson, M.A., and Markovic, S.N. (2009). Evidence of systemic Th2-driven chronic inflammation in patients with metastatic melanoma. Clinical cancer research : an official journal of the American Association for Cancer Research 15, 1931-1939. O'Shea, J.J., and Murray, P.J. (2008). Cytokine signaling modules in inflammatory responses. Immunity 28, 477-487. Pollard, J.W. (2004). Tumour-educated macrophages promote tumour progression and metastasis. Nature reviews Cancer 4, 71-78. Qian, B.Z., and Pollard, J.W. (2010). Macrophage diversity enhances tumor progression and metastasis. Cell 141, 39-51. Scharenberg et al. (2020) Nat Commun 11(1):3327 Shi et al. (2018) Molecules 23(11):3044 Taylor et al. (2012) Cell Stem Cell 11(2):147-52 Tillmanns et al. (2007) Mol Cell Biol 27(15):5554-64 Wang, B., Li, Q., Qin, L., Zhao, S., Wang, J., and Chen, X. (2011). Transition of tumor associated macrophages from MHC class II(hi) to MHC class II(low) mediates tumor progression in mice. BMC immunology 12, 43. Wang et al. (2018) Mol Ther Nucleic Acids 11:130-141 Yoshida et al., (2005) Nucleic Acids Res 33(11):3465-78 Yumlu et al. (2017) Methods 121-122:29-44 Zhang et al. (2020) Front Immunol 11:1871

### DETAILED DESCRIPTION OF THE INVENTION

### Definitions:

A "chromosome" as used herein is one of the 46 regular human chromosomes. A "gene located on a chromosome" is a gene that is not extrachromosomal.

The term "gene" means a DNA sequence that codes for an RNA or a particular sequence of amino acids which comprise all or part of one or more proteins or enzymes, and may or may not include regulatory DNA sequences, such as promoter sequences, which determine for example the conditions under which the gene is expressed. A "promoter" or "promoter sequence" is a DNA regulatory region capable of binding RNA polymerase in a cell and initiating transcription of a downstream (3' direction) coding sequence. Some genes, which are not structural genes, may be transcribed from DNA to RNA, but are not translated into an amino acid sequence. Other genes may function as regulators of structural genes or as regulators of DNA transcription. In particular, the term gene may be intended for the genomic sequence encoding a protein, i.e. a sequence comprising regulator, promoter, intron and exon sequences.

Within the context of the present invention the terms "mutant" and "mutation" mean a detectable change in genetic material, i.e. genomic DNA. Mutations include deletion, insertion or substitution of one or more nucleotides. The mutation may occur in the coding region of a gene (i.e. in exons), in introns, or in the regulatory regions (e.g. enhancers, response elements, suppressors, signal sequences, polyadenylation sequences, promoters) of the gene. Generally, a mutation is identified in a subject by comparing the sequence of a nucleic acid or polypeptide expressed by said subject with the corresponding nucleic acid or polypeptide expressed in a control population. Where the mutation is within the gene coding sequence, the mutation may be a "missense" mutation, where it replaces one amino acid with another in the gene product, or a "nonsense" mutation, where it replaces an amino acid codon with a stop codon. A mutation may also occur in a splicing site where it creates or destroys signals for exon-intron splicing and thereby lead to a gene product of altered structure. Within the context of the present invention a mutation is not silent, i.e. it results at least in an alteration of the nucleotide sequence (where the gene product is a functional RNA) or an amino acid sequence (where the gene product is a protein) that renders the gene product non-functional or that reduces expression of the gene product at the RNA-level by at least 80%. Preferred mutations, for example deletions of whole genes, abolish gene expression.

As used herein gene expression is "inhibited" when expression of the gene at the RNA-level is reduced by at least 80% compared to gene expression in the corresponding wildtype, as measured by quantitative rt-PCR. Preferably expression of the gene at the RNA-level is reduced by at least 90%, such as by at least 95%.

As used herein gene expression is "abolished" when expression of the gene is not detectable at the RNA-level by q-PCR. In a qPCR an mRNA which is "expressed" and thus present at detectable levels will a) give a sigmoidal fluorescence curve that b) reaches a plateau at least within 38 PCR-cycles, preferably at least within 36 PCR-cycles, and c) produces a PCR-product of the expected length, i.e. corresponding in length to a PCR-product derived from mature mRNA and not from genomic DNA or unprocessed RNA intermediates. Preferably mRNA expression can be confirmed by these three criteria in three repeated qPCR experiments.

"Mutagenesis" as used herein is a laboratory process by which the genetic information of an organism is deliberately changed, resulting in a mutation. Preferred methods for mutagenesis herein are methods based on site-specific endonucleases.

A "site specific endonuclease" as used herein is an enzyme that cleaves a phosphodiester bond within a polynucleotide chain only at a very specific nucleotide sequence in the middle (endo) portion of a double-stranded DNA molecule, which sequence occurs preferably only once within the whole human genome so as to allow specific genetic engineering of a human target cell. Examples of site-specific endonucleases, which are typically used for genetic engineering in human target cells are zinc finger nucleases, TALENs and the CRISPR/Cas9 system.

As used herein the term "expression" may refer to gene expression of a polypeptide or protein, or to gene expression of a polynucleotide, such as miRNAs or IncRNAs, depending on the context. Expression of a polynucleotide may be determined, for example, by measuring the production of RNA transcript levels using methods well known to those skilled in the art. Expression of a protein or polypeptide may be determined, for example, by immunoassay using (an) antibody(ies) that binds the polypeptide specifically, using methods well known to those skilled in the art.

As used herein the "expression of an mRNA" relates to the transcriptional level of gene expression.

In the present invention, known methods can be used to detect such an expression of a gene. Examples of the method for quantitatively detecting an mRNA level in a cell or collection of cells include for example PCR-based methods (real-time PCR, quantitative PCR), and DNA microarray analysis. In addition, an mRNA level can be quantitatively detected by counting the number of reads according to what is called a new generation sequencing method. Exemplary methods, conditions and materials to be used for the determination of the expression level of an mRNA are described in the experimental section of this disclosure. A preferred method for determining expression of an mRNA is qPCR, as explained under "abolished expression" above.

Those skilled in the art can prepare an mRNA or a nucleic acid cDNA to be detected by the aforementioned detection methods by taking the type and state of the specimen and so forth into consideration and selecting a known method appropriate therefor. When the gene expression level in human macrophages of the invention is compared with the expression level of the same gene in wildtype macrophages, it is compared under otherwise identical conditions, i.e. both types of macrophages are to be cultured and treated in the same manner in order to allow for a scientifically meaningful comparison of mRNA levels.

An "allele" as used herein refers to one of the two copies of the same human gene. The two copies of a gene, one each on the two homologous chromosomes, can be identical or vary slightly in their individual sequences. The term allele is thus used slightly differently from its typical use herein, because it includes identical versions of the same human gene on the same relative place on the two homologous chromosomes. In a diploid cell, the two alleles of a given gene occupy corresponding loci on a pair of homologous chromosomes.

As used herein an "allele" or a "gene" is rendered nonfunctional if no further expression of the protein encoded by the gene or allele is detectable after the procedure that rendered the allele or gene nonfunctional. That is, no new protein is being expressed from an allele or gene that has been rendered nonfunctional. Eventually the protein encoded by an allele or gene that has been rendered nonfunctional will become undetectable in a population of cells consisting of cells with only nonfunctional alleles. The time until the protein encoded by said gene or allele will become undetectable depends on the dynamics of protein and mRNA turnover for said gene.

As used herein the term "deletion" means that a part of a DNA-sequence is missing compared to a wildtype reference sequence.

As used herein an "exon" is any part of a gene that will encode a part of the final mature RNA produced by that gene after introns have been removed by RNA splicing. The term exon refers to both the DNA sequence within a gene and to the corresponding sequence in RNA transcripts. In RNA splicing, introns are removed and exons are covalently joined to one another as part of generating the mature messenger RNA.

The term "chromosome rearrangement" as used herein is a chromosome abnormality involving a change in the structure of a native chromosome. Usually, chromosome rearrangements are caused by a breakage in the DNA double helices at two different locations, followed by a rejoining of the broken ends to produce a new chromosomal arrangement of genes, different from the gene order of the chromosomes or the chromosomes before it or they were broken. Such changes may involve several different classes of events, like large deletions of more than 10000 base pairs, gene duplications, gene inversions and translocations, within one or between two chromosomes.

The term "karyotyping" as used herein is the analysis of the chromosomes within cells from a sample. Chromosomes are stained and the skilled person then uses a microscope to examine the size, shape, and number of chromosomes in the cells of the cell sample. Usually, the stained sample is photographed to show the arrangement of the chromosomes. Karyotypes describe the chromosome count of an organism and what these chromosomes look like under a light microscope, in particular the length of the chromosomes, the position of the centromeres within those chromosomes, the banding pattern of the stained chromosomes, any differences between the sex chromosomes, and any other physical characteristics.

The term "guide RNA" as used herein relates to "guide RNA" as used in the context of a CRISPR/Cas9 DNA editing system. The guide RNA confers target sequence specificity to the CRISPR-Cas9 system. Guide RNAs are non-coding short RNA sequences which first bind to the Cas9 enzyme and then the guide RNA sequence guides the complex via base pairing to a specific location on the DNA, where Cas9 acts as an endonuclease and cuts the target DNA strand. Examples of guide RNAs are a) a synthetic trans-activating CRISPR RNA (tracrRNA) plus a synthetic CRISPR RNA (crRNA), wherein the crRNA is designed to identify the gene target site of interest, and b) a single guide RNA (sgRNA) that combines both the crRNA and tracrRNA within a single construct.

The term "MAF" denotes the human MAF transcription factor. MAF and other Maf family members form homodimers and heterodimers with each other and with Fos and Jun, consistent with the known ability of the AP-1 proteins to pair with each other (Kerppola and Curran (1994); Kataoka, K. et al. (1994)). The DNA target sequence to which MAF homodimers bind, termed the MAF response element (MARE), is a 13 or 14 bp element which contains a core TRE (T-MARE) or CRE (C-MARE) palindrome respectively, but MAF may also bind to DNA sequences diverging from these consensus sites including composite AP-1/MARE sites and MARE half sites with 5' AT rich extensions (Yoshida et al., 2005). MAF has been shown to stimulate transcription from several promoters, as well as to repress the transcription of other promoters. MAF has also been shown to induce the differentiation of T helper 2 (Th2) cells (Ho et al., 1996) due to its ability to activate the tissue specific transcription of the interleukin-4 (IL-4) (Kim et al. 1999). Furthermore, the over-expression of MAF in myeloid cell lines induces macrophage differentiation (Hegde et al., 1999). The gene for human MAF is located on chromosome 16, location 16q23.2 and is described in detail as Gene ID 4094 in the NCBI Gene database. Sequence and location information refer to the annotation release 109.20201120, which is the current release on December 9, 2020, Reference sequence assembly GCF_000001405.39 of the Genome Reference Consortium Human Build 38 patch release 13. This reference identifies the gene for MAF on the complementary strand between 79,593,838 and 79,600,737.

The term "MAFB" denotes the human MAFB transcription factor. This gene is expressed in a variety of cell types (including lens epithelial, pancreas endocrine, epidermis, chondrocyte, neuronal and hematopoietic cells, in particular macrophages) and encodes a protein containing a typical bZip motif in its carboxy-terminal region. In the bZip domain, MAFB shares extensive homology not only with MAF but also with other Maf-related proteins. MAFB can form a homodimer through its leucine repeat structure and specifically binds Maf-recognition elements (MAREs) palindromes, composite AP-1/MARE sites or MARE halfsites with AT rich 5' extensions (Yoshida, et al. 2005). In addition, MAFB can form heterodimers with Maf or Fos through its zipper structure but not with Jun or other Maf family members (Kataoka et al., 1994). MAFB is also known under the name kreisler, kr or Krml1 (for 'Kreisler Maf leucine Zipper 1'), because an x-ray induced chromosomic micro-inversion in kreisler mutant mice causes the tissue specific loss of MAFB expression in the developing hindbrain that is responsible for the kreisler phenotype (Cordes et al., 1994) (Eichmann et al., 1997). In the hematopoietic system MAFB is expressed selectively in the myeloid lineage and is up-regulated successively during myeloid differentiation from multipotent progenitors to macrophages. Indeed, this induction reflects an important role of MAFB in monocytic and macrophage differentiation. Thus the overexpression of MAFB in transformed chicken myeloblasts (Kelly et al., 2000, Bakri et al. 2005) and in human hematopoetic progenitors (Gemelli et al., 2006) inhibits progenitor proliferation (Tillmanns et al., 2007) and accelerates the formation of macrophages (Kelly et al., 2000, Bakri et al.2005, Gemelli et al., 2006), whereas a dominant negative version of MAFB inhibits this process (Kelly et al., 2000). Deletion of MafB in conjunction with Maf in mouse monocytes and macrophages enables extended proliferation (Aziz 2009, Soucie 2016). Together this indicates that MAFB induction is a specific and important determinant of the monocytic program in hematopoietic cells and is important for cell cycle arrest in differentiated monocyte and macrophages.

The gene for human MAFB is located on chromosome 20, location 20q12 and is described in detail as Gene ID 9935 in the NCBI Gene database. Sequence and location information refer to the annotation release 109.20201120, which is the current release on December 9, 2020, Reference sequence assembly GCF_000001405.39 of the Genome Reference Consortium Human Build 38 patch release 13. This reference identifies the gene for MAFB on the complementary strand between 40685848 and 40689236.

IL-4 as used herein relates to human interleukin 4. The protein encoded by IL-4 is a pleiotropic cytokine produced by activated T cells. IL-4 is a glycoprotein which is composed of 129 amino acids and has a molecular weight of about 20kDa. It is a ligand for interleukin 4 receptors. The interleukin 4 receptor also binds to IL13, which may contribute to many overlapping functions of this cytokine and IL13. IL4 is considered an important cytokine for tissue repair, counterbalancing the effects of proinflammatory type 1 cytokines.

The gene for human IL-4 is located on chromosome 5, location 5q31.1 and is described in detail as Gene ID 3565 in the NCBI Gene database. Sequence and location information refer to the annotation release 109.20210226. For cell culture experiments recombinant human IL-4, a 15.1 kDa globular protein containing 130 AAs, from Preprotech, catalog#200-04, was used. Specific activity was at least 5×10⁶ U/mg in a human TF-1 cell proliferation assay. IL-4 signaling occurs through interaction with its receptor. Interaction of IL-4 with its receptor results in receptor dimerization and activation. The activated receptor activates JAK1 and 3, which are associated with the receptor subunits. The activated JAK phosphorylates tyrosine residues the cytoplasmic tails of the receptor which then serves as docking sites for a number of adaptor or signaling molecules including STAT6. Activated STAT6 dimerizes, translocates to the nucleus and transcriptionally actives genes responsive to IL-4.

IL-13 as used herein relates to human interleukin 13. The protein encoded by IL-13 is a cytokine produced by a variety of immune cells, such as primarily by activated Th2 cells, but also by CD4 cells, NKT cells, mast cells, basophils and eosinophils. IL-13 is a central regulator in IgE synthesis, goblet cell hyperplasia, mucus hypersecretion, airway hyperresponsiveness, fibrosis and chitinase up-regulation.

The gene for human IL-13 is located on chromosome 5, location 5q31.1 and is described in detail as Gene ID 3596 in the NCBI Gene database. Sequence and location information refer to the annotation release 109.20210226.

ARG-1 as used herein relates to human arginase 1. Arginase catalyzes the hydrolysis of arginine to ornithine and urea. The type I isoform encoded by this gene, is a cytosolic enzyme and, under normal physiological conditions, expressed predominantly in the liver as a component of the urea cycle. However, ARG1 is also an immunosuppressive signal found predominantly with tumor associated macrophages. Macrophages polarized toward an immunosuppressive phenotype express ARG1.

The gene for human ARG-1 is located on chromosome 6, location 6q23.2 and is described in detail as Gene ID 383 in the NCBI Gene database. Sequence and location information refer to the annotation release 109.20210226.

IL-10 as used herein relates to human interleukin 10. IL-10 is a cytokine produced primarily by monocytes and macrophages and to a lesser extent by lymphocytes. This cytokine has pleiotropic effects in immunoregulation and inflammation. It down-regulates the expression of Th1 cytokines, MHC class II, and costimulatory molecules on macrophages. This cytokine can block NF-kappa B activity, and is involved in the regulation of the JAK-STAT signaling pathway. High IL-10 expression is found with tumor associated macrophages. Macrophages polarized toward immunosuppressive phenotype express IL-10. IL-10 expression has been described as a predictor of advanced tumor stage and associated with worse overall survival.

The gene for human IL-10 is located on chromosome 1, location 1q32.1 and is described in detail as Gene ID 3586 in the NCBI Gene database. Sequence and location information refer to the annotation release 109.20210226.

IL-6 as used herein relates to human interleukin 6. The protein encoded by IL-6 a cytokine that functions in inflammation and the maturation of B cells. In addition, the encoded protein has been shown to be an endogenous pyrogen capable of inducing fever in people with autoimmune diseases or infections. The protein is primarily produced at sites of acute and chronic inflammation, where it is secreted into the serum and induces a transcriptional inflammatory response through interleukin 6 receptor alpha.

The gene for human IL-6 is located on chromosome 7, location 7p15.3 and is described in detail as Gene ID 3569 in the NCBI Gene database. Sequence and location information refer to the annotation release 109.20210226.

CXCL10 as used herein relates to the CXC motif chemokine ligand 10. The protein encoded by CXCL10 is a chemokine of the CXC subfamily and a ligand for the receptor CXCR3. Binding of this protein to CXCR3 results in pleiotropic effects, including stimulation of monocytes, natural killer and T-cell migration, and modulation of adhesion molecule expression.

The gene for human CXCL10 is located on chromosome 4, location 4q21.1 and is described in detail as Gene ID 3627 in the NCBI Gene database. Sequence and location information refer to the annotation release 109.20210226.

C2TA (CIITA) as used herein relates to the class II major histocompatibility complex transactivator. C2TA is a protein with an acidic transcriptional activation domain, four leucine-rich repeats and a GTP binding domain. The protein is located in the nucleus and acts as a positive regulator of class II major histocompatibility complex gene transcription, and is referred to as the "master control factor" for the expression of these genes.

The gene for human C2TA is located on chromosome 16, location 16p13.13 and is described in detail as Gene ID 4261 in the NCBI Gene database. Sequence and location information refer to the annotation release 109.20210226.

TNF as used herein relates to the tumor necrosis factor. TNF is a multifunctional proinflammatory cytokine that belongs to the tumor necrosis factor (TNF) superfamily. This cytokine is mainly secreted by macrophages. It can bind to, and thus functions through its receptors TNFRSF1A/TNFR1 and TNFRSF1B/TNFBR. This cytokine is involved in the regulation of a wide spectrum of biological processes including cell proliferation, differentiation, apoptosis, lipid metabolism, and coagulation.

The gene for human TNF is located on chromosome 6, location 6p21.33 and is described in detail as Gene ID 7124 in the NCBI Gene database. Sequence and location information refer to the annotation release 109.20210226.

M-CSF as used herein, also called CSF1, relates to human colony stimulating factor 1. M-CSF is a cytokine that controls the production, differentiation, proliferation and function of macrophages. Different active isoforms of the protein are found as membrane bound or extracellular disulfide-linked homodimer, and are thought to be produced by proteolytic cleavage of membrane-bound precursors.

The gene for human M-CSF is located on chromosome 1, location 1p13.3 and is described in detail as Gene ID 1435 in the NCBI Gene database. Sequence and location information refer to the annotation release 109.20210226. For cell culture experiments recombinant human M-CSF, a 38 kDa homodimer produced in HEK293 cells from Gibco, catalog#PHC9501, was used. ED50 was at most 5 ng/ml as determnined by the dose dependent proliferation of M-NSF60 cell.

MHCII as used herein relates to the class II of the major histocompatibility complex (MHC) molecules which is typically found only on professional antigen-presenting cells such as mononuclear phagocytes including dendritic cells and macrophages as well as B cells. The MHC II presents peptides to T cells which are derived from extracellular proteins by endocytosis, and not derived from cytosolic proteins as is the case for MHC class I.

In humans, the MHC class II protein complex is encoded by the human leukocyte antigen gene complex (HLA). HLAs corresponding to MHC class II are HLA-DP, HLA-DM, HLA-DOA, HLA-DOB, HLA-DQ, and HLA-DR.

HLA-A or "human leukocyte antigen 1" relates to a protein belonging to the HLA class I heavy chain paralogues. This class I molecule is a heterodimer consisting of a heavy chain and a light chain (beta-2 microglobulin). The heavy chain is anchored in the membrane. Class I molecules play a central role in the immune system by presenting cytosolic peptides shuttled to the endoplasmic reticulum lumen so that they can be recognized by cytotoxic T cells. They are expressed in nearly all cells. The heavy chain is approximately 45 kDa and its gene contains 8 exons. Exon 1 encodes the leader peptide, exons 2 and 3 encode the alpha1 and alpha2 domains, which both bind the peptide, exon 4 encodes the alpha3 domain, exon 5 encodes the transmembrane region, and exons 6 and 7 encode the cytoplasmic tail. Polymorphisms within exon 2 and exon 3 are responsible for the peptide binding specificity of each class one molecule. Typing for these polymorphisms is routinely done for bone marrow and kidney transplantation. More than 6000 HLA-A alleles have been described. The gene for human HLA-A is located on chromosome 6, location 6p22.1 and is described in detail as Gene ID 3105 in the NCBI Gene database. Sequence and location information refer to the annotation release 109.20201120, which is the current release on December 9, 2020, Reference sequence assembly GCF_000001405.39 of the Genome Reference Consortium Human Build 38 patch release 13. This reference identifies the gene for HLA-A on the coding strand between 29942532 and 29945870.

HLA-B relates to a protein belonging to the HLA class I heavy chain paralogues. Hundreds of HLA-B alleles have been described. The gene for human HLA-B is located on chromosome 6, location 6p21.33 and is described in detail as Gene ID 3106 in the NCBI Gene database. Sequence and location information refer to the annotation release 109.20201120, which is the current release on December 9, 2020, Reference sequence assembly GCF_000001405.39 of the Genome Reference Consortium Human Build 38 patch release 13.

HLA-DR is a dimeric protein belonging to the HLA class II. The HLA class II molecule is a heterodimer consisting of an alpha (such as HLA-DRA) and a beta chain (such as HLA-DRB1, HLA-DRB3, HLA-DRB4 or HLA-DRB5), both anchored in the membrane. It plays a central role in the immune system by presenting peptides derived from extracellular proteins. Class II molecules are expressed in antigen presenting cells. As the alpha chain is practically invariable, variability in the composition of HLA-DR within an individual derives mostly from the ability of the alpha chain to pair with the beta chain from three different DR beta loci, HLA-DRB1 and two of any DRB3, DRB4 or DRB5 alleles. Within the DR molecule the beta chain contains essentially all the polymorphisms specifying the peptide binding specificities. In particular hundreds of DRB1 alleles have been described and some alleles have increased frequencies associated with certain diseases or conditions. These alleles are responsible for variability in the composition of HLA-DR within a population, and an individual can be homozygous with regard to HLA-DRB1 (i.e. father and mother carried the same allele of HLA-DRB1), but in general most individuals are heterozygous for HLA-DRB1. The gene for human HLA-DRB1 is located on chromosome 6, location 6p21.32 and is described in detail as Gene ID 31223 in the NCBI Gene database. Sequence and location information refer to the annotation release 109.20201120, which is the current release on December 9, 2020, Reference sequence assembly GCF_000001405.39 of the Genome Reference Consortium Human Build 38 patch release 13.

HLA-DQ is a dimeric protein belonging to the HLA class II. The HLA class II molecule is a heterodimer consisting of an alpha (such as HLA-DQA1) and a beta chain (such as HLA-DQB1), both anchored in the membrane. It plays a central role in the immune system by presenting peptides derived from extracellular proteins. Class II molecules are expressed in antigen presenting cells. Both α-chain and β-chain vary greatly between individuals and there are thus numerous alleles described for both, HLA-DQA1 and HLA-DQB1. As an MHC class II antigen-presenting receptor, DQ functions as a dimer containing two protein subunits, alpha (DQA1 gene product) and beta (DQB1 gene product), a DQ heterodimer. These receptors can be made from alpha+beta sets of two different DQ haplotypes, one set from the maternal and paternal chromosome. If one carries haplotype -A-B- from one parent and -a-b- from the other, that person makes 2 alpha isoforms (A and a) and 2 beta isoforms (B and b). This can produce 4 slightly different receptor heterodimers (or more simply, DQ isoforms). Two isoforms are in the cis-haplotype pairing (AB and ab) and 2 are in the trans-haplotype pairing (Ab and aB). Such a person is a double heterozygote for these genes, for DQ the most common situation. If a person carries haplotypes -A-B- and -A-b- then they can only make 2 DQ (AB and Ab), but if a person carries haplotypes -A-B- and -A-B- then they can only make DQ isoform AB, called a double homozygote. The gene for human HLA-DQA1 is located on chromosome 6, location 6p21.32 and is described in detail as Gene ID 3117 in the NCBI Gene database. The gene for human HLA-DQB1 is also located on chromosome 6, location 6p21.32 and is described in detail as Gene ID 3119 in the NCBI Gene database.

HLA-DP is also a dimeric protein belonging to the HLA class II. The HLA class II molecule is a heterodimer consisting of an alpha (such as HLA-DPA1) and a beta chain (such as HLA-DPB1), both anchored in the membrane. It also plays a role in the immune system by presenting peptides derived from extracellular proteins. The situation is similar as described for HLA-DQ. Both α-chain and β-chain vary between individuals and there are several alleles described for both, HLA-DPA1 and HLA-DPB1. As described for HLA-DQ, HLA-DP can be made from alpha+beta sets of two different HLA-DP haplotypes, one set from the maternal and paternal chromosome, and a person can be a double heterozygote for HLA-DPA1 and HLA-DPB1, for DP the most common situation, a person can be a single homozygote, and a person can also be a double homozygote for HLA-DP. The gene for human HLA-DPA1 is located on chromosome 6, location 6p21.32 and is described in detail as Gene ID 3113 in the NCBI Gene database. The gene for human HLA-DPB1 is also located on chromosome 6, location 6p21.32 and is described in detail as Gene ID 3115 in the NCBI Gene database.

CD2 as used herein relates, depending on the context, to the CD2 gene or to CD2 as a surface marker, which is the extracellular part of the CD2 protein, also known as LFA-2. The CD2 gene is described in detail as gene ID 914 in the NCBI Gene database.

CD28 as used herein relates, depending on the context, to the CD28 gene or to a surface marker, which is the extracellular part of the CD28 protein. The CD28 gene is described in detail as gene ID 940 in the NCBI Gene database.

CD38 as used herein relates, depending on the context, to the CD38 gene or to a surface marker, which is the extracellular part of the CD38 protein, also known as ADPRC1. The CD38 gene is described in detail as gene ID 952 in the NCBI Gene database.

CD64 as used herein relates, depending on the context, to the CD64 gene or to a surface marker, which is the extracellular part of FCGR1A, the Fc gamma receptor la. FCGR1A is described in detail as gene ID 2209 in the NCBI Gene database.

CD74 as used herein relates, depending on the context, to the CD74 gene or to a surface marker, which is the extracellular part of the CD74 protein, also known as HLADG. The CD74 gene is described in detail as gene ID 972 in the NCBI Gene database.

C5AR1 as used herein relates, depending on the context, to the C5AR1 gene or to a surface marker, which is the extracellular part of the C5AR1 protein, also known as CD88. The C5AR1 gene is described in detail as gene ID 728 in the NCBI Gene database.

CD70 as used herein relates, depending on the context, to the CD70 gene or to a surface marker, which is the extracellular part of the CD70 protein. CD70 is described in detail as gene ID 970 in the NCBI Gene database.

CD206 as used herein relates, depending on the context, to the CD206 gene or to a surface marker, which is the extracellular part of the mannose receptor C-type 1. CD206 is described in detail as gene ID 4360 in the NCBI Gene database.

CD163 as used herein relates, depending on the context, to the CD163 gene or to a surface marker, which is the extracellular part of the CD163 protein. CD163 is described in detail as gene ID 9332 in the NCBI Gene database.

IL15 as used herein relates, depending on the context, to the IL15 gene or to the secreted form of the IL15 protein, the active cytokine interleukine 15. IL15 is described in detail as gene ID 3600 in the NCBI Gene database.

IL18 as used herein relates, depending on the context, to the IL18 gene or to the secreted form of the IL18 protein, the active cytokine interleukine 18. IL18 is described in detail as gene ID 3606 in the NCBI Gene database.

IL23A as used herein relates, depending on the context, to the IL23A gene or to the IL23A as a subunit of the heterodimeric cytokine IL23. In that context, IL23A is detectable as the secreted active cytokine interleukine 23. IL23A is described in detail as gene ID 51561 in the NCBI Gene database.

RXFP2 as used herein relates, depending on the context, to the RXFP2 gene or to a surface marker, which is the extracellular part of the RXFP2 protein, the relaxin family peptide receptor 2. RXFP2 is described in detail as gene ID 122042 in the NCBI Gene database.

LYVE1 as used herein relates, depending on the context, to the LYVE1 gene or to a surface marker, which is the extracellular part of the LYVE1 protein, the lymphatic vessel hyaluronan receptor 1. LYVE1 is described in detail as gene ID 10894 in the NCBI Gene database.

STAB1 as used herein relates, depending on the context, to the STAB1 gene or to a surface marker, which is the extracellular part of the STAB1 protein, the stabilin1 (also called SCARH2 due to its possible role as a scavenger receptor). STAB1 is described in detail as gene ID 23166 in the NCBI Gene database.

LILRB5 as used herein relates, depending on the context, to the LILRB5 gene or to a surface marker, which is the extracellular part of the LILRB5 protein, the leukocyte immunoglobulin like receptor B5. LILRB5 is described in detail as gene ID 10990 in the NCBI Gene database.

RNASE1 as used herein relates, depending on the context, to the RNASE1 gene or to the secreted form of the RNASE1 protein, the ribonuclease A family member 1. RNASE1 is described in detail as gene ID 6035 in the NCBI Gene database.

F13A1 as used herein relates, depending on the context, to the F13A1 gene or to the secreted form of the F13A1 protein, the A subunit of the coagulation factor XIII. F13A1 is described in detail as gene ID 2162 in the NCBI Gene database.

QPCT as used herein relates, depending on the context, to the QPCT gene or to the secreted form of the QPCT protein, a glutaminyl cyclase. QPCT is described in detail as gene ID 25797 in the NCBI Gene database.

CCL7 as used herein relates, depending on the context, to the CCL7 gene or to the secreted form of the CCL7 protein, the chemokine CCL7. CCL7 is described in detail as gene ID 6354 in the NCBI Gene database.

RNF128 as used herein relates, depending on the context, to the RNF128 gene or to the RNF128 protein, an E3 ubiquitin ligase which is also known as GRAIL. RNF128 is described in detail as gene ID 79589 in the NCBI Gene database.
STAT6 is described in detail as gene ID 6778 in the NCBI Gene database.
IRF4 is described in detail as gene ID 3662 in the NCBI Gene database.
PPARγ is described in detail as gene ID 5468 in the NCBI Gene database.
KLF4 is described in detail as gene ID 9314 in the NCBI Gene database.
C/EPBβ (CEBPB) is described in detail as gene ID 1051 in the NCBI Gene database.
GATA3 is described in detail as gene ID 2625 in the NCBI Gene database.
JMJD3 (KDM6B) is described in detail as gene ID 23135 in the NCBI Gene database.
SOCS2 is described in detail as gene ID 8835 in the NCBI Gene database.
SOCS1 is described in detail as gene ID 8651 in the NCBI Gene database.
AKT1 is described in detail as gene ID 207 in the NCBI Gene database.
FCGBP is described in detail as gene ID 8857 in the NCBI Gene database.

In the context of the experiments which were done in mice, references to genes relate to the mouse genes which correspond to the indicated human gene names (e.g. a reference to "IL-6" in the context of the mouse experiments described in examples 1 to 8 relates to mouse IL-6).

As used herein a negative regulator is a gene product, in particular a polypeptide, which obstructs the binding of RNA polymerase to the promoter region, which inhibits the activity of an enhancer or which inhibits the activity of an activating transcription factor, thus leading to a reduction of transcription of a target gene, such as C2TA.

CD8+ T cells (also called cytotoxic T lymphocytes, or CTLs) develop in the thymus and express the T-cell receptor. They express a dimeric co-receptor, CD8, usually composed of one CD8α and one CD8β chain. CD8+ T cells recognize peptides presented by MHC Class I molecules, found on all nucleated cells. CD8+ T cells are very important for immune defence against intracellular pathogens, including viruses and bacteria, and for tumour surveillance.

NK cells, also known as natural killer cells or large granular lymphocytes (LGL), are a type of cytotoxic lymphocyte critical to the innate immune system. NK cells can be identified by the presence of CD56 and the absence of CD3 (CD56+, CD3-). NK cells are innate immune cells with analogous functions to that of cytotoxic T cells in the adaptive immune response. NK cells provide rapid responses to virusinfected cells, acting at around 3 days after infection, and respond to tumor formation. NK cells have the ability to recognize and kill stressed cells in the absence of antibodies and MHC. This role is important because harmful cells that are missing MHC I markers cannot be detected and destroyed by other immune cells, such as T lymphocyte cells.

"Recruitment" of cells, such as CD8+ T cells and/or NK cells, to a tumor can be tested in a mouse model, for example a humanized mouse model, by extracting the tumor mass, separating the cells and analyzing, for example by FACS after appropriate staining, the number of CD8+ T cells and/or NK cells associated with the tumor mass. A treatment that is able to recruit said cells to the tumor (such as the injection of macrophages of the invention) will over time lead to a situation where these cells make up a higher percentage in terms of cell numbers of the analyzed tumor mass when compared to appropriate controls without said treatment.

The term "proliferating cell" as used herein refers to a cell that is capable of cell division. A cell is a proliferating cell if a population of at least 1000 "proliferating cells" increases in cell number by at least 4-fold after 8 days under suitable cultivation conditions, i.e. when n(192h) / n(0h) is at least 4,00 with n being the total number of cells in the cell population at the indicated time points.

The term "differentiated human cell" as used herein is a cell that does not change cell type and even upon cell division gives rise to two cells of the same cell type. This is in contrast to "pluripotent cells" which can differentiate into all cell types of the adult organism and oligopotent cells, which can differentiate into a few closely related cell types.

A "myeloid cell" as used herein is a cell of hematopoietic origin that is not lymphoid and not erythromegakaryocytic and not a multi-lineage progenitor with more than myeloid lineage potential.

An iPS cell or "induced pluripotent stem cell" as used herein means a cell having pluripotency, which is obtained by reprogramming a somatic cell. Several groups including the group of Professor Shinya Yamanaka et al. of Kyoto University, the group of Rudolf Jaenisch et al. of Massachusetts Institute of Technology, the group of James Thomson et al. of the University of Wisconsin, and the group of Konrad Hochedlinger et al. of Harvard University have succeeded in producing such induced pluripotent stem cells. The induced pluripotent stem cells have attracted great interest as ideal pluripotent cells having neither immunological rejections nor ethical issues. For example, International Publication WO2007/069666 describes nuclear reprogramming factors for somatic cells that include the gene products of Oct family gene, Klf family gene and Myc family gene, and nuclear reprogramming factors for somatic cells that include the gene products of Oct family gene, Klf family gene, Sox family gene and Myc family gene. This publication also describes a method for producing induced pluripotent stem cells by nuclear reprogramming of somatic cells, which comprises a step of allowing the aforementioned nuclear reprogramming factors to come into contact with somatic cells.

A "monocyte" is a mononuclear phagocyte of the peripheral blood. Monocytes vary considerably, ranging in size from 10 to 30 µm in diameter. The nucleus to cytoplasm ratio ranges from 2:1 to 1:1. The nucleus is often band shaped (horseshoe), or reniform (kindey-shaped). It may fold over on top of itself, thus showing brainlike convolutions. No nucleoli are visible. The chromatin pattern is fine, and arranged in skein-like strands. The cytoplasm is abundant and appears blue gray with many fine azurophilic granules, giving a ground glass appearance in Giemsa staining. Vacuoles may be present. More preferably, the expression of specific surface antigens is used to determine whether a cell is a monocyte cell. Phenotypic markers of human monocyte cells include CD11b, CD11c, CD33, CD45 and CD115. Generally, human monocyte cells express CD9, CD11b, CD11c, CDw12, CD13, CD15, CDw17, CD31, CD32, CD33, CD35, CD36, CD38, CD43, CD45, CD49b, CD49e, CD49f, CD63, CD64, CD65s, CD68, CD84, CD85, CD86, CD87, CD89, CD91, CDw92, CD93, CD98, CD101, CD102, CD111, CD112, CD115, CD116, CD119, CDw121b, CDw123, CD127, CDw128, CDw131, CD147, CD155, CD156a, CD157, CD162, CD163, CD164, CD168, CD171, CD172a, CD180, CD131a1, CD213a2, CDw210, CD226, CD281, CD282, CD284, CD286 and optionally CD4, CD14, CD16, CD40, CD45RO, CD45RA, CD45RB, CD62L, CD74, CD141, CD142, CD169, CD170, CD181, CD182, CD184, CD191, CD192, CD194, CD195, CD197, CD206, CX3CR1. Unless specifically excluded, "monocytes" are comprised by the term "macrophage" as used herein.

A "dendritic cell" (DC) is an antigen presenting cell existing in vivo, in vitro, ex vivo, or in a host or subject, or which can be derived from a hematopoietic stem cell, a hematopoietic progenitor or a monocyte. Dendritic cells and their precursors can be isolated from a variety of lymphoid organs, e.g., spleen, lymph nodes, as well as from bone marrow and peripheral blood. The DC has a characteristic morphology with thin sheets (lamellipodia) extending in multiple directions away from the dendritic cell body. DCs express constitutively both MHC class I and class II molecules, which present peptide antigens to CD8+ and CD4+ T cells respectively and can activate naïve T-cells. In addition, human skin and mucosal DCs also express the CD1 gene family, MHC class I-related molecules that present microbial lipid or glycolipid antigens. The DC membrane is also rich in molecules that allow adhesion of T cells (e.g. intercellular adhesion molecule 1 or CD54) or that co-stimulate T-cell activation such as B7-1 and B7-2 (also known as CD80 and CD86 respectively). Generally, DCs express CD85, CD180, CD187 CD205 CD281, CD282, CD284, CD286 and in a subset manner CD206, CD207, CD208 and CD209. Unless specifically excluded, "dendritic cells" are comprised by the term "macrophage" as used herein.

A "macrophage" as used herein comprises macrophages, monocytes and dendritic cells. Preferably, however, a "macrophage" as used herein is a macrophage strictu sensu. A macrophage is a cell exhibiting properties of phagocytosis. The morphology of macrophages varies among different tissues and between normal and pathologic states, and not all macrophages can be identified by morphology alone. However, most macrophages are large cells with a round or indented nucleus, a well-developed Golgi apparatus, abundant endocytotic vacuoles, lysosomes, and phagolysosomes, and a plasma membrane covered with ruffles or microvilli. The key functions of macrophages in innate and adaptive immunity are the phagocytosis and subsequent degradation of senescent or apoptotic cells, microbes and neoplastic cells, the secretion of cytokines, chemokines and other soluble mediators, and the presentation of foreign antigens (peptides) on their surface to T lymphocytes. Macrophages are derived from multipotent progenitor cells, common myeloid progenitor cells and granulocytemonocyte progenitor cells in the bone marrow of mammalian organisms, which ultimately develop through further progenitor stages into monocytes that then enter the peripheral bloodstream. Unlike neutrophils, with their multilobed nuclei, monocytes have kidney-shaped nuclei and assume a large cell body during further differentiation and activation. Throughout life, some monocytes adhere to and migrate through the endothelium of the capillaries into all organs, where some of them can differentiate into resident tissue macrophages or dendritic cells (see below). Besides monocyte origin, tissue resident macrophages can also develop from early primitive macrophage progenitors of the yolk sac from before the establishment of definitive hematopoiesis, from erythroid-macrophage progenitors (EMP) of diverse hematopoietic sites of the embryo or from embryonic hematopoietic stem cell derived fetal monocytes. These embryo derived macrophages can persist into adulthood and be maintained long term independently of input from adult hematopoietic stem cells and monocytes. Lymphatic tissues, such as the lymph nodes and the spleen, are particularly rich in macrophages but tissue resident macrophages are present in essentially every organ of the body. In some organs the macrophages carry special names, as summarized in Table 1.

**Table 1 Examples of tissue macrophages**

| **Organ** | **Macrophage population** |
|---|---|
| Bone | Osteoclasts |
| Central nervous system | Microglia |
| Connective tissue | Histiocytes |
| Chorion villi | Hofbauer cells |
| Kidney | Mesangial cells |
| Liver | Kupffer cells |
| Peritoneal cavity | Peritoneal macrophages |
| Pulmonary airways | Alveolar macrophages, lung interstitium interstitial macrophages |
| Skin | Epidermal langerhans cells and dermal macrophages |
| Spleen | Marginal zone macrophages, Metallophilic macrophages, Red pulp macrophages, White pulp macrophages |

Further examples of macrophages are peritubular and interstitial testicular macrophages, cardiac macrophages from heart, adipose tissue macrophages from fat tissue, large and small intestinal macrophages from intestine, skeletal muscle macrophages, synovial macrophages from joints, arterial adventitia macrophages, arterial intima macrophages, blood vessel associated macrophages, resident pancreatic macrophages, meningeal macrophages, pleural macrophages and omentum macrophages.

In the context of the invention, the macrophage can be selected from any one of the macrophages mentioned above, preferably the macrophage is selected from the group consisting of microglia, histiocytes, Hofbauer cells, mesangial cells, Kupffer cells, peritoneal macrophages, alveolar macrophage, epidermal or dermal macrophages, marginal zone macrophages, metallophilic macrophages, Red pulp macrophages, white pulp macrophages and osteoclasts. Macrophages can also be derived from human iPS cells via in vitro differentiation protocols, as will be described elsewhere.

Macrophages are an important source of cytokines. Functionally, the numerous products can be placed into several groups: (1) cytokines that mediate a proinflammatory response, i.e. help to recruit further inflammatory cells (e.g. IL-1, II-6, TNFs, CC and CXC chemokines, such as IL-8 and monocytechemotactic protein 1); (2) cytokines that mediate T cell and natural killer (NK) cell activation (e.g. IL-1, IL-12, IL-15, IL-18); (3) cytokines that exert a feedback effect on the macrophage itself (e.g. IL-1, TNFs, IL-12, IL-18, M-CSF, IFNα/β, IFNγ); (4) cytokines that downregulate the macrophage and/or help to terminate the inflammation (e.g. IL-10, TGFβs), (5) cytokines important for wound healing or to support tissue stem cells (e.g. EGF, PDGF, bFGF, TGFβ) or to support blood vessel growth (e.g. VEGF) or neurons (e.g. neurotrophic factors, kinins). The production of cytokines by macrophages can be triggered by microbial products such as LPS, by interaction with type 1 T-helper cells, or by soluble factors including prostaglandins, leukotrienes and, most importantly, other cytokines (e.g. IFNγ). Generally, human macrophages express CD11c, CD11b, CD14, CD18, CD26, CD31, CD32, CD36, CD45RO, CD45RB, CD63, CD68, CD71, CD74, CD87, CD88, CD101, CD115, CD119, CD121b, CD155, CD156a, CD204, CD206 CDw210, CD281, CD282, CD284, CD286 and in a subset manner CD163, CD169 CD170, MARCO, FOLR2, LYVE1. Activated macrophages can further express CD23, CD25, CD69, CD105 and HLA-DR, HLA-DP and HLA-DQ

As used herein a macrophage is resistant to M2-polarization by M-CSF (or a combination of cytokines such as M-CSF with IL-4 and/or IL-13), if prolonged cultivation of the macrophage (or macrophages) does not lead to a loss of all features that are characteristic of an M1-polarized macrophage, in particular it does not lead to a loss of all features that are typical of an M1-polarized macrophage. Characteristic features of M1-polarized macrophages as used herein can be any one of upregulated expression of HLA class II genes, such as HLA-DPA1, HLA-DPB1, HLA-DPB2, HLA-DRA, HLA-DRB5, HLA-DQA1, HLA-DQB1, and/or upregulated expression of RXFP2, CD74, CD38, CD2, IL18 and/or IL23A, and/or upregulated secretion of IL18, IL15 and/or IL23, and/or downregulated expression of RNASE1, PPBP, CD28, LYVE1, FCGBP, F13A1, QPCT, CCL7 and/or RNF128, and/or downregulated secretion of PPBP, CCL7, RNASE1, F13A1, QPCT and/or FCGBP. M1 macrophages can also be characterized by the surface markers that they express or don't express and/or the pattern of expressed and/or nonexpressed surface markers. Further examples of typical features of M1-macrophages as used herein can be being positive for MHC II, being positive for CD74, being positive for CD2, being negative for LYVE1, being negative for CD28, being negative for STAB1 and/or being negative for LILRB5.

A "surface marker" is a molecule, typically a protein or a carbohydrate structure, that is present and accessible on the exterior of the plasma membrane of a cell and that is specific for a particular cell type or a limited number of cell types, thereby being a "marker" for these cell types. Examples of surface markers on human macrophages are CD11c, CD11b, CD14, CD16, CD18, CD26, CD31, CD32, CD33, CD36, CD45RO, CD45RB, CD63, CD64, CD68, CD71, CD74, CD87, CD88, CD101, CD115, CD119, CD121b, CD155, CD156a, CD163, CD169, CD170, CD204, CD206 CDw210, CD281, CD282, CD284, CD286, MARCO, FOLR2, CX3CR1 and LYVE1.

A cell is "positive" for a surface marker if staining with a surface-marker-specific antibody creates a specific fluorescence signal in a FACS experiment. The principles of FACS are explained in detail in the book "practical flow cytometry", 4th edition by Howard M. Shapiro. In a FACS experiment a collection of cells is typically stained with several fluorescent antibodies, each one selectively binding a different surface marker and having a different fluorochrome. This allows the selection of particular cell types within a heterogeneous collection of cells by appropriate gating strategies in a FACS experiment. A specific fluorescence signal by the surface-marker-specific antibody is typically then verified in a onedimensional histogram plot by comparing the histograms for the staining with all antibodies with the histogram for the staining with the mix of antibodies where only the surface-marker-specific antibody has been omitted (so called "FMO" or "fluorescence minus one" signal). If the two histograms are different such that the staining with the mix of all antibodies produces more fluorescence than the FMO control, then the tested collection of cells is positive for the tested cell surface marker. In terms of visual appearance of the histogram this means that the peak of fluorescence for the staining with the mix of all antibodies is shifted to higher fluorescence values when compared to the FMO control. Preferably the two histograms - all antibodies on the one hand and FMO on the other - overlap by at most 70 area% (area under the curve), such as at most 50 area%, for example by at most 25 area %.

"Secretion" of a cytokine, such as IL18 and/or IL15, can be determined by qualitatively and/or quantitatively measuring the appearance of said cytokine in the supernatant of a cell culture with immunobiochemical methods, for example by ELISA-based methods or a bead-based multiplex assay, such as the Luminex technology, to name but two examples. Briefly, the medium used for cell growth is analyzed with regard to the presence of a particular cytokine to-be-tested BEFORE being added to a collection of cells and AFTER the cells, which are to be tested for cytokine secretion, have been cultured in it. A cytokine is "secreted" by the cells which were cultured in the medium if the concentration of the cytokine in the supernatant has increased during the cultivation period and if this increase in cytokine concentration can be confirmed in three consecutive independent measurements. IL6 can be detected at or even below a concentration of 0.1pg/ml, for example by the meso scale discovery immunoassay "V-PLEX Human IL-6 Kit" of Meso Scale Diagnostics. CXCL10 can be detected at or even below a concentration of 5pg/ml, for example by the LANCE Ultra human CXCL10 detection kit of Perkin Elmer.

The terms "phagocytic cells" and "phagocytes" are used interchangeably herein to refer to a cell that is capable of phagocytosis. There are different main categories of professional phagocytes: mononuclear phagocytes, comprising macrophages sensu strictu, monocytes and dendritic cells as well as polymorphonuclear leukocytes (neutrophils). However, there are also "non-professional" phagocytic cells known to participate in efferocytosis, efferocytosis being the process by which professional and nonprofessional phagocytes dispose of apoptotic cells in a rapid and efficient manner.

The term "progenitor cell" as used herein relates to cells which are descendants of stem cells and which can further differentiate to create specialized cell types. There are many types of progenitor cells throughout the human body. Each progenitor cell is only capable of differentiating into cells that belong to the same tissue or organ. Some progenitor cells have one final target cell that they differentiate to, while others have the potential to terminate in more than one cell type. Progenitor cells are thus an intermediary cell type involved in the creation of mature cells in human tissues and organs, the blood, and the central nervous system. Hematopoietic progenitor cells are an intermediate cell type in blood cell development. They are immature cells that develop from hematopoietic stem cells and eventually differentiate into one of more than ten different types of mature blood cells.

The term "CD34+ multipotent progenitors" as used herein are a CD34 surface antigen expressing stemcell enriched hematopoietic progenitor population, that are not macrophages, monocytes or dendritic cells.

The term "monoblasts" as used herein relates to committed progenitor cells in bone marrow that differentiated from myeloid progenitor cells in the process of hematopoiesis. They can mature into monocytes which, in turn, can develop into macrophages.

The term "collection of cells" as used herein relates to at least 10000 cells, which cells are alive.

The term "expansion" of cells as used herein is the process of culturing cells under suitable laboratory conditions and increasing the number of living cells by mitotic divisions of the cultured cells.

The term "after having been exposed" as used herein means that cells were cultivated in the continuous presence of a particular agent, such as a cytokine, for the indicated period of time and then tested immediately thereafter.

The term "genetically modified" cell as used herein relates to a cell wherein the cell's DNA has been changed using biotechnological methods. For example, cells wherein the cells' DNA has been manipulated by the use of a CRISPR/Cas9 DNA editing system, wherein the manipulation has left a detectable change in the cells' DNA, are genetically modified cells.

The term "under suitable cultivation conditions" as used herein are conditions under which the phagocytic cells of the invention are able to grow. The cultivation is typically carried out in a cell culture medium supplemented with appropriate growth factors and at a suitable temperature and in a suitable controlled atmosphere. RPMI medium (RPMI derived its name from the Roswell Park Memorial Institute and is a medium frequently used for the culture of human lymphocytes) supplemented with 10% FBS (PAA-GE Healthcare, A15-101), supplemented with 100 units/ml penicillin, 100 ug/ml streptomycin (Thermo Fisher, #15140122), 2 mM GlutaMAX (Thermo Fisher, #35050038), 1 mM sodium pyruvate (Thermo Fisher, #11360-039), 50 ng/ml M-CSF (Thermo Fisher, #PHC9504), and, when indicated, 50 ng/ml GM-CSF (Peprotech, #300-03), as described in example 3, is a suitable cultivation medium. Cultivation is typically at 37°C and 5% CO2, 21% O2.

The term "reactive oxygen species" as used herein relates to unstable molecules that contains oxygen and that easily react with other molecules in a cell. Typically, reactive oxygen species are free radicals. Reactive Oxygen Species are known to be a component of the killing response of immune cells to microbial invasion.

The term "activation" as used herein relates to the phenomenon that external stimuli can induce changes to cell, thereby activating it. Macrophages, for example, can be activated by cytokines such as interferon-gamma (IFN-gamma) and bacterial endotoxins, such as lipopolysaccharide (LPS). Activated macrophages undergo many changes which allow them to kill invading bacteria or infected cells. They release toxic chemicals and proteins which have toxic effects on other cells. Activated macrophages are larger, have increased metabolism, increased levels of lysosomal proteins, and a greater ability to phagocytosis and kill microbes. Activated macrophages also release proteases, neutrophil chemotatic factors; reactive oxygen species such as nitric oxide and superoxide; cytokines such as tumor necrosis factor-alpha (TNF-alpha), interleukin one and eight (IL-1 and IL-8), eicosanoids, as well as growth factors. These products of activated macrophages can result in the kind of tissue destruction which is a hallmark of inflammation

The term "adherent" as used herein relates to the property of adherent cells that they attach to a solid substrate, such as the bottom of a tissue culture flask. In contrast, suspension cells will float and grow suspended in the culture medium, so they don't need to be mechanically or chemically removed.

Lamellipodium as used herein is a projection on the leading edge of the cell. It contains a quasi-two-dimensional actin mesh. A filopodium is a finger-like structure within the lamellipodium that has spread beyond the lamellipodium frontier and thus extends from it.

By "purified" and "isolated" it is meant, when referring to a polypeptide or a nucleotide sequence, that the indicated molecule is present in the substantial absence of other biological macromolecules. When referring to a cell or a population of cells, the term means that said cell or said population of cells is present in the substantial absence of other cells or population of cells. The term "purified" as used herein preferably means at least 75% by weight or number, more preferably at least 85% by weight or number, still preferably at least 95% by weight or number, and most preferably at least 98% by weight or number, of biological macromolecules or cells of the same type are present. An "isolated" nucleic acid molecule, which encodes a particular polypeptide refers to a nucleic acid molecule which is substantially free of other nucleic acid molecules that do not encode the subject polypeptide; however, the molecule may include some additional bases or moieties which do not deleteriously affect the basic characteristics of the composition.

As used herein, the terms "tumor," "tumor cells," "cancer," and "cancer cells," refer to cells which exhibit relatively autonomous growth, so that they exhibit an aberrant growth phenotype characterized by a significant loss of control of cell proliferation (i.e., de-regulated cell division) and/or disruption/non respect of normal tissue organization and architecture. Tumor cells can be malignant or benign, while cancer cells are malignant. A "metastatic cell or tissue" means that the cell can invade, settle in and destroy neighboring and distant body structures.

A "solid tumor" as used herein is a non-hematological tumor. Solid tumors may be benign (not cancer), or malignant (cancer). Different types of solid tumors are named for the type of cells that form them. Examples of solid tumors are sarcomas, carcinomas, and some lymphomas, while leukemias (cancers of the blood) generally do not form solid tumors.

As used herein, the term "subject" denotes a human being.

In the context of the invention, the term "treating" or "treatment", as used herein, means reversing, alleviating, inhibiting the progress of, or preventing the disease or condition to which such term applies, or one or more symptoms of such disease or condition.

The term "chimeric antigen receptor" as used herein is a fusion of an extracellular recognition domain (e.g. an antigen-specific targeting region), a transmembrane domain, and one or more intracellular signaling domains. Upon antigen engagement by the extracellular recognition domain, the intracellular signaling portion of the CAR can initiate an activation-related response in an immune cell, such as the release of cytolytic molecules to induce tumor cell death, etc.

The term "ex-vivo" as used herein means outside of a living body.

The term "in-vitro" as used herein means outside of a living body and within a laboratory environment. For example, cells which are cultured "in-vitro" are cultured in controlled, and often artificial, culture media.

As used herein "autologous" is a term referring to cells derived from an individual's own cells. For example, in autologous blood transfusions, the patient's own blood is collected and reinfused into the body.

As used herein "allogeneic" is a term referring to human cells that are not derived from an individual's own cells. For example, an allogeneic stem cell transplant is different from an autologous stem cell transplant, which uses stem cells from the patient's own body.

All numerical designations, e.g., pH, temperature, time, concentration, and molecular weight, including ranges, are approximations which are varied (+) or (-) by increments of 0.1. It is to be understood, although not always explicitly stated that all numerical designations are preceded by the term "about." It also is to be understood, although not always explicitly stated, that the reagents described herein are merely examples and that equivalents of such are known in the art.

It is to be understood that this invention is not limited to the particular materials and methods described herein. It is also to be understood that the terminology used herein is for the purpose of describing particular embodiments and is not intended to limit the scope of the present invention, which will be limited only by the appended claims. As used herein, the singular forms "a", "an", and "the" include plural reference unless the context clearly indicates otherwise. Unless defined otherwise, all technical and scientific terms used herein have the same meanings as commonly understood by one of ordinary skill in the art to which this invention belongs. The following references provide one of skill with a general definition of many of the terms used in this invention - unless defined otherwise herein - and ranked in increasing order of priority: Singleton et al., Dictionary of Microbiology and Molecular Biology (3rd ed. 2006); The Glossary of Genomics Terms (JAMA. 2013; 309(14):1533-1535), Janeway's Immunobiology, 9th edition and "Practical Flow Cytometry", 4th edition by H.M. Shapiro.

All publications mentioned herein are cited for the purpose of describing and disclosing the cell lines, protocols, reagents and vectors which are reported in the publications and which might be used in connection with the invention. Nothing herein is to be construed as an admission that the invention is not entitled to antedate such disclosure by virtue of prior invention.

### Detailed description

We have observed, for example in example 8, that lymphoid cells, in particular T-cells, and/or NK cells are involved in an important aspect of the antitumor activity of macrophages. In Rag2yc mice, where lymphoid cells are missing, administration of myeloid cells AFTER establishment of metastases did no longer lead to a regression of metastases, while in wildtype mice with a physiological lymphocyte compartment it did. In example 11 we observed a dramatic upregulation of certain HLA class II genes in the human MAF/MAFB DKO macrophages, which human MAF/MAFB macrophages correspond to the mouse MAF/MAFB DKO macrophages which have antitumor activity. Based on these observations, but without wishing to be bound by any theory, we suggest that one aspect of the antitumor activity of myeloid cells, such as macrophages, is mediated by the interaction of the myeloid cells, such as macrophages, with T-cells, such as CD4+ T-cells, via the interaction of the MHC II complex on myeloid cells, in particular myeloid cells having at least one typical M1-marker, with the T-cell receptor on CD4+ cells. This has important implications for improved anticancer cell therapies with allogeneic myeloid cells, such as macrophages, monocytes or dendritic cells, and in particular anticancer cell therapies with macrophages strictu sensu.

Because of MHC restriction those T-cells, which are specific for tumor-derived neoantigens should respond much better to a tumor-derived peptide-antigen bound to an autologous MHC complex than to the same tumor-derived peptide-antigen bound to an unmatched allogenic MHC complex. We therefore suggest that it is important to consider that the allogeneic myeloid cells, such as the allogeneic macrophages, carry at least one version of an HLA-DP, HLA-DQ and/or HLA-DR complex, which is also present in the individual to be treated. In other words, there should be at least a partial match between the patient's lymphoid immune cells and the allogeneic myeloid cells, such as the allogeneic macrophages, at the level of the MHC-II complex, so that, for example, the antigen-specific recognition of a tumor-derived neoantigen by a neoantigen-specific patient's CD4+ T-cell is significantly improved when said neoantigen is presented on an allogeneic myeloid antigen presenting cell, such as a macrophage.

Thus, in a further aspect the present invention relates to an allogeneic myeloid cell, such as an allogeneic macrophage for use in therapy, such as the treatment of cancer, in a human patient, wherein the allogeneic myeloid cell, such as the allogeneic macrophage, comprises at least one MHC-II complex which is identical with any one MHC-II complex of said human patient. This has the advantage that an antigen-specific response of a CD4+ T-cell of said human patient can be triggered. The invention also relates to an allogeneic myeloid cell, such as an allogeneic macrophage, for use in the treatment of cancer in a human patient, wherein the allogeneic macrophage comprises at least one protein complex selected from the group consisting of HLA-DR, HLA-DP and HLA-DQ, which protein complex is identical with any corresponding HLA complex of said human patient, preferably wherein the allogeneic myeloid cell, such as an allogeneic macrophage, comprises two protein complexes selected from said groups or even wherein the allogeneic myeloid cell, such as an allogeneic macrophage, comprises three complexes selected from said group. The invention also relates to an allogeneic myeloid cell, such as an allogeneic macrophage, for use in the treatment of cancer in a human patient, wherein the allogeneic myeloid cell, such as an allogeneic macrophage, comprises at least one HLA-DR protein complex which is identical with any corresponding HLA-DR complex of said human patient. The invention also relates to an allogeneic myeloid cell, such as an allogeneic macrophage, for use in the treatment of cancer in a human patient, wherein the allogeneic myeloid cell, such as an allogeneic macrophage, comprises at least one HLA-DP protein complex which is identical with any corresponding HLA-DP complex of said human patient. The invention also relates to an allogeneic myeloid cell, such as an allogeneic macrophage, for use in the treatment of cancer in a human patient, wherein the allogeneic myeloid cell, such as an allogeneic macrophage, comprises at least one HLA-DQ protein complex which is identical with any corresponding HLA-DQ complex of said human patient.

The (partial) MHC II match between the allogeneic myeloid cell, such as an allogeneic macrophage, and the patient to be treated can also be described at the genetic level. The present invention therefore also relates to an allogeneic myeloid cell, such as an allogeneic macrophage, for use in the treatment of cancer in a patient, wherein the allogeneic myeloid cell, such as an allogeneic macrophage, and the patient to be treated have at least one pair of alleles in common selected from the group consisting of the following pairs: HLA-DRA / HLA-DRB1, HLA-DRA / HLA-DRB3, HLA-DRA / HLA-DRB4, HLA-DRA / HLA-DRB5, HLA-DPA1 / HLA-DPB1 and HLA-DQA1 /HLA-DQB1, preferably wherein the allogeneic myeloid cell, such as an allogeneic macrophage, and the patient to be treated have two, three, four, five or even all six pairs of alleles in common. It should be noted that for heterozygous patients this means an overlap of one allele pair with one allele pair of the allogeneic myeloid cell, such as an allogeneic macrophage, but not necessarily a match of both patient allele pairs of the patient with both allele pairs of the allogeneic myeloid cell, such as an allogeneic macrophage, (but such a match of both allele pairs is, of course, included).

The principle of an at least partial MHC-II overlap as described above is relevant for all myeloid cells, such as macrophages, which are intended for cancer treatment, for example allogeneic macrophages which are polarized towards an M1-phenotype ex vivo prior to administration or genetically engineered human macrophages fitted out with CARs (CAR=chimeric antigen receptor), for example as described by Klichinsky et al., 2020, as discussed in the introduction. Preferably, however, the allogeneic human myeloid cell comprises at least one mutation in both alleles of a gene located on a chromosome, wherein the human macrophage is resistant to M-CSF induced M2-polarization.

Thus, the present invention also relates to an allogeneic human myeloid cell, such as an allogeneic macrophage, for use in the treatment of cancer in a human patient, wherein the allogeneic myeloid cell, such as the allogeneic macrophage, comprises at least one MHC-II complex which can trigger an antigen-specific response of a CD4+ T-cell of said human patient and/or wherein the allogeneic myeloid cell and the patient to be treated have at least one pair of alleles in common selected from the group consisting of the following pairs: HLA-DRA / HLA-DRB1, HLA-DRA / HLA-DRB3, HLA-DRA / HLA-DRB4, HLA-DRA / HLA-DRB5, HLA-DPA1 / HLA-DPB1 and HLA-DQA1 /HLA-DQB1,

And wherein the human myeloid cell, such as the human macrophage, is resistant to M-CSF induced M2-polarization and in particular wherein the human myeloid cell, such as the human macrophage, comprises a typical feature of a M1-macrophage after having been exposed to 50ng/ml M-CSF for 48 hours. The human myeloid cell, such as the human macrophage, can be genetically modified, for example by introduction of gene encoding a surface protein, such as a chimeric antigen receptor, but the human myeloid cell, such as the human macrophage, can also be a genetically unmodified myeloid cell, such as the human macrophage, for example derived from iPS cells, and having been polarized towards an M1 phenotype, for example by LPS or by interferon gamma.

The present invention also relates to a method for treating a human subject affected with cancer, which method comprises administering to a human subject an allogeneic human myeloid cell, such as the human macrophage, or a collection of allogeneic human myeloid cells, such as human macrophages, wherein the allogeneic myeloid cell, such as the human macrophage, comprises at least one MHC-II complex which can trigger a response of a CD4+ T-cell of said human patient and/or wherein the allogeneic myeloid cell, such as the human macrophage, and the patient to be treated have at least one pair of alleles in common selected from the group consisting of the following pairs: HLA-DRA/ HLA-DRB1, HLA-DRA/ HLA-DRB3, HLA-DRA/ HLA-DRB4, HLA-DRA/ HLA-DRB5, HLA-DPA1 / HLA-DPB1 and HLA-DQA1 /HLA-DQB1, in an amount sufficient to at least inhibit further growth of said cancer.

Preferred human myeloid cells for therapy are myeloid cells comprising at least one mutation in both alleles of a gene located on a chromosome, wherein the macrophage is resistant to M-CSF induced M2-polarization. Macrophages of the invention are anti-tumorigenic and thus useful for macrophage cell-therapy, in particular for anti-cancer therapy.

In particular, the human myeloid cell for therapy of the invention is resistant to M2-polarization by a combination of M-CSF and IL-4 and/or even a combination of M-CSF and IL-4 and IL-13.

Resistant to M2-polarization means for example that the allogeneic human myeloid cell, such as the allogeneic human macrophage, for therapy of the invention comprises a typical feature of a M1-macrophage even after having been exposed M-CSF and/or IL-4 and/or IL-13 for 24 hours, in particular for 48 hours, such as for 72 hours or more. For example, the allogeneic human myeloid cell, such as the allogeneic human macrophage, can be exposed to 50ng/ml M-CSF and/or 20ng/ml IL-4 for 24 hours, or even, for example to 50ng/ml M-CSF and/or 20ng/ml IL-4 for 48 hours, such as for 72 hours, and still comprise at least one typical feature of a M1-macrophage. Preferred allogeneic human myeloid cells, such as the allogeneic human macrophages, for therapy of the present invention still display at least one, such as at least 2, 3, 4 or even 5, typical feature(s) of an M1-macrophage after having been exposed to a combination of 50ng/ml M-CSF and 40ng/ml IL-4 and 20ng/ml IL-13 for 24 hours, and in particular after having been exposed for 48 hours, or even for 72 hours.

The human allogeneic human myeloid cell, such as the allogeneic human macrophage, for therapy of the invention can be characterized by their gene expression profile. A typical feature of a M1-macrophage can be, for example, expression of the HLA-DRA gene. In particular, the expression level of HLA-DRA mRNA in the human myeloid cell, for example the human macrophage, comprising at least one mutation in both alleles of a gene located on a chromosome, is higher, such as at least 4-fold, for example at least 8-fold, such as at least 16- or even at least 32-fold higher than the expression of HLA-DRA mRNA in an otherwise identical wildtype myeloid cell, for example the human macrophage, tested under otherwise equivalent cultivation conditions.

Alternatively, or additionally, a typical feature of a M1-macrophage can be, for example, expression of the HLA-DRB5 gene. In particular, the expression level of HLA-DRB5 mRNA in the human myeloid cell, for example the human macrophage, comprising at least one mutation in both alleles of a gene located on a chromosome, is higher, such as at least 4-fold, for example at least 8-fold, such as at least 16-, or even at least 32-fold higher than the expression of HLA-DRB5 mRNA in an otherwise identical wildtype myeloid cell, for example the human macrophage, tested under otherwise equivalent cultivation conditions.

Alternatively, or additionally, a typical feature of a M1-macrophage can be, for example, expression of the HLA-DPA1 gene. In particular, the expression level of HLA-DPA1 mRNA in the human myeloid cell, for example the human macrophage, comprising at least one mutation in both alleles of a gene located on a chromosome, is higher, such as at least 4-fold, for example at least 8-fold, such as at least 16-, or even at least 32--fold higher than the expression of HLA-DPA1 mRNA in an otherwise identical wildtype myeloid cell, for example the human macrophage, tested under otherwise equivalent cultivation conditions.

Alternatively, or additionally, a typical feature of a M1-macrophage can be, for example, expression of the HLA-DQA1 gene. In particular, the expression level of HLA-DQA1 mRNA in the human myeloid cell, for example the human macrophage, comprising at least one mutation in both alleles of a gene located on a chromosome, is higher, such as at least 4-fold, for example at least 8-fold, such as at least 16-, or even at least 32-fold higher than the expression of HLA-DQA1 mRNA in an otherwise identical wildtype myeloid cell, for example the human macrophage, tested under otherwise equivalent cultivation conditions.

Alternatively, or additionally, a typical feature of a M1-macrophage can be, for example, expression of the RXFP2 gene. In particular, the expression level of RXFP2 mRNA in the human myeloid cell, for example the human macrophage, comprising at least one mutation in both alleles of a gene located on a chromosome, is higher, such as at least 4-fold, for example at least 8-fold, such as at least 16-, or even at least 32-fold higher than the expression of RXFP2 mRNA in an otherwise identical wildtype myeloid cell, for example the human macrophage, tested under otherwise equivalent cultivation conditions.

Alternatively, or additionally, a typical feature of a M1-macrophage can be, for example, expression of the CD74 gene. In particular, the expression level of CD74 mRNA in the human myeloid cell, for example the human macrophage, comprising at least one mutation in both alleles of a gene located on a chromosome, is higher, such as at least 4-fold, for example at least 8-fold, or even at least 16-fold higher than the expression of CD74 mRNA in an otherwise identical wildtype myeloid cell, for example the human macrophage, tested under otherwise equivalent cultivation conditions.

Alternatively, or additionally, a typical feature of a M1-macrophage can be, for example, expression of the CD70 gene. In particular, the expression level of CD70 mRNA in the human myeloid cell, for example the human macrophage, comprising at least one mutation in both alleles of a gene located on a chromosome, is higher, such as at least 4-fold, for example at least 8-fold, or even at least 16-fold higher than the expression of CD70 mRNA in an otherwise identical wildtype myeloid cell, for example the human macrophage, tested under otherwise equivalent cultivation conditions.

Alternatively, or additionally, a typical feature of a M1-macrophage can be, for example, expression of the CD38 gene. In particular, the expression level of CD38 mRNA in the human myeloid cell, for example the human macrophage, comprising at least one mutation in both alleles of a gene located on a chromosome, is higher, such as at least 4-fold, for example at least 8-fold, or even at least 16-fold higher than the expression of CD38 mRNA in an otherwise identical wildtype myeloid cell, for example the human macrophage, tested under otherwise equivalent cultivation conditions.

Alternatively, or additionally, a typical feature of a M1-macrophage can be, for example, expression of the IL15 gene. In particular, the expression level of IL15 mRNA in the human myeloid cell, for example the human macrophage, comprising at least one mutation in both alleles of a gene located on a chromosome, is higher, such as at least 2-fold, for example at least 2.5-fold higher than the expression of IL15 mRNA in an otherwise identical wildtype myeloid cell, for example the human macrophage, tested under otherwise equivalent cultivation conditions.

Alternatively, or additionally, a typical feature of a M1-macrophage can be, for example, expression of the IL18 gene. In particular, the expression level of IL18 mRNA in the human myeloid cell, for example the human macrophage, comprising at least one mutation in both alleles of a gene located on a chromosome, is higher, such as at least 4-fold, for example at least 6-fold higher than the expression of IL18 mRNA in an otherwise identical wildtype myeloid cell, for example the human macrophage, tested under otherwise equivalent cultivation conditions.

Alternatively, or additionally, a typical feature of a M1-macrophage can be, for example, expression of the IL23A gene. In particular, the expression level of IL23A mRNA in the human myeloid cell, for example the human macrophage, comprising at least one mutation in both alleles of a gene located on a chromosome, is higher, such as at least 4-fold, for example at least 6-fold higher than the expression of IL23A mRNA in an otherwise identical wildtype myeloid cell, for example the human macrophage, tested under otherwise equivalent cultivation conditions.

Alternatively, or additionally, a typical feature of a M1-macrophage can be, for example, expression of the APOBEC3A gene. In particular, the expression level of APOBEC3A mRNA in the human myeloid cell, for example the human macrophage, comprising at least one mutation in both alleles of a gene located on a chromosome, is higher, such as at least 4-fold, for example at least 5-fold higher than the expression of APOBEC3A mRNA in an otherwise identical wildtype myeloid cell, for example the human macrophage, tested under otherwise equivalent cultivation conditions.

A preferred typical feature of a human macrophage of the invention is expression of the HLA-DRA gene, the HLA-DRB5 gene, the HLA-DPA1 gene, the HLA-DPB1 gene, the HLA-DQA1 gene and the HLA-DQB1 gene. In particular, the expression level of every one of the mRNAs corresponding to the above mentioned genes in the human myeloid cell, for example the human macrophage, comprising at least one mutation in both alleles of a gene located on a chromosome, is higher, such as at least 4-fold, for example at least 8-fold, such as at least 16-, or even at least 32-fold higher than the expression of the mRNAs an otherwise identical wildtype myeloid cell, for example the human macrophage, tested under otherwise equivalent cultivation conditions.

A preferred typical feature of a human macrophage of the invention is expression of the HLA-DRA gene, the HLA-DRB5 gene, the HLA-DPA1 gene, the HLA-DPB1 gene, the HLA-DQA1 gene, the HLA-DQB1 gene, the RXFP2 gene and the CD74 gene. In particular, the expression level of every one of the mRNAs corresponding to the above-mentioned genes in the human myeloid cell, for example the human macrophage, comprising at least one mutation in both alleles of a gene located on a chromosome, is higher, such as at least 4-fold, for example at least 8-fold, such as at least 16-fold higher than the expression of the mRNAs an otherwise identical wildtype myeloid cell, for example the human macrophage, tested under otherwise equivalent cultivation condition.

Alternatively, or additionally, a typical feature of a M1-macrophage can be, for example, downregulated expression of the RNASE1 gene. In particular, the expression level of RNASE1 mRNA in the human myeloid cell, for example the human macrophage, comprising at least one mutation in both alleles of a gene located on a chromosome, is lower, such as at least 4-fold, for example at least 8-fold, such as at least 16-, at least 32- or even at least 64-fold lower than the expression of RNASE1 mRNA in an otherwise identical wildtype myeloid cell, for example the human macrophage, tested under otherwise equivalent cultivation conditions. 64-fold lower means that the number of copies of RNASE1 mRNA in the human myeloid cell, for example the human macrophage, of the invention is only 1/64^{th} of the number of copies of RNASE1 mRNA in the corresponding wt myeloid cell, for example the human macrophage.

Alternatively, or additionally, a typical feature of a M1-macrophage can be, for example, downregulated expression of the CD28 gene. In particular, the expression level of CD28 mRNA in the human myeloid cell, for example the human macrophage, comprising at least one mutation in both alleles of a gene located on a chromosome, is lower, such as at least 4-fold, for example at least 8-fold, such as at least 16-, at least 32- or even at least 64-fold lower than the expression of CD28 mRNA in an otherwise identical wildtype myeloid cell, for example the human macrophage, tested under otherwise equivalent cultivation conditions.

Alternatively, or additionally, a typical feature of a M1-macrophage can be, for example, downregulated expression of the LYVE1 gene. In particular, the expression level of LYVE1 mRNA in the human myeloid cell, for example the human macrophage, comprising at least one mutation in both alleles of a gene located on a chromosome, is lower, such as at least 4-fold, for example at least 8-fold, such as at least 16-, at least 32- or even at least 64-fold lower than the expression of LYVE1 mRNA in an otherwise identical wildtype myeloid cell, for example the human macrophage, tested under otherwise equivalent cultivation conditions.

Alternatively, or additionally, a typical feature of a M1-macrophage can be, for example, downregulated expression of the C5AR1 gene. In particular, the expression level of C5AR1 mRNA in the human myeloid cell, for example the human macrophage, comprising at least one mutation in both alleles of a gene located on a chromosome, is lower, such as at least 4-fold, for example at least 8-fold, such as at least 16- or even at least 25-fold lower than the expression of C5AR1 mRNA in an otherwise identical wildtype myeloid cell, for example the human macrophage, tested under otherwise equivalent cultivation conditions.

Alternatively, or additionally, a typical feature of a M1-macrophage can be, for example, downregulated expression of the FCGBP gene. In particular, the expression level of FCGBP mRNA in the human myeloid cell, for example the human macrophage, comprising at least one mutation in both alleles of a gene located on a chromosome, is lower, such as at least 4-fold, for example at least 8-fold, such as at least 16-, at least 32- or even at least 64-fold lower than the expression of FCGBP mRNA in an otherwise identical wildtype myeloid cell, for example the human macrophage, tested under otherwise equivalent cultivation conditions.

Alternatively, or additionally, a typical feature of a M1-macrophage can be, for example, downregulated expression of the F13A1 gene. In particular, the expression level of F13A1 mRNA in the human myeloid cell, for example the human macrophage, comprising at least one mutation in both alleles of a gene located on a chromosome, is lower, such as at least 4-fold, for example at least 8-fold, such as at least 16-, at least 32- or even at least 64-fold lower than the expression of F13A1 mRNA in an otherwise identical wildtype myeloid cell, for example the human macrophage, tested under otherwise equivalent cultivation conditions.

Alternatively, or additionally, a typical feature of a M1-macrophage can be, for example, downregulated expression of the QPCT gene. In particular, the expression level of QPCT mRNA in the human myeloid cell, for example the human macrophage, comprising at least one mutation in both alleles of a gene located on a chromosome, is lower, such as at least 4-fold, for example at least 8-fold, such as at least 16-, at least 32- or even at least 64-fold lower than the expression of QPCT mRNA in an otherwise identical wildtype myeloid cell, for example the human macrophage, tested under otherwise equivalent cultivation conditions.

Alternatively, or additionally, a typical feature of a M1-macrophage can be, for example, downregulated expression of the DUSP6 gene. In particular, the expression level of DUSP6 mRNA in the human myeloid cell, for example the human macrophage, comprising at least one mutation in both alleles of a gene located on a chromosome, is lower, such as at least 4-fold, for example at least 6-fold lower than the expression of DUSP6 mRNA in an otherwise identical wildtype myeloid cell, for example the human macrophage, tested under otherwise equivalent cultivation conditions.

Alternatively, or additionally, a typical feature of a M1-macrophage can be, for example, downregulated expression of the CCL7 gene. In particular, the expression level of CCL7 mRNA in the human myeloid cell, for example the human macrophage, comprising at least one mutation in both alleles of a gene located on a chromosome, is lower, such as at least 4-fold, for example at least 8-fold, such as at least 16- or even at least 32-fold lower than the expression of CCL7 mRNA in an otherwise identical wildtype myeloid cell, for example the human macrophage, tested under otherwise equivalent cultivation conditions.

Alternatively, or additionally, a typical feature of a M1-macrophage can be, for example, downregulated expression of the RNF128 gene. In particular, the expression level of RNF128 mRNA in the human myeloid cell, for example the human macrophage, comprising at least one mutation in both alleles of a gene located on a chromosome, is lower, such as at least 4-fold, for example at least 8-fold, such as at least 16-, at least 32- or even at least 64-fold lower than the expression of RNF128 mRNA in an otherwise identical wildtype myeloid cell, for example the human macrophage, tested under otherwise equivalent cultivation conditions.

Alternatively, or additionally, a typical feature of a M1-macrophage can be, for example, downregulated expression of the IL10 gene. In particular, the expression level of IL10 mRNA in the human myeloid cell, for example the human macrophage, comprising at least one mutation in both alleles of a gene located on a chromosome, is lower, such as at least 4-fold, for example at least 8-fold, such as at least 16-, at least 32- or even at least 64-fold lower than the expression of IL10 mRNA in an otherwise identical wildtype myeloid cell, for example the human macrophage, tested under otherwise equivalent cultivation conditions.

A preferred typical feature of a human macrophage of the invention is downregulated expression of the RNASE1 gene, the CD28 gene and the LYVE1 gene. In particular, the expression level of every one of the mRNAs corresponding to the above-mentioned genes in the human myeloid cell, for example the human macrophage, comprising at least one mutation in both alleles of a gene located on a chromosome, is lower, such as at least 4-fold, for example at least 8-fold, such as at least 16-, at least 32- or even at least 64-fold lower than the expression of the mRNAs an otherwise identical wildtype myeloid cell, for example the human macrophage, tested under otherwise equivalent cultivation conditions.

Another preferred typical feature of a human macrophage of the invention is upregulated expression of the HLA-DRA gene, the HLA-DRB5 gene, the HLA-DPA1 gene, the HLA-DPB1 gene, the HLA-DQA1 gene and the HLA-DQB1 gene on the one hand and downregulated expression of the RNASE1 gene, the CD28 gene and the LYVE1 gene on the other hand. In particular, the expression level of every one of the mRNAs corresponding to the above mentioned upregulated genes in the human myeloid cell, for example the human macrophage, comprising at least one mutation in both alleles of a gene located on a chromosome, is at least 4-fold higher, for example at least 8-fold, such as at least 16-, or even at least 32-fold higher than the expression of the mRNAs an otherwise identical wildtype myeloid cell, for example the human macrophage, tested under otherwise equivalent cultivation conditions, while the expression level of every one of the mRNAs corresponding to the above-mentioned downregulated genes in the human myeloid cell, for example the human macrophage, comprising at least one mutation in both alleles of a gene located on a chromosome, is at least 4-fold lower, for example at least 8-fold, such as at least 16-, at least 32- or even at least 64-fold lower than the expression of the mRNAs an otherwise identical wildtype myeloid cell, for example the human macrophage, tested under otherwise equivalent cultivation conditions.

Other preferred typical features of a human myeloid cell, for example the human macrophage, for therapy of the invention are upregulated expression by at least 10-fold (compared to the expression of the respective mRNAs in an otherwise identical wildtype myeloid cell, for example the human macrophage, tested under otherwise equivalent cultivation conditions) of the following combinations of genes: combination A) HLA-DRA and RXFP2, B) HLA-DRA and CD74, C) HLA-DOA and RXFP2, D) HLA-DOA and CD74, E) HLA-DPA1 and RXFP2, F) HLA-DPA1 and CD74, G) HLA-DRA and RXFP2 and CD74, H) HLA-DOA and RXFP2 and CD74, and combination I) HLA-DPA1 and RXFP2 and CD74.

Other preferred typical features of a human myeloid cell, for example the human macrophage, for therapy of the invention are downregulated expression by at least 50-fold (compared to the expression of the respective mRNAs in an otherwise identical wildtype myeloid cell, for example the human macrophage, tested under otherwise equivalent cultivation conditions) of the following combinations of genes: combination m) RNASE1 and LYVE1, n) RNASE1 and FCGBP, o) RNASE1 and CD28, p) RNASE1 and F13A1, q) RNASE1 and RNF128, r) LYVE1 and CD28, s) LYVE1 and FCGBP, t) LYVE1 and F13A1, u) LYVE1 and RNF128, v) CD28 and F13A1, and combination w) CD28 and RNF128.

Other preferred typical features of a human myeloid cell, for example the human macrophage, for therapy of the invention are upregulated expression of one combination of genes and downregulation of one other combination of genes, as described by the following combined combinations: Am (A and m as described directly above; that is upregulated expression by at least 10-fold of both HLA-DRA and RXFP2 (A) combined with downregulated expression by at least 50-fold of both RNASE1 and LYVE1(m)), An, Ao, Ap, Aq, Ar, As, At, Au, Av, Aw, Bm, Bn, Bo, Bp, Br, Bq, Bs, Bt, Bu, Bv, Bw, Vm, Cn, Co, Cp, Cq, Cr, Cs, Ct, Cu, Cv, Cw, Dm, Dn, Do, Dp, Dq, Dr, Ds, Dt, Du, Dv, Dw, Em, En, Eo, Ep, Eq, Er, Es, Et, Eu, Ev, Ew, Fm, Fn, Fo, Fp, Fq, Fr, Fs, Ft, Fu, Fv, Fw, Gm, Gn, Go, Gp, Gq, Hr, Hs, Ht, Hu, Hv, Hw, Im, In, Io, Ip, Iq, Ir, Is, It, lu, Iv, and Iw.

The human myeloid cell, for example the human macrophage, for therapy of the invention can also be characterized by their secretion profile of cytokines and/or enzymes into the culture medium. Alternatively, or additionally, a typical feature of a M1-Macrophage can be, for example, secretion of IL23A. In particular, the secretion of IL23A by the human myeloid cell, for example the human macrophage, comprising at least one mutation in both alleles of a gene located on a chromosome, is higher, such as at least 3-fold, for example at least 4-fold, such as at least 5-, 6- or even 8-fold higher than the secretion of IL23A by an otherwise identical wildtype myeloid cell, for example the human macrophage, tested under otherwise equivalent cultivation conditions.

Alternatively, or additionally a typical feature of a M1-Macrophage can be, for example, secretion of IL18. In particular, the secretion of IL18 by the human myeloid cell, for example the human macrophage, comprising at least one mutation in both alleles of a gene located on a chromosome, is higher, such as at least 3-fold, for example at least 4-fold, such as at least 5-, 6- or even 8-fold higher than the secretion of IL18 by an otherwise identical wildtype myeloid cell, for example the human macrophage, tested under otherwise equivalent cultivation conditions.

Alternatively, or additionally a typical feature of a M1-Macrophage can be, for example, secretion of IL15. In particular, the secretion of IL15 by the human myeloid cell, for example the human macrophage, comprising at least one mutation in both alleles of a gene located on a chromosome, is higher, such as at least 2-fold, for example at least 3-fold, such as at least 4-fold higher than the secretion of IL15 by an otherwise identical wildtype myeloid cell, for example the human macrophage, tested under otherwise equivalent cultivation conditions.

Alternatively, or additionally a typical feature of a M1-Macrophage can be, for example, downregulated secretion of RNASE1. In particular, the secretion of RNASE1 by the human myeloid cell, for example the human macrophage, comprising at least one mutation in both alleles of a gene located on a chromosome, is lower, such as at least 3-fold, for example at least 5-fold, such as at least 7-, 10- or even 30-fold lower than the secretion of RNASE1 by an otherwise identical wildtype myeloid cell, for example the human macrophage, tested under otherwise equivalent cultivation conditions.

Alternatively, or additionally a typical feature of a M1-Macrophage can be, for example, downregulated secretion of FCGBP. In particular, the secretion of FCGBP by the human myeloid cell, for example the human macrophage, comprising at least one mutation in both alleles of a gene located on a chromosome, is lower, such as at least 3-fold, for example at least 5-fold, such as at least 7-, 10- or even 30-fold lower than the secretion of FCGBP by an otherwise identical wildtype myeloid cell, for example the human macrophage, tested under otherwise equivalent cultivation conditions.

Alternatively, or additionally a typical feature of a M1-Macrophage can be, for example, downregulated secretion of CCL7. In particular, the secretion of CCL7 by the human myeloid cell, for example the human macrophage, comprising at least one mutation in both alleles of a gene located on a chromosome, is lower, such as at least 3-fold, for example at least 5-fold, such as at least 7-, 10- or even 20-fold lower than the secretion of CCL7 by an otherwise identical wildtype myeloid cell, for example the human macrophage, tested under otherwise equivalent cultivation conditions.

Alternatively, or additionally a typical feature of a M1-Macrophage can be, for example, downregulated secretion of IL10. In particular, the secretion of IL10 by the human myeloid cell, for example the human macrophage, comprising at least one mutation in both alleles of a gene located on a chromosome, is lower, such as at least 3-fold, for example at least 5-fold, such as at least 7-, 10- or even 30-fold lower than the secretion of IL10 by an otherwise identical wildtype myeloid cell, for example the human macrophage, tested under otherwise equivalent cultivation conditions.

Another preferred typical feature of a human macrophage of the invention is therefore upregulated secretion of IL18 and/or IL23A on the one hand and downregulated secretion of RNASE1 and/or FCGBP and/or IL10 and/or CCL7.

The human myeloid cell, for example the human macrophage, for therapy of the invention may also be characterized by their cell surface markers. Alternatively, or additionally a typical feature of a M1-Macrophage can be, for example, the presence of the cell surface marker MHC II, or, more specifically, the presence of HLA class II proteins, such as a protein selected from the group consisting of HLA-DRA, HLA-DRB5, HLA-DPA1, HLA-DPB1, HLA-DQA1 and HLA-DQB1, and in particular HLA-DRA on the surface of the myeloid cell, for example the human macrophage, of the invention. In particular, the number of antibody-accessible MHC II proteins, such as HLA-DRA, on the cell surface of the human macrophage comprising at least one mutation in both alleles of a gene located on a chromosome, is higher, such as at least 3-fold, for example at least 4-fold, such as at least 5-, 8- or even 10-fold higher than the number of antigen-accessible MHC II proteins on the cell surface of an otherwise identical wildtype myeloid cell, for example the human macrophage, tested under otherwise equivalent cultivation and immunostaining conditions.

Alternatively, or additionally a typical feature of a M1-Macrophage can be, for example, the presence of the cell surface marker CD74. In particular, the number of CD74 on the cell surface of the human myeloid cell, for example the human macrophage, comprising at least one mutation in both alleles of a gene located on a chromosome, is higher, such as at least 3-fold, for example at least 4-fold, such as at least 5-, 6- or even 8-fold higher than the number of CD74 on the cell surface of an otherwise identical wildtype myeloid cell, for example the human macrophage, tested under otherwise equivalent cultivation and immunostaining conditions.

Alternatively, or additionally a typical feature of a M1-Macrophage can be, for example, the presence of the cell surface marker CD70. In particular, the number of CD70 on the cell surface of the human myeloid cell, for example the human macrophage, comprising at least one mutation in both alleles of a gene located on a chromosome, is higher, such as at least 3-fold, for example at least 4-fold, such as at least 5-, 6- or even 8-fold higher than the number of CD70 on the cell surface of an otherwise identical wildtype myeloid cell, for example the human macrophage, tested under otherwise equivalent cultivation and immunostaining conditions.

Alternatively, or additionally a typical feature of a M1-Macrophage can be, for example, the presence of the cell surface marker CD2. In particular, the number of CD2 on the cell surface of the human myeloid cell, for example the human macrophage, comprising at least one mutation in both alleles of a gene located on a chromosome, is higher, such as at least 3-fold, for example at least 4-fold, such as at least 5-, 6- or even 8-fold higher than the number of CD2 on the cell surface of an otherwise identical wildtype myeloid cell, for example the human macrophage, tested under otherwise equivalent cultivation and immunostaining conditions.

Alternatively, or additionally a typical feature of a M1-Macrophage can be, for example, the downregulation of the cell surface marker CD28. In particular, the number of CD28 on the cell surface of the human myeloid cell, for example the human macrophage, comprising at least one mutation in both alleles of a gene located on a chromosome, is lower, such as at least 2-fold, for example at least 4-fold, such as at least 6-, 8- or even 10-fold lower than the number of CD28 on the cell surface of an otherwise identical wildtype myeloid cell, for example the human macrophage, tested under otherwise equivalent cultivation and immunostaining conditions.

Alternatively, or additionally, a typical feature of a M1-Macrophage can be, for example, the downregulation of the cell surface marker LYVE1. In particular, the number of LYVE1 on the cell surface of the human myeloid cell, for example the human macrophage, comprising at least one mutation in both alleles of a gene located on a chromosome, is lower, such as at least 2-fold, for example at least 4-fold, such as at least 6-, 8- or even 10-fold lower than the number of LYVE1 on the cell surface of an otherwise identical wildtype myeloid cell, for example the human macrophage, tested under otherwise equivalent cultivation and immunostaining conditions.

Alternatively, or additionally a typical feature of a M1-Macrophage can be, for example, the downregulation of the cell surface marker STAB1. In particular, the number of STAB1 on the cell surface of the human myeloid cell, for example the human macrophage, comprising at least one mutation in both alleles of a gene located on a chromosome, is lower, such as at least 2-fold, for example at least 4-fold, such as at least 6-, 8- or even 10-fold lower than the number of STAB1 on the cell surface of an otherwise identical wildtype myeloid cell, for example the human macrophage, tested under otherwise equivalent cultivation and immunostaining conditions.

Alternatively, or additionally a typical feature of a M1-Macrophage can be, for example, the downregulation of the cell surface marker LILRB5. In particular, the number of LILRB5 on the cell surface of the human myeloid cell, for example the human macrophage, comprising at least one mutation in both alleles of a gene located on a chromosome, is lower, such as at least 2-fold, for example at least 4-fold, such as at least 6-, 8- or even 10-fold lower than the number of LILRB5 on the cell surface of an otherwise identical wildtype myeloid cell, for example the human macrophage, tested under otherwise equivalent cultivation and immunostaining conditions. For the purpose of the present invention, the strength of a signal in an immunostaining assay is taken to be representative for the number of cell surface markers of a particular type.

Alternatively, or additionally a typical feature of a M1-Macrophage can be, for example, the downregulation of the cell surface marker CD163. In particular, the number of CD163 on the cell surface of the human myeloid cell, for example the human macrophage, comprising at least one mutation in both alleles of a gene located on a chromosome, is lower, such as at least 2-fold, for example at least 4-fold, such as at least 6-, 8- or even 10-fold lower than the number of CD163 on the cell surface of an otherwise identical wildtype myeloid cell, for example the human macrophage, tested under otherwise equivalent cultivation and immunostaining conditions. For the purpose of the present invention, the strength of a signal in an immunostaining assay is taken to be representative for the number of cell surface markers of a particular type.

A particularly preferred human macrophage for therapy of the present invention is positive for at least one, preferably two, or even all three of the surface markers MHC II, CD70 and CD74, but negative for at least one, preferably two, such as three, four or even all five of the surface markers CD28, LYVE1, STAB1, CD163 and LILRB5.

Preferred combinations of surface markers are: positive for HLA-DR and CD74, but negative for LYVE1; positive for HLA-DR and CD70, but negative for LYVE1; positive for HLA-DR and CD74, but negative for CD28; positive for HLA-DR and CD70, but negative for CD28; positive for HLA-DR and CD74, but negative for CD163; positive for HLA-DR and CD70, but negative for CD163; positive for HLA-DR and CD70, but negative for STAB1; positive for HLA-DR and CD74, but negative for STAB1; positive for HLA-DR and CD70, but negative for LILRB5; positive for HLA-DR and CD74, but negative for LILRB5; negative for LYVE1 and STAB1, but positive for HLA-DR; negative for LYVE1 and STAB1, but positive for CD70; negative for LYVE1 and STAB1, but positive for CD74; negative for LYVE1 and CD163, but positive for HLA-DR; negative for LYVE1 and CD163, but positive for CD70; negative for LYVE1 and CD163, but positive for CD74; negative for LYVE1 and LILRB5, but positive for HLA-DR; negative for LYVE1 and LILRB5, but positive for CD74; negative for LYVE1 and LILRB5, but positive for CD70.

The human myeloid cell, for example the human macrophage, for therapy of the invention comprising at least one mutation in both alleles of a gene located on a chromosome can also be characterized by combinations of any one of the above described gene expression markers, in particular above described preferred gene expression markers, with the above described secretion profiles, in particular with above described preferred secretion profiles.

The human myeloid cell, for example the human macrophage, for therapy of the invention comprising at least one mutation in both alleles of a gene located on a chromosome can also be characterized by combinations of any one of the above described gene expression markers, in particular above described preferred gene expression markers, with the above described surface markers, in particular with above described preferred surface marker profiles.

The human myeloid cell, for example the human macrophage, for therapy of the invention comprising at least one mutation in both alleles of a gene located on a chromosome can also be characterized by combinations of any one of the above described secretion profiles, in particular above described preferred secretion profiles, with the above described surface markers, in particular with above described preferred surface marker profiles.

The human myeloid cell, for example the human macrophage, for therapy of the invention comprising at least one mutation in both alleles of a gene located on a chromosome can also be characterized by combinations of any one of the above described gene expression profiles, in particular above described preferred gene expression profiles, with the above described surface markers, in particular with above described preferred surface marker profiles, and with the above described secretion profiles, in particular with above described preferred secretion profiles.

The human myeloid cell, for example the human macrophage, for therapy of the invention comprises at least one mutation in both alleles of a gene located on a chromosome. The mutation can render the gene non-functional and/or it can inhibit gene expression or even abolish gene expression.

The mutation can be an insertion or a frameshift. However, a preferred mutation is a deletion, for example a deletion within a promotor, an exon or a splice site. While small deletions of only a few base pairs can have an effect on gene function, preferably the deletion is a deletion of at least 50 base pairs, such as at least 100 base pairs, at least 200 base pairs, at least 500 base pairs or even at least 1000 base pairs.

A preferred human myeloid cell, for example the human macrophage, for therapy of the invention comprises at least two different mutations, and preferably said two mutations are deletions within at least two different genes.

As explained above, the present invention preferably uses a myeloid cell, for example the human macrophage, comprising at least one mutation in both alleles of a gene located on a chromosome, wherein the macrophage is resistant to M-CSF induced M2-polarization, for the therapy of the invention. The present invention provides a new selectable phenotype for cells comprising at least one mutation in both alleles of a gene located on a chromosome, for which can be screened and thus yet unknown genes whose mutation renders the myeloid cell, for example the human macrophage, resistant to M-CSF induced M2-polarization can be identified. Thus, the nature of the gene is not particularly limiting, as long as biallelic mutation of the gene produces the desired phenotype. However, preferred human myeloid cell, for example the human macrophage, for therapy of the invention have biallelic mutations in genes which are involved in M-CSF, IL-4, IL-10 and/or TGFB1 mediated downregulation of C2TA, such as negative regulators, and in particular negative transcriptional regulators, of C2TA. Preferably the gene can be selected from the group consisting of STAT6, IRF4, PPARg, MAFB, MAF, KLF4, C/EPBb, GATA3, JMJD3, SOCS2, SOCS1, TMEM106A and AKT1, and in particular from the group consisting of STAT6, IRF4, TMEM106A, MAFB and MAF.

A preferred human myeloid cell, for example the human macrophage, comprising at least one mutation in both alleles of a gene located on a chromosome, wherein the myeloid cell, for example the human macrophage, is resistant to M-CSF induced M2-polarization, is a myeloid cell, for example the human macrophage, wherein said mutated gene is MAFB. It is preferred that MAFB is the only transcription factor-encoding gene comprising biallelic deletions. Alternatively it is preferred that MAFB is the only protein-coding gene comprising biallelic deletions. Alternatively it is preferred that MAFB is the only gene comprising biallelic deletions. If further transcription factors - other than MAF and/or MAFB - are deleted, myeloid cell, for example the human macrophage, function might be compromised or differentiation of induced pluripotent stem cells into macrophages might not be possible. For example Buchrieser et al. have shown that induced pluripotent stem cells having a deletion of the transcription factor RUNX1 or SPI1 cannot be differentiated into iPSC-derived macrophages.

The preferred human myeloid cell, for example the human macrophage, for therapy of the invention wherein said mutated gene is MAFB can also be characterized by the nature and size of the deletions. For example, all deletions can be in exonic parts of the MAFB. For example, the deletions can be larger than 50 base pairs each, such as at least 100 base pairs each or at least 250 base pairs each. For example, the deletions of or within the MAFB gene can be from 100 base pairs to 10000 base pairs each, such as from 500 base pairs to 3000 base pairs each, in particular from 700 base pairs to 2000 base pairs. The preferred human myeloid cell, for example the human macrophage, for therapy of the invention wherein said mutated gene of the invention is MAFB can show one or more, such as all, of these deletions.

The preferred human myeloid cell, for example the human macrophage, for therapy of the invention wherein said mutated gene is MAFB can also be characterized by the location of the deletion in a particular chromosome. For example, the MAFB deletions are preferred within the region on human chromosome 22 from 40685000 to 40690000, such as from 40685200 to 40689800, for example from 40685400 to 40689600, such as from 40685600 to 40689400 and in particular from 40685700 to 40689300.

A preferred human myeloid cell, for example the human macrophage, comprising at least one mutation in both alleles of a gene located on a chromosome, wherein the myeloid cell, for example the human macrophage, is resistant to M-CSF induced M2-polarization, is a myeloid cell, for example the human macrophage, wherein said mutated gene is MAF. It is preferred that MAF is the only transcription factor-encoding gene comprising biallelic deletions. Alternatively it is preferred that MAF is the only protein-coding gene comprising biallelic deletions. Alternatively it is preferred that MAF is the only gene comprising biallelic deletions.

The preferred human myeloid cell, for example the human macrophage, for therapy of the invention wherein said mutated gene is MAF can also be characterized by the nature and size of the deletions. For example, all deletions can be in exonic parts of the MAF. For example, the deletions can be larger than 50 base pairs each, such as at least 100 base pairs each or at least 250 base pairs each. For example, the deletions of or within the MAF gene can be from 100 base pairs to 10000 base pairs each, such as from 500 base pairs to 3000 base pairs each, in particular from 700 base pairs to 2000 base pairs. The preferred human myeloid cell, for example the human macrophage, for therapy of the invention wherein said mutated gene of the invention is MAF can show one or more, such as all, of these deletions.

The preferred human myeloid cell, for example the human macrophage, for therapy of the invention wherein said mutated gene is MAF can also be characterized by the location of the deletion in a particular chromosome. For example, the MAF deletions are preferred within the region on human chromosome 16 from 79593000 to 79602000, such as from 79593200 to 79601500, for example from 79593400 to 79601100, and in particular from 79593600 to 79600900.

A preferred human myeloid cell, for example the human macrophage, comprising at least one mutation in both alleles of a gene located on a chromosome, wherein the myeloid cell, for example the human macrophage, is resistant to M-CSF induced M2-polarization, is a myeloid cell, for example the human macrophage, wherein both alleles of MAFB and both alleles of MAF have been rendered nonfunctional, preferably by deletions of at least 50 base pairs. The present inventors have found that inhibition of MAFB and MAF gene expression in human cells has proven surprisingly more difficult than in murine cells, in part because a genetic approach to double-deficient cells is not an option for human cells. While the use of one guide RNA per gene in CRISPR/Cas9-based approaches has in the past provided many successful examples for inactivation of gene expression of other genes, for example Chu et al. (2016), several attempts to render both MAFB and MAF genes non-functional by creating small deletions with a CRISPR/Cas9 approach and one guide RNAs for each of MAFB and MAF did not yield cells that were observed to expand in vitro. Surprisingly, the use of two guide RNAs per gene, generating larger deletions of at least 50 base pairs, was successful in providing proliferating phagocytic cells. These cells had the additional advantage of being non-tumorigenic. It is preferred that MAF and MAFB are the only transcription factor-encoding genes comprising biallelic deletions. Alternatively it is preferred that MAF and MAFB are the only protein-coding genes comprising biallelic deletions. Alternatively it is preferred that MAF and MAFB are the only genes comprising biallelic deletions.

The preferred human myeloid cell, for example the human macrophage, of the invention wherein both alleles of MAFB and both alleles of MAF have been rendered nonfunctional can also be characterized by the nature and size of the deletions. For example, all deletions can be in exonic parts of the MAFB and MAF genes. For example, the deletions can be larger than 50 base pairs each, such as at least 100 base pairs each or at least 250 base pairs each. For example, the deletions of or within the MAFB and MAF genes can be from 100 base pairs to 10000 base pairs each, such as from 500 base pairs to 3000 base pairs each, in particular from 700 base pairs to 2000 base pairs. The human macrophage with stable M1-polarizationof the invention can show one or more, such as all, of these deletions.

The preferred human myeloid cell, for example the human macrophage, with stable M1-polarizationof the invention can also be characterized by the location of the deletion in a particular chromosome. For example, the MAFB deletions are preferred within the region on human chromosome 22 from 40685000 to 40690000, such as from 40685200 to 40689800, for example from 40685400 to 40689600, such as from 40685600 to 40689400 and in particular from 40685700 to 40689300. For example, the MAF deletions are preferred within the region on human chromosome 16 from 79593000 to 79602000, such as from 79593200 to 79601500, for example from 79593400 to 79601100, and in particular from 79593600 to 79600900.

The human myeloid cell, for example the human macrophage, of the invention, wherein the myeloid cell, for example the human macrophage, is resistant to M-CSF induced M2-polarization, may be prepared by manipulation of a cell. Cells, from which the human myeloid cell, for example the human macrophage, of the invention can be derived, can be cells which are progenitors for the development of human myeloid cell, for example the human macrophage,, for example cells selected from the group consisting of iPS cells; blood monocytes; monocytes, monoblasts, myeloid or CD34+ multipotent progenitors from cord blood; monocytes, monoblasts, myeloid or CD34+ multipotent progenitors from bone marrow; mobilized monoblasts, myeloid or CD34+ multipotent progenitors from adult blood; and monocytes, monoblasts, myeloid or CD34+ progenitors from extramedullary hematopoiesis. The human myeloid cell, for example the human macrophage, of the invention, wherein the myeloid cell, for example the human macrophage, is resistant to M-CSF induced M2-polarization, can also be derived from macrophages, in particular macrophages which are relatively easily accessible such as alveolar macrophages or macrophages from fat tissue. A preferred cell from which the human macrophage of the invention can be derived is a human iPS cell. Another preferred cell from which the human myeloid cell, for example the human macrophage, of the invention can be derived is a CMP (common myeloid progenitor). Another preferred cell from which the human myeloid cell, for example the human macrophage, of the invention can be derived is a GMP (granulocyte/macrophage progenitor).

The preparation of human induced pluripotent stem cells is by now well established in the art. Isogai et al. (2018), for example, describe the preparation of iPS cells from human blood monocytes. Fusaki N., et al. (2009) describe the generation of human iPS cell lines with a modified Sendai virus. In particular when human induced pluripotent stem cells are used as the starting material for myeloid cell, for example the human macrophage, differentiation it is preferred that MAF and/or MAFB is/are the only transcription factor-encoding gene(s) comprising biallelic deletions. Alternatively it is preferred that MAF and/or MAFB are the only protein-coding genes comprising biallelic deletions. Alternatively it is preferred that MAF and/or MAFB are the only genes comprising biallelic deletions. If further genes are deleted, macrophage function might be compromised or differentiation of induced pluripotent stem cells into macrophages might not be possible.

Human mononuclear phagocytes for the biallelic mutation of genes can also be derived from primary human blood monocytes, which can be obtained by leukapheresis and elutriation, or from monocyte derived macrophages, for which culture conditions are well known to the art. Human macrophages can also be obtained from bronchoalveolar lavage and by differentiation of CD34+ hematopoietic stem and progenitor cells obtained from umbilical cord blood, stem cell mobilized peripheral blood or bone marrow, using differentiation protocols well known to the art.

Cas9 and gRNAs can be expressed by publicly and commercially available DNA expression plasmids well known in the art (for example Santa Cruz Sc-418922, https://www.scbt.com/de/p/control-crispr-cas9-plasmid). DNA expression plasmids can be introduced into human mononuclear phagocyte target cells by electroporation or lipid-based transfection protocols well known to the art. Cas9 and gRNA can also be introduced into target cells as a ribonucleic/protein complex by electroporation. Cas9/gRNA mediated gene editing has been demonstrated in mononuclear phagocytes using such methods (Zhang et al. (2020); Wang et al. (2018); Freund et al (2020)) and in human CD34+ hematopoietic stem and progenitor cells differentiating to macrophages (Scharenberg et al. (2020)). Target genes, such as the MAFB and MAF genes, could also be deleted utilizing engineered site directed recombinases (Lansing et al. (2020); Karpinski et al. (2016)), which can be introduced by methods known to the art, such as electroporation of the protein, coding mRNA or DNA expression plasmids, and which have been used for gene editing in mononuclear phagocytes (Shi et al. (2018)).

Gonzalez F. et al. (2014) describe an iCRISPR platform for rapid, multiplexable and inducible genome editing in human pluripotent stem cells based on the introduction of a doxycycline-inducible Cas9 expression cassette into the AAVS1 locus. This strategy can be used to introduce an inducible Cas9-expression cassette into a wide variety of self-generated or publicly available human iPS cell lines. Such modified cell lines can then be used for targeting selected genes, such as MAF and/or MAFB.

The human macrophage wherein both MAF and MAFB have been rendered non-functional have been found to be non-tumorigenic. Karyotyping showed that they contain 46 chromosomes and not any one of those chromosomes shows a chromosome rearrangement. These cells are thus useful for macrophage-based cell therapy.

These markers can be maintained even in the presence of tumor cells in vitro.

Stem cells are useful intermediates and/or starting materials for the preparation of allogeneic MHC II matched myeloid cell for the therapeutic uses of the present invention.

Therefore the present invention also relates to an allogeneic human induced pluripotent stem cell or an allogeneic human embryonic stem cell or an allogenic hematopoietic stem cell or an allogenic multipotent progenitor cell or an an allogeneic human common myeloid progenitor or an allogeneic human granulocyte/macrophage progenitor -and in particular a human induced pluripotent stem cell - wherein the allogeneic above-mentioned cell, such as the allogeneic induced pluripotent stem cell, comprises at least one MHC-II complex which is identical with any one MHC-II complex of a human patient to be treated. This has the advantage that an antigen-specific response of a CD4+ T-cell of said human patient can be triggered. The invention also relates to an allogeneic stem or progenitor cell, such as an allogeneic induced pluripotent stem cell, for use in the preparation of an MHC II-matched allogeneic myeloid cell comprising at least one protein complex selected from the group consisting of HLA-DR, HLA-DP and HLA-DQ, which protein complex is identical with any corresponding HLA complex of said human patient, preferably wherein the allogeneic myeloid cell, such as an allogeneic macrophage, comprises two protein complexes selected from said groups or even wherein the allogeneic myeloid cell, such as an allogeneic macrophage, comprises three complexes selected from said group. The invention also relates to an allogeneic stem or progenitor cell, such as an allogeneic induced pluripotent stem cell, for use in the preparation of an MHC II-matched allogeneic myeloid cell an allogeneic myeloid cell, such as an allogeneic macrophage, wherein the allogeneic myeloid cell is for use in the treatment of cancer in a human patient, wherein the allogeneic myeloid cell, such as an allogeneic macrophage, comprises at least one HLA-DR protein complex which is identical with any corresponding HLA-DR complex of said human patient.

The invention also relates to the use of the human induced pluripotent stem cell or human embryonic stem cell or human common myeloid progenitor or human granulocyte/macrophage progenitor - and in particular the use of the human induced pluripotent stem cell - of the invention for the production of human MHC II-matched antigen-presenting cells, and in particular for the production of human MHC II-matched myeloid antigen presenting cells, such as macrophages or dendritic cells.

The invention further relates to the following embodiments
1. An allogeneic human myeloid cell for therapeutic use in a human patient, wherein the allogeneic myeloid cell comprises at least one MHC-II complex which is identical with any one MHC-II complex of said human patient.
2. The allogeneic human myeloid cell for therapeutic use according to 1, wherein the therapeutic use is the treatment of cancer.
3. The allogeneic human myeloid cell for therapeutic use according to any one of 1 or 2, wherein the myeloid cell is a macrophage, a dendritic cell or a monocyte.
4. The allogeneic human myeloid cell for use in the treatment of cancer in a human patient according to 2, wherein the allogeneic myeloid cell is an allogeneic macrophage and wherein the allogeneic macrophage comprises at least one MHC-II complex which is identical with any one MHC-II complex of said human patient.
5. The allogeneic human myeloid cell for therapeutic use according to any one of 1 to 4, wherein the allogeneic myeloid cell comprises at least one protein complex selected from the group consisting of HLA-DR, HLA-DP and HLA-DQ, which protein complex is identical with any corresponding HLA complex of said human patient.
6. The allogeneic human myeloid cell for therapeutic use according to any one of 1 to 5, wherein the allogeneic myeloid cell comprises two protein complexes selected from said group, preferably wherein the allogeneic macrophages comprises three complexes selected from said group.
7. The allogeneic human myeloid cell for therapeutic use according to any one of 1 to 6, wherein the allogeneic myeloid cell comprises at least one HLA-DR protein complex which is identical with any corresponding HLA-DR complex of said human patient.
8. The allogeneic human myeloid cell for therapeutic use according to any one of 1 to 7, wherein the allogeneic myeloid cell comprises at least one HLA-DP protein complex which is identical with any corresponding HLA-DP complex of said human patient.
9. The allogeneic human myeloid cell for therapeutic use according to any one of 1 to 8, wherein the allogeneic macrophage comprises at least one HLA-DQ protein complex which is identical with any corresponding HLA-DQ complex of said human patient.
10. An allogeneic human myeloid cell for therapeutic use in a patient, wherein the allogeneic myeloid cell and the patient to be treated have at least one pair of alleles in common selected from the group consisting of the following pairs: HLA-DRA / HLA-DRB1, HLA-DRA / HLA-DRB3, HLA-DRA / HLA-DRB4, HLA-DRA / HLA-DRB5, HLA-DPA1 / HLA-DPB1 and HLA-DQA1 /HLA-DQB1.
11. The allogeneic human myeloid cell for therapeutic use according to 10, wherein the therapeutic use is the treatment of cancer.
12. The allogeneic human myeloid cell for therapeutic use according to 11, wherein the cancer is a solid tumor.
13. The allogeneic human myeloid cell for therapeutic use according to any one of 10 to 12, wherein the myeloid cell is a macrophage, a monocyte or a dendritic cell, and in particular a macrophage.
14. The allogeneic human myeloid cell for therapeutic use according to any one of 10 to 13, wherein the allogeneic myeloid cell and the patient to be treated have two, three, four, five or even all six pairs of alleles in common.
15. The allogeneic human myeloid cell for therapeutic use according to any one of 1 to 14, wherein the human myeloid cell, and in particular the human macrophage, is resistant to M-CSF induced M2-polarization.
16. The allogeneic human myeloid cell for therapeutic use according to any one of 1 to 15, wherein the human myeloid cell, and in particular the human macrophage, comprises a typical feature of a M1-macrophage after having been exposed to 50ng/ml M-CSF for 48 hours.
17. The allogeneic human myeloid cell for therapeutic use according to any one of 1 to 16, wherein the human myeloid cell, and in particular the human macrophage, wherein the human myeloid cell is genetically modified.
18. The allogeneic human myeloid cell for therapeutic use according to 17, wherein the genetic modification is introduction of a gene encoding a surface protein, such as a chimeric antigen receptor.
19. The allogeneic human myeloid cell for therapeutic use according to any one of 1 to 16, wherein the human macrophage is a genetically unmodified macrophage.
20. The allogeneic human myeloid cell for therapeutic use according to any one of 1 to 19, wherein the human myeloid cell is derived from an iPS cell.
21. The allogeneic human myeloid cell, and in particular an allogeneic human macrophage for therapeutic use according to any one of 1 to 20, wherein the allogeneic human myeloid cell, such as the allogeneic human macrophage, is a myeloid cell according to any one of 22 to 114.
22. A human myeloid cell, preferably a macrophage, comprising at least one mutation in both alleles of a gene located on a chromosome, wherein the macrophage is resistant to M-CSF induced M2-polarization, preferably where the human macrophage is not a dendritic cell and more preferably wherein the human macrophage is not a dendritic cell and not a monocyte.
23. The human myeloid cell, preferably a macrophage, according to 22, wherein the myeloid cell, preferably the macrophage, is resistant to M2-polarization by a combination of M-CSF and IL-4.
24. The human myeloid cell, preferably a macrophage, according to any one of 22 to 23, wherein the macrophage is resistant to M2-polarization by a combination of M-CSF and IL-4 and IL-13.
25. The human myeloid cell, preferably a macrophage, according to any one of 22 to 24, wherein the myeloid cell comprises a typical feature of a M1-macrophage after having been exposed to 50ng/ml M-CSF for 24 hours.
26. The human myeloid cell, preferably a macrophage, according to 25, wherein the myeloid cell comprises a typical feature of a M1-macrophage after having been exposed to 50ng/ml M-CSF for 48 hours.
27. The human myeloid cell, preferably a macrophage, according to any one of 22 to 26, wherein the myeloid cell comprises a typical feature of a M1-macrophage after having been exposed to 20ng/ml IL-4 for 24 hours.
28. The human myeloid cell, preferably a macrophage, according to 27, wherein the myeloid cell comprises a typical feature of a M1-macrophage after having been exposed to 20ng/ml IL-4 for 48 hours.
29. The human myeloid cell, preferably a macrophage, according to any one of 22 to 28, wherein the myeloid cell comprises a typical feature of a M1-macrophage after having been exposed to a combination of 40ng/ml IL-4 and 50ng/ml M-CSF for 24 hours.
30. The human myeloid cell, preferably a macrophage, according to 29, wherein the myeloid cell comprises a typical feature of a M1-macrophage after having been exposed to a combination of 40ng/ml IL-4 and 50ng/ml M-CSF for 48 hours.
31. The human myeloid cell, preferably a macrophage, according to any one of 22 to 30, wherein the myeloid cell comprises a typical feature of a M1-macrophage, wherein GM-CSF was not present during the last 2 hours.
32. The human myeloid cell, preferably a macrophage, according to 31, wherein the myeloid cell comprises a typical feature of a M1-macrophage, wherein GM-CSF was not present during the last 6 hours.
33. The human myeloid cell, preferably a macrophage, according to any one of 25 to 32, wherein a typical feature of a M1-macrophage is at least 4-fold increased expression of at least one mRNA in the human myeloid cell comprising at least one mutation in both alleles of a gene located on a chromosome, in comparison to expression of the mRNA in an otherwise identical wildtype myeloid cell, wherein the at least one mRNA is selected from the list consisting of HLA-DRA, HLA-DRB5, HLA-DPA1, HLA-DQA1, RXFP2, CD74, CD38, CD2, IL18 and IL23A.
34. The human myeloid cell according to 33, wherein the expression of the at least one mRNA in the human myeloid cell comprising at least one mutation in both alleles of a gene located on a chromosome, is at least 6-fold higher in comparison to expression of the same mRNA in an otherwise identical wildtype myeloid cell.
35. The human myeloid cell according to any one of 25 to 32, wherein a typical feature of a M1-macrophage is at least 4-fold increased expression of at least three mRNAs in the human myeloid cell comprising at least one mutation in both alleles of a gene located on a chromosome, in comparison to expression of the same at least three mRNAs in an otherwise identical wildtype myeloid cell, wherein the at least three mRNAs are selected from the list consisting of HLA-DRA, HLA-DRB5, HLA-DPA1, HLA-DQA1, RXFP2, CD74, CD38, CD2, IL18 and IL23A.
36. The human myeloid cell according to 35, wherein the expression of the at least three mRNAs in the human myeloid cell comprising at least one mutation in both alleles of a gene located on a chromosome, is at least 6-fold higher in comparison to expression of the same at least three mRNAs in an otherwise identical wildtype myeloid cell.
37. The human myeloid cell according to any one of 25 to 32, wherein a typical feature of a M1-macrophage is at least 4-fold increased expression of at least six mRNAs in the human myeloid cell comprising at least one mutation in both alleles of a gene located on a chromosome, in comparison to expression of the same at least six mRNAs in an otherwise identical wildtype myeloid cell, wherein the at least six mRNAs are selected from the list consisting of HLA-DRA, HLA-DRB5, HLA-DPA1, HLA-DQA1, RXFP2, CD74, CD38, CD2, IL18 and IL23A.
38. The human myeloid cell according to 37, wherein the expression of the at least six mRNAs in the human myeloid cell comprising at least one mutation in both alleles of a gene located on a chromosome, is at least 6-fold higher in comparison to expression of the same at least six mRNAs in an otherwise identical wildtype myeloid cell.
39. The human myeloid cell according to any one of 33 to 38, wherein expression of HLA-DRB5 mRNA in the human myeloid cell comprising at least one mutation in both alleles of a gene located on a chromosome, is at least 4-fold, such as at least 8-fold, at least 16-fold or even at least 32-fold, higher in comparison to expression of HLA-DRB5 mRNA in an otherwise identical wildtype myeloid cell.
40. The human myeloid cell according to any one of 33 to 39, wherein the expression of HLA-DPA1 mRNA in the human myeloid cell comprising at least one mutation in both alleles of a gene located on a chromosome, is at least 4-fold higher, such as at least 8-fold, at least 16-fold or even at least 32-fold, in comparison to expression of HLA-DPA1 mRNA in an otherwise identical wildtype myeloid cell.
41. The human myeloid cell according to any one of 33 to 40, wherein the expression of HLA-DQA1 mRNA in the human myeloid cell comprising at least one mutation in both alleles of a gene located on a chromosome, is at least 4-fold higher, such as at least 8-fold, at least 16-fold or even at least 32-fold, in comparison to expression of HLA-DQA1 mRNA in an otherwise identical wildtype myeloid cell.
42. The human myeloid cell according to any one of 33 to 41, wherein the expression of HLA-DRA mRNA in the human myeloid cell comprising at least one mutation in both alleles of a gene located on a chromosome, is at least 4-fold higher, such as at least 8-fold, at least 16-fold or even at least 32-fold, in comparison to expression of HLA-DRA mRNA in an otherwise identical wildtype myeloid cell.
43. The human myeloid cell according to any one of 33 to 42, wherein a typical feature of a M1-myeloid cell is expression of the HLA-DRA gene, the HLA-DRB5 gene, the HLA-DPA1 gene, the HLA-DPB1 gene, the HLA-DQA1 gene and the HLA-DQB1 gene, and wherein expression of the HLA-DRA gene, the HLA-DRB5 gene, the HLA-DPA1 gene, the HLA-DPB1 gene, the HLA-DQA1 gene and the HLA-DQB1 gene is at least 4-fold higher such as at least 8-fold, at least 16-fold or even at least 32-fold, each in the human myeloid cell comprising at least one mutation in both alleles of a gene located on a chromosome, in comparison to expression of said mRNAs in an otherwise identical wildtype myeloid cell.
44. The human myeloid cell according to any one of 31 to 43, wherein a typical feature of a M1-macrophage is at least 10-fold decreased expression of at least one mRNA in the human myeloid cell comprising at least one mutation in both alleles of a gene located on a chromosome, in comparison to expression of the mRNA in an otherwise identical wildtype myeloid cell, wherein the at least one mRNA is selected from the list consisting of RNASE1, CD28, LYVE1, FCGBP, F13A1, QPCT, CCL7 and RNF128.
45. The human myeloid cell according to 44, wherein the expression of the at least one mRNA in the human myeloid cell comprising at least one mutation in both alleles of a gene located on a chromosome, is at least 50-fold lower in comparison to expression of the same mRNA in an otherwise identical wildtype myeloid cell.
46. The human myeloid cell according to any one of 31 to 45, wherein a typical feature of a M1-macrophage is at least 10-fold decreased expression of at least three mRNAs in the human myeloid cell comprising at least one mutation in both alleles of a gene located on a chromosome, in comparison to expression of the same at least three mRNAs in an otherwise identical wildtype myeloid cell, wherein the at least three mRNAs are selected from the list consisting of RNASE1, CD28, LYVE1, FCGBP, F13A1, QPCT, CCL7 and RNF128.
47. The human myeloid cell according to 46, wherein the expression of the at least three mRNAs in the human myeloid cell comprising at least one mutation in both alleles of a gene located on a chromosome, is at least 50-fold lower in comparison to expression of the same at least three mRNAs in an otherwise identical wildtype myeloid cell.
48. The human myeloid cell according to any one of 31 to 47, wherein a typical feature of a M1-macrophage is at least 10-fold decreased expression of at least six mRNAs in the human myeloid cell comprising at least one mutation in both alleles of a gene located on a chromosome, in comparison to expression of the same at least six mRNAs in an otherwise identical wildtype myeloid cell, wherein the at least six mRNAs are selected from the list consisting of RNASE1, CD28, LYVE1, FCGBP, F13A1, QPCT, CCL7 and RNF128.
49. The human myeloid cell according to any one of 31 to 48, wherein the typical feature of a M1-macrophage is downregulated secretion of RNASE1.
50. The human myeloid cell according to any one of 31 to 49, wherein the typical feature of a M1-macrophage is downregulated secretion of FCGBP.
51. The human myeloid cell according to any one of 22 to 50, wherein said myeloid cell is positive for the surface marker HLA-DRA.
52. The human myeloid cell according to any one of 22 to 51, wherein said myeloid cell is positive for the surface marker HLA-DPA1.
53. The human myeloid cell according to any one of 22 to 52, wherein said myeloid cell is positive for the surface marker HLA-DQA1.
54. The human myeloid cell according to any one of 22 to 53, wherein said myeloid cell is positive for the surface marker HLA-DRB5.
55. The human myeloid cell according to any one of 22 to 54, wherein said myeloid cell is positive for the surface marker CD74.
56. The human myeloid cell according to any one of 22 to 55, wherein said myeloid cell is positive for the surface marker CD2.
57. The human myeloid cell according to any one of 22 to 56, wherein said myeloid cell is negative for the surface marker CD28.
58. The human myeloid cell according to any one of 22 to 57, wherein said myeloid cell is negative for the surface marker LYVE1.
59. The human myeloid cell according to any one of 22 to 58, wherein said myeloid cell is negative for the surface marker STAB1.
60. The human myeloid cell according to any one of 22 to 59, wherein said myeloid cell is negative for the surface marker LILRB5.
61. The human myeloid cell according to any one of 22 to 60, wherein said myeloid cell is positive for the surface markers HLA-DR, but negative for the surface markers CD28 and LYVE1.
62. The human myeloid cell according to any one of 22 to 61, wherein said myeloid cell is positive for the surface markers HLA-DR and CD74, but negative for the surface markers CD28, LYVE1, STAB1 and LILRB5.
63. The human myeloid cell according to any one of 22 to 62, wherein said mutation renders the gene non-functional.
64. The human myeloid cell according to any one of 22 to 62, wherein said mutation inhibits gene expression.
65. The human myeloid cell according to any one of 22 to 62, wherein said mutation abolishes gene expression
66. The human myeloid cell according to any one of 22 to 65, wherein said mutation is a deletion.
67. The human myeloid cell according to 66, wherein said deletion is within a promotor, an exon or a splice site.
68. The human myeloid cell according to any one of 66 to 67, wherein said deletion is a deletion of at least 50 base pairs.
69. The human myeloid cell according to 22 to 68, comprising at least two different mutations.
70. The human myeloid cell according to 69 wherein said two mutations are deletions within at least two different genes.
71. The human myeloid cell according to any one of 22 to 70, wherein said gene is a gene involved in M-CSF, IL-4, IL-10 and/or TGFB1 mediated downregulation of C2TA.
72. The human myeloid cell according to any one of 22 to 71, wherein said gene is selected from the group consisting of STAT6, IRF4, PPARγ, MAFB, MAF, KLF4, C/EPBβ, GATA3, JMJD3, SOCS2, SOCS1 and AKT1.
73. The human myeloid cell according to any one of 22 to 72, wherein said gene encodes a negative regulator of C2TA transcription.
74. The human myeloid cell according to any one of 1 to 73, wherein said gene is MAFB.
75. The human myeloid cell according to 74, wherein said mutation is a mutation within the region on human chromosome 22 from 40685000 to 40690000.
76. The human myeloid cell according to 75, wherein said mutation is a mutation within the region on human chromosome 22 from 40685200 to 40689800, for example from 40685400 to 40689600, such as from 40685600 to 40689400 and in particular from 40685700 to 40689300.
77. The human myeloid cell according to any one of 74 to 76, wherein MAFB has been rendered nonfunctional by a deletion of at least 50 base pairs.
78. The human myeloid cell according to any one of 77, wherein the deletion is a deletion of at least 100 base pairs.
79. The human myeloid cell according to any one of 77 to 78, wherein the deletion is a deletion of at least 250 base pairs.
80. The human myeloid cell according to any one of 77 to 79, wherein the deletion is a deletion of from 100 base pairs to 10000 base pairs.
81. The human myeloid cell according to 80, wherein the deletion is a deletion of from 500 base pairs to 3000 base pairs, such as from 600 base pairs to 1500 base pairs.
82. The human myeloid cell according to any one of 74 to 81, further comprising a mutation in the gene MAF.
83. The human myeloid cell according to 82, wherein the mutation in MAF is a deletion.
84. The human myeloid cell according to any one of 1 to 73, wherein said gene is MAF.
85. The human myeloid cell according to 84, wherein said mutation is a mutation within the region on human chromosome 16 from 79593000 to 79602000.
86. The human myeloid cell according to 85, wherein said mutation is a mutation within the region on human chromosome 16 from 79593200 to 79601500, for example from 79593400 to 79601100, and in particular from 79593600 to 79600900.
87. The human myeloid cell according to any one of 84 to 86, wherein MAF has been rendered nonfunctional by a deletion of at least 50 base pairs.
88. The human myeloid cell according to 87, wherein the deletion is a deletion of at least 100 base pairs.
89. The human myeloid cell according to any one of 87 to 88, wherein the deletion is a deletion of at least 250 base pairs.
90. The human myeloid cell according to any one of 88 to 89, wherein the deletion is a deletion of from 100 base pairs to 10000 base pairs.
91. The human myeloid cell according to 90, wherein the deletion is a deletion of from 500 base pairs to 3000 base pairs, such as from 600 base pairs to 1500 base pairs.
92. The human myeloid cell according to any one of 84 to 91, further comprising a mutation in the gene MAFB.
93. The human myeloid cell according to 92, wherein the mutation in MAFB is a deletion.
94. The human myeloid cell according to 69, wherein said two genes are MAFB and MAF.
95. The human myeloid cell according to 94, wherein both alleles of MAFB and both alleles of MAF have been rendered nonfunctional by deletions of at least 50 base pairs.
96. The human myeloid cell according to 95, wherein all deletions comprise exonic DNA.
97. The human myeloid cell according to any one of 94 to 96, wherein the deletions are at least 100 base pairs each.
98. The human myeloid cell according to any one of 94 to 96, wherein the deletions are at least 250 base pairs each.
99. The human myeloid cell according to any one of 94 to 98, wherein the deletions are from 100 base pairs to 10000 base pairs each.
100. The human myeloid cell according to any one of 94 to 99, wherein the deletions are from 500 base pairs to 3000 base pairs, such as from 600 base pairs to 1500 base pairs.
101. The human myeloid cell according to any one of 94 to 100, wherein the MAFB deletions are within the region on human chromosome 22 from 40685000 to 40690000.
102. The human myeloid cell according to 101, wherein the MAFB deletions are within the region on human chromosome 22 from 40685200 to 40689800, for example from 40685400 to 40689600, such as from 40685600 to 40689400 and in particular from 40685700 to 40689300.
103. The human myeloid cell according to any one of 94 to 102, wherein the MAF deletions are within the region on human chromosome 16 from 79593000 to 79602000.
104. The human myeloid cell according to 103, wherein the MAF deletions are within the region on human chromosome 16 from 79593200 to 79601500, for example from 79593400 to 79601100, and in particular from 79593600 to 79600900.
105. The human myeloid cell according to any one of 1 to 104, wherein the myeloid cell is non-tumorigenic.
106. The human myeloid cell according to any one of 1 to 105, wherein the myeloid cell has 46 chromosomes.
107. The human myeloid cell according to any one of 1 to 106, wherein the myeloid cell does not contain a chromosome with a chromosome rearrangement.
108. The human myeloid cell according to any one of 1 to 107, wherein the myeloid cell maintains the expression of at least two, such as at least four, markers of an M1-phenotype in the presence of tumor cells in vitro.
109. The human myeloid cell according to any one of 1 to 108, wherein said gene located on a chromosome comprising biallelic deletions is the only gene comprising biallelic deletions.
110. The human myeloid cell according to any one of 1 to 109, wherein said gene located on a chromosome comprising biallelic deletions is the only protein coding gene comprising biallelic deletions.
111. The human myeloid cell according to any one of 1 to 110, wherein said gene located on a chromosome comprising biallelic deletions is the transcription-factor encoding gene comprising biallelic deletions.
112. The human myeloid cell according to any one of 1 to 111, wherein MAF and/or MAFB is/are the only transcription factor-encoding gene(s) comprising biallelic deletions.
113. The human myeloid cell according to any one of 1 to 112, wherein MAF and/or MAFB is/are the only protein-coding gene(s) comprising biallelic deletions.
114. The human myeloid cell according to any one of 1 to 113, wherein MAF and/or MAFB is/are the only gene(s) comprising biallelic deletions.

The practice of the present invention employs, unless otherwise indicated, conventional techniques of molecular biology (including recombinant techniques), microbiology, cell biology, biochemistry and immunology, which are well within the purview of the skilled artisan. Such techniques are explained fully in the literature, such as, "Molecular Cloning: A Laboratory Manual", fourth edition (Sambrook, 2012); "Handbook of Experimental Immunology" (Weir, 1997); "Short Protocols in Molecular Biology" (Ausubel, 2002); "Polymerase Chain Reaction: Principles, Applications and Troubleshooting", (Babar, 2011); "Current Protocols in Immunology" (Coligan, 2002). These techniques may be considered in making and practicing the invention.

Those skilled in the art will appreciate that the invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended Claims, and all changes which come within the meaning and range of equivalency are intended to be embraced therein.

Various references are cited throughout this specification, each of which is incorporated herein by reference in its entirety.

The foregoing description will be more fully understood with reference to the following Examples. Such Examples, are, however, exemplary of methods of practicing the present invention and are not intended to limit the scope of the invention.

### EXAMPLES

Aziz et al., 2009 have shown that the combined deficiency of MafB and c-Maf does not affect hematopoiesis in mice, as all the lineages could be generated including monocytes and macrophages (Aziz et al., 2009). Instead, it enables extended expansion of mature mouse macrophages without loss of differentiated phenotype or function (Aziz et al., 2009).

We investigated the role of Maf-DKO macrophages in the ID8 ovarian or the B16 melanoma tumor models. To do so, we treated tumor-bearing mice with Maf-DKO mouse macrophages and we showed that mouse Maf-DKO macrophages inhibit initial and established tumor growth. In addition, our in vitro results demonstrated that Maf-DKO macrophages have an M1-like phenotype and are not repolarized to an M2-like phenotype by typical M2-stimuli. Importantly, tumor cells also do not repolarize the mouse Maf-DKO macrophages.

We have then generated human MAF/MAFB DKO macrophages. In vitro experiments confirm that, like mouse Maf-DKO macrophages, they have a stable M1-like phenotype and are not repolarized to an M2-like phenotype by typical M2-stimuli or tumor cells. MAF/MAFB DKO macrophages are, to the best of our knowledge, the first example of human macrophages which are resistant to tumor-induced repolarization due to a loss-of-function mutation in both alleles of a chromosomal gene. More generally this indicates that human macrophages, which are in a stable anti-tumor polarization state due to a loss-of-function mutation in both alleles of a chromosomal gene necessary for M2-induction, are useful for cell therapy, such as in tumor cell therapy.

### MOUSE MafB/c-Maf DKO MACROPHAGES

### Materials and methods

Mice: Female mice (C57BL/6 or Rag2yc knock out) aged 6 to 8 weeks, were used for all experiments. Mice were housed under specific pathogen free conditions and handled in accordance with French and European directives in the CIML (Centre d'Immunologie de Marseille Luminy).

Cell lines: The mouse ovarian cancer cell line ID8 and the B16 melanoma cell line were cultured in DMEM medium supplemented with 10% fetal calf serum (FCS), 1% penicillin and streptomycin, at 37°C under 5% CO2. Cells were passaged twice a week with trypsin.

Generation of mouse macrophages: Mouse macrophages were derived from the bone marrow of wildtype or Maf-double knockout mice and cultured for 10-12 days in DMEM medium supplemented with 10% FBS, 10-50ng/ml rMCSF or 20% M-CSF L929 cell conditioned IMDM/0.5FCS medium (LCM), 2mM glutamine, 1% sodium pyruvate, and 50ug/ml penicillin/streptomycin, at 37°C under 5% CO2. Maf-DKO primary cell line (sorted from the blood (Aziz et al., 2009)) was passaged every 4 days with partial medium change every 2 days.

Adoptive transfer of macrophages into mice bearing tumors: 6-8 weeks old C57BL/6 female mice were injected i.p. with 5×10⁶ ID8-Luc cells. Mice were either immediately treated with macrophages or tumors were allowed to establish for 14 days before the transfer of macrophages. 5×10⁶ WT or Maf-DKO macrophages were i.p. transferred into mice, transfer was repeated for four successive times in day 0, 2, 4 and 6 or day 14, 16, 18, and 20 respectively for the immediate or delayed treatment. To assess tumor progression, ID8 cells were counted by flow cytometry and luciferase activity was measured in the cell lysates, at day 27 after scarifying the mice.

1×10⁵ B16-F10 (or B16-Luc) cells in 100µl of PBS were injected i.v. into the eye. As previously mice were either treated immediately with 1×10⁶ macrophages or after tumor development for 7 days. Macrophage treatment was repeated 4 successive times. The mice were sacrificed on day14 after tumor injection and lungs and the liver were removed. The number of surface-visible metastatic colonies on the lungs and the liver were counted.

Bioluminescent imaging: Bioluminescent imaging was performed based on Berthold Night-Owl technologies. Mice were injected i.p. with luciferin solution (3 mg per mouse) and imaged until peak radiance was achieved (10 minutes). Total flux values were obtained from the anatomic region of interest. Data were presented as relative light units (RLU) of photon emission/s/mm2. Images were taken in a kinetic of tumor development from day 0 to day 27 (for ID8 model) or day 14 (for B16 model). Luciferase assay: Luciferase assay was performed using the luciferase assay system from promega (TB281). Briefly cells were washed with 1X PBS and lysed in 20µl of the provided lysis buffer. Cells were then resuspended in 100µl of luciferase assay reagent and produced light was measured by the luminometer.

Isolation of cells from the peritoneal cavity: Following sacrifice, cells from the peritoneal cavity exudate were obtained by the washing of the cavity with 10ml of PBS. Erythrocytes were removed by incubating with red blood lysis buffer for 5 minutes at RT. Cellular content of the cavity was assessed by using multi-color flow-cytometry. Gene expression of the total population was assessed by qPCR.

Flow cytometry analysis: Cell suspension was resuspended in FACS buffer (PBS1X containing 2mM EDTA and 0.5% FCS). All samples were blocked with 1:100 CD16/32 (2.4G2 BD PharMingen) before surface staining on ice with antibodies to CD45, CD11b (M1/70), F4/80, CD3e, Ly6G (1A8), NK1.1, CD19, CD8α, and MHCII. Absolute cell counting was possible by adding 20µl of calibrated suspension of microbeads to each sample before acquisition. Flow cytometry analyses and cell sorting were performed on LSRII and FACSAria (Becton Dickinson).

M1/M2 in vitro stimulation: 200000 WT BMDM or Maf-DKO macrophages were incubated in DMEM medium containing 20ng/ml of murine recombinant M-CSF. Then cells were stimulated with 100ng/ml LPS (for 8h) or 100ng/ml IFNy (for 8h) or with 20ng/ml IL-4 for 24h. Macrophage stimulation occurs in the presence or in the absence of 50% of supernatant from ID8 conditioning medium. The ID8 conditioning medium was harvested after 72h of culture. After the indicated time points the supernatant were harvested to assess cytokine production by CBA and the cells were lysed by RLT to assess gene expression by QPCR. Conditions are summarized in the table below:

| Conditions | stimuli | time of analysis |
|---|---|---|
| 1 | at ratio 0.5:0.5 ID8-SN:Medium + 20ng/ml M-CSF | 24h |
| 2 | at ratio 0.5:0.5 ID8-SN:Medium + 20ng/ml M-CSF + 20ng/ml IL-424h | |
| 3 | at ratio 0.5:0.5 ID8-SN:Medium + 20ng/ml M-CSF + 100ng/ml LPS | 8h |
| 4 | at ratio 0.5:0.5 ID8-SN:Medium + 20ng/ml M-CSF + 100ng/ml IFNg | 8h |
| 5 | Medium + 20ng/ml M-CSF | 24h |
| 6 | Medium + 20ng/ml M-CSF + 20ng/ml IL-424h | |
| 7 | Medium + 20ng/ml M-CSF + 100ng/ml LPS | 8h |
| 8 | Medium + 20ng/ml M-CSF + 100ng/ml IFNg | 8h |

RNA extraction and reverse transcription: Total peritoneal cells population, sorted CD11b+CD45+Lin-macrophages or in vitro stimulated macrophages were harvested at time points as mentioned above. Cells were washed with PBS and lysed in RLT buffer containing β-ME. RNA was extracted using Qiagen RNeasy Mini Kit (Qiagen Biotech) according to the manufacturers' instructions. RNA quantity was measured by nanodrop.

500ng of RNA from each sample was used for reverse transcription. In sterile nuclease free water, 1µl of oligodt (500µg/ml), 1µl of dNTP mix (10mM each) were added to a final volume of 2µl. This mixture was heated to 65°C to denature the RNA and quickly chilled to keep the denatured form. After a brief centrifugation, 4µl of first stranded buffer (5x), 2µl of 0.1M DTT and 1µl of RNaseOut (40Unit/µl) from Invitrogen were added. This mixture was incubated at 42°C for 2min followed by the addition of 1µl of Super-ScriptII reverse transcriptase (200 units) from Invitrogen. Then the mixture was incubated for 50min at 42°C. Reverse transcriptase was heat inactivated at 70°C for 15min.
Quantitative PCR: cDNA was diluted 1 to 10, and 2µl of diluted cDNA was used to perform quantitative PCR. SyberGreen (applied Biosciences) 2X master mix was used to perform QPCR according to manufacturers' instructions. Primers used for QPCR are listed in the table below.
CBA: Cytokine Bead Array: Cytokines were assayed using a BD Biosciences "Mouse Inflammation CBA" kit Cat No: 552364 and a Canto II according to manufacturers' instructions. Sample supernatants were analysed undiluted.
Statistical analysis: All experiments were performed in triplicate and representative data are shown. Results were tested for statistical significance using Student's t test with GraphPad prism software.

### Results

### Example 1

MafB and c-Maf double knock out macrophages inhibit initial tumor growth.

We previously showed that MafB and c-Maf double knock out (Maf-DKO) mouse macrophages could be indefinitely expanded ex vivo without loss of function or malignant transformation upon M-CSF stimulation (Aziz et al., 2009). In order to assess the role of MafB and c-Maf in the biology of TAMs, we examined development of ID8 ovarian tumor cells in mice treated with Maf-DKO macrophages. We first investigated the role of macrophages in early steps of tumor establishment.

The ID8 mouse ovarian surface epithelial cell line is frequently used as a syngeneic mouse model for human ovarian cancer. ID8 cancer cells that expressed firefly luciferase were injected i.p at day 0 into wild-type C57BL6 mice, then mice were treated with either wild-type bone marrow derived macrophages (WT) or Maf-DKO bone marrow derived macrophages (BM-DKO) or left untreated at day 0,1,2,3 (figure 1a). Tumor progression was assessed in situ by bioluminescence imaging at indicated days between day 0 and day 27 after adoptive transfer (data not shown). Untreated tumor bearing mice develop malignant ascites from day 4 to day 27 as assessed by quantification of emitted photons. In the same way, tumor bearing mice treated with WT bone marrow derived macrophages (WT-BMDM) develop ID8 tumor during the time course of the experiment. In contrast, mice treated with bone marrow derived Maf-DKO macrophages (DKO-BMDM) show a significant decrease in tumor mass 27 days after the tumor induction. We then quantified the luciferase levels in the peritoneal ascites by lysing peritoneal cells and performing a luciferase assay (figure 1b). Luciferase levels were significantly lower in the DKO-BMDM treated group (squares) compared to WT treated or untreated groups (black dots). We then investigated the absolute number of the tumor cell population in the peritoneum of tumor bearing mice. To do so, we perform a flow cytometry analysis on the peritoneal cells, as shown in figure 1c; ID8 cells were SSC high and CD45 negative (CD45-). The quantification of tumor cell number showed that there is an elevated number of ID8 cells in the peritoneum of untreated and WT treated mice, however this number was significantly decreased when mice were treated with DKO-BMDM macrophages. Thus Maf-DKO macrophages were able to reduce peritoneal tumor mass.

### Example 2

MafB and c-Maf DKO macrophages induce regression of established tumor.

To assess the role of Maf-DKO macrophages on an advanced tumor, ID8-luciferase tumor cells were injected i.p. at day 0 into C57BL6 mice (figure 2a). At day 14, these mice had malignant peritoneal ascites and established tumors, as visible by bioluminescence (data not shown). We then adoptively transferred WT-, DKO-BMDM or Blood-DKO four times at 2 days intervals (figure 2a). As earlier, tumor progression was assessed by bioluminescent imaging in situ at indicated days between day 0, and 27. The bioluminescent quantification of the emitted photons showed that the untreated tumor bearing mice have huge peritoneal malignant ascites at day 27 (data not shown). Similarly, mice treated with WT-BMDM develop a strong peritoneal tumor at day 27 (data not shown). In contrast, mice treated with DKO macrophages show a significant decrease in the bioluminescent signal and thus in the tumor mass at day 27 (data not shown). For further analysis we sacrificed the mice at day 27 and we quantified the tumor mass by a luciferase assay and by flow cytometry. Luciferase levels were high in the peritoneal cells of untreated mice and even slightly higher in WT-BMDM treated mice (figure 2 b, black dots) reflecting a high level of peritoneal tumor mass. On the contrary, mice treated with DKO macrophages showed a reduced luciferase level, which reflects a reduction of the tumor mass (figure 2 b, squares, right column). In order to confirm the reduction of the tumor cell number we analyzed the absolute number of ID8 cells in the peritoneal cavity by flow cytometry. As previously, ID8 cells were identified as SSChi CD45- cells (figure 2c). Untreated and the WT-BMDM treated mice exhibit a high proportion of ID8 cells (figure 2 c, left panels); whereas Maf-DKO treated mice show a significant reduction in ID8 cells proportion (figure 2 c, right panel). These data indicate that Maf-DKO macrophages are even able to fight and to reduce an established tumor mass.

### Example 3

Cellular composition of the peritoneal ascites.

In general, tumors consist of a wide array of immune cells that contribute to the tumor stroma of a growing malignancy (Kerkar and Restifo, 2012). In order to determine if leukocyte subsets (such T cells, NK cells etc) were recruited during tumor development, we performed a flow cytometry analysis on the peritoneal ascites of tumor bearing mice at day 27. Peritoneal wash was recovered and cells were stained with a cocktail of antibodies directed against NK cells, CD8 T cells, granulocytes and macrophages, which are the most represented cells in the tumor stroma (Kerkar and Restifo, 2012).

Initially, at 21 days post tumor initiation and using the established tumor strategy (figure 2a), besides macrophages we were able to detect NK cells and T cells, such as CD8 T cells in the peritoneal leukocyte fraction as shown in the figure 3a. Those populations of cells were not detected when mice were immediately treated with macrophages as in example 1 (data not shown). We observed almost no CD8 T cells infiltration in the peritoneum of untreated or WT-BMDM treated mice (figure 3b, dots and triangles). However, mice treated with Maf-DKO macrophages show a significant increase in the CD8 T cells population (figure 3 b, rectangles). In addition, we observed that Maf-DKO treated mice harbour more NK cells recruitment in comparison to untreated or WT-BMDM treated mice. These results suggest the possible involvement of CD8T cells and NK cells in the tumor mass reduction mediated by Maf-DKO macrophages.

We next examined the macrophages peritoneal population based on CD11b and F4/80 markers (figure 3c). We saw that the peritoneal cavity of untreated, WT-BMDM treated and Maf-DKO treated was full of macrophages, but we detected no difference in the macrophages proportion in the three groups (data not shown). Thus, based on previous published study that suggest that the level of MHCII in the macrophages population could change, being high in the tumor suppressing macrophages and low in the tumor promoting macrophages (Wang et al., 2011); we hypothesized that activation state of macrophage population might be different, which might explain our previous results concerning the reduction of the tumor mass in Maf-DKO treated mice. To verify this hypothesis, we stained peritoneal cells with different macrophage activation markers (CD80, CD86, CD69 and MHCII). We observed that all macrophages from the Maf-DKO treated mice are MHCII+ (figure 3d, rectangles), while only a small fraction of macrophages from untreated or WT-BMDM treated mice are MHCII+ (figure 3d, dots and triangles). In addition, the mean fluorescence intensity of MHCII+ in macrophages of Maf-DKO macrophage treated mice is high in comparison with MHCII+ macrophages of untreated or WT-BMDM treated mice that are MHCII low (figure 3e), suggesting that Maf-DKO macrophages are MHCII high. Our data indicate that MHCII hi TAMs are associated with tumor suppression (the case of Maf-DKO macrophages) whereas the MHCII lo macrophages are associated with tumor promotion (the case of WT macrophages).

### Example 4

MafB and c-Maf deficiency leads to classical macrophages activation in vivo

The production of proinflammatory versus anti-inflammatory cytokines by tumor infiltrating leukocytes plays a crucial role in the modulation of the immune response to a tumor (Balkwill and Mantovani, 2001). Classically activated macrophages (M1) display a cytotoxic proinflammatory phenotype (high IL-12, IL-6, IFNγ, NOS2) whereas, alternatively activated macrophages (M2) suppress immune and pro-inflammatory response by increasing the production of IL-10 or arginase (O'Shea and Murray, 2008) (Gordon, 2003) (Murray and Wynn, 2011) (Mantovani et al., 2002). To analyse the macrophage phenotype in vivo, we sorted out peritoneal macrophages (CD11b+Lin-) from tumor bearing mice (that have been treated as previously). mRNA level of NOS2, C2TA (MHC II transactivator), IL-6, IL-12, and IL-10 were measured by real-time RTPCR.

CD11b+ cells from Maf-DKO treated mice (figure 4, striped), but not WT-treated or untreated control mice (dotted and black, respectively), exhibit significantly increased NOS2, CIITA, and IL-6 mRNA level as well as IL-12 mRNA (figure 4 a-d). In contrast, CD11b+ from WT-treated mice exhibit high IL-10 expression as compared to Maf-DKO CD11b+ sorted macrophages (figure 4e). Together these results show the Maf-DKO macrophages appear to be refractory to in vivo re-education by tumor cells toward an M2 phenotype.

### Example 5

Maf-DKO macrophages show stronger classical M1 activation in vitro.

Our in vivo results on macrophages phenotype indicate that Maf-DKO macrophages are most likely to resemble M1 macrophages in tumor bearing mice. To further characterize Maf-DKO macrophage we performed an in vitro experiment, in which macrophages (WT or Maf-DKO) were stimulated either with known M1 stimuli (LPS, IFNγ) or M2 stimuli (IL-4). Cells were then lysed to measure gene expression by real-time QPCR.

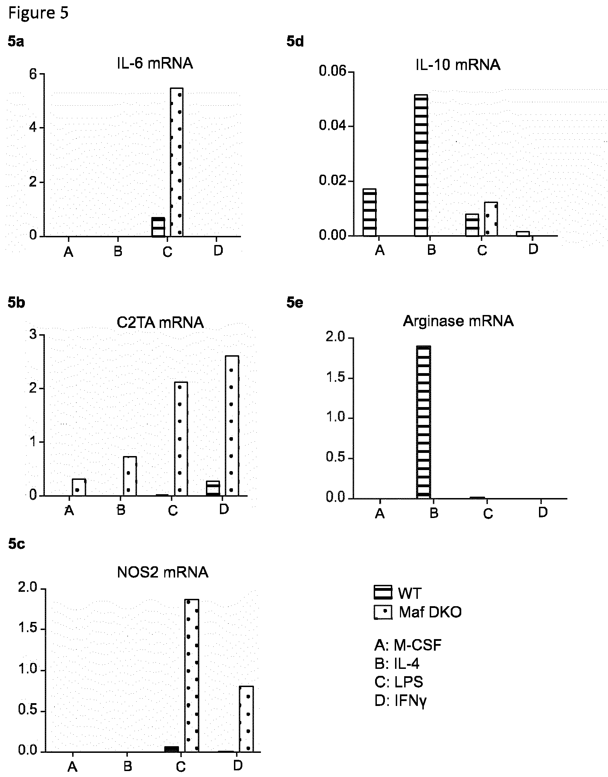

As expected, IL-6 expression was detected upon LPS stimulation (figure 5a) and IL-6 mRNA levels were significantly higher in Maf-DKO macrophages as compared to WT (figure 5a, note that in Fig.5 DKO is dotted and WT is striped). The expression of C2TA (which is a positive regulator of the MHCII gene, an M1 marker) was increased in Maf-DKO macrophages upon IFNγ, LPS and, to a lesser extent, also upon IL-4 stimulation when compared with WT macrophages (figure 5b dotted versus striped), another indication of the M1-like phenotype of Maf-DKO macrophages. NOS2 expression was as well enhanced in Maf-DKO macrophages upon LPS or IFNγ stimulation in comparison to WT macrophages (figure 5c). In contrast, when monitoring the expression of M2 genes, we observed that IL-10 (upon IL-4 and LPS stimulation) (figure 5d) and arginase (upon IL-4 stimulation) (figure 5e) were highly expressed by WT macrophages, but not in Maf-DKO macrophages. These results suggested that Maf-DKO macrophages are sensitive to M1 stimuli resembling classical activated macrophages, but much less sensitive, if at all, to M2 stimulation.

### Example 6

Maf-DKO macrophages are refractory to the re-education by tumor cells in vitro.

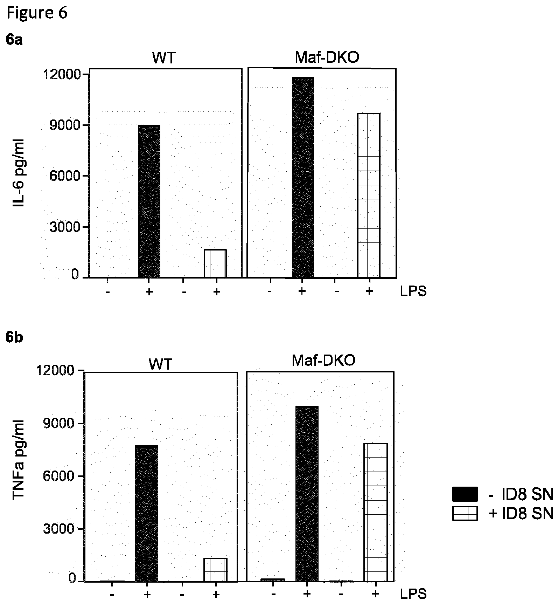

We then investigated if the Maf-DKO macrophages could be educated by the tumor in vitro. To do so, we mimicked the in vivo environment by culturing either WT or Maf-DKO macrophages in a medium containing ID8 supernatant. We checked the production of inflammatory cytokines by CBA (see Materials and methods). IL-6 was produced by both WT and Maf-DKO macrophages upon LPS stimulation (figure 6a black bars). The level of IL-6 production by WT macrophages was drastically decreased in presence of the ID8 tumor cells supernatant, while the level produced by Maf-DKO macrophages remained high at the same conditions (figure 6a, checkered bars). Similarly to IL-6, we showed that TNFα was produced by both WT and Maf-DKO macrophages in the absence of ID8-SN (figure 6b black bars). However, in the presence of ID8-SN, Maf-DKO macrophages still produced high amounts of TNFα, whereas WT macrophages showed a strong decrease of TNFα production (figure 6b, checkered bars). These results suggest that Maf-DKO macrophages are M1 like macrophages and that they are refractory and resistant to re-programming by the tumor microenvironment whereas WT macrophages can be re-programmed by tumors.

### Example 7

Maf-DKO macrophages inhibit melanoma development.

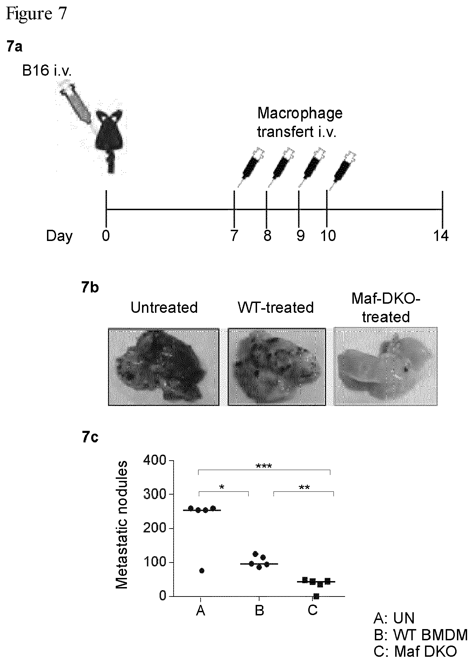

In order to address the antitumor activity of Maf-DKO macrophages in a second independent tumor model, we examined the effect of these macrophages on the development of B16 melanoma. We used B16 as an experimental metastasis model. B16 melanoma is a murine tumor cell line used for research as a model for the study of metastasis and solid tumor formation. B16 tumor cells (1×10⁵) were transferred intravenously in C57BL6 mice via retro-orbital injection, and tumors developed in the lung. Mice were then injected with Maf-DKO macrophages, WT macrophages or left non injected. Mice were only treated after the tumor establishment (figure 7a) with the different types of macrophages. At day 14, mice were sacrificed and the metastatic tumors appeared as black-pigmented colonies, of 1 to 3 mm in diameter, and had a tendency to fuse with each other. We showed that B16 tumor cells results in a huge number of tumor nodule formation in the lungs of untreated mice (figure7b, left column) as confirmed by the tumor colonies quantification (figure 7c left panel). Maf-DKO macrophages (figure 7b and c, right column) strongly reduced tumor formation in comparison to untreated control mice and had a stronger effect than WT-treated mice (figure 7b and c, center column). Thus the absence of MafB and c-Maf in macrophages resulted in a reduced pulmonary metastasis.

Altogether these data show that Maf-DKO macrophages are able to suppress tumor development regardless of tumor model, in a preventive as well as in a therapeutic setting.

### Example 8

T cells, NK cells and B cells are also involved in protection against B16 melanoma.

In order to assess the potential contribution of other immune cells such as T cells, B cells and NK cells to antitumor mechanisms, we used Rag2yc knockout mice, which are devoid of T cells, B cells and NK cells. We challenged mice with intra-venous injection of 1×10⁵ B16 tumor cells that expressed luciferase firefly. Immediately after challenge (figure 8a) or 7 days after tumor establishment (figure 8b) we treated the mice for four successive times with Maf-DKO, WT macrophages or we left them untreated. We used bioluminescent imaging as well as tumor nodules quantification to monitor tumor growth. All the mice develop tumors as evidenced by bioluminescence imaging at the day 14. We found that the tumor was localized in the lungs and the liver and the number of metastatic colonies was increased in the both organs (data shown for liver only; figure 8c to f) compared to our results on B16 development in WT mice (example 7). We showed a strong infiltration of both lungs and liver of untreated and WT treated mice with the B16 tumor cells (data shown for liver, c). Importantly, mice treated immediately with Maf-DKO macrophages demonstrated a considerable decrease in liver metastasis (figure 8 c, right panel) as compared to WT treated mice (left panel) or untreated ones (not shown). This Maf-DKO mediated tumor reduction was confirmed by metastatic nodules quantification (figure 8e, dotted column). However, only slight tumor reduction was observed in lung following the treatment with both Maf-DKO and WT macrophages in mice with an established tumor. Moreover, no liver metastasis reduction was observed in this second group of mice (figure 8 d,f), suggesting that the effector immune cells (NK, T and B cells) are involved in the Maf-DKO anti-tumor effect. These data indicate that the Maf-DKO macrophages have both, a direct effect on inhibiting tumor growth and metastasis in the early steps of tumor development, and an indirect effect for sustained tumor suppression by cooperating with other immune cells to achieve their therapeutic role described above.

### Discussion of mouse experiments

In this study we have provided evidence on the role of Maf-DKO macrophages in preventing tumor growth. We showed that transfer of Maf-DKO macrophages, but not WT macrophages, into tumor bearing mice (ID8 ovarian carcinoma or B16 melanoma) resulted in a significant reduction of both initial and established tumor mass (figures 1, 2 & 7) and enhanced survival of mice. In addition, longterm maintenance of Maf-DKO macrophages in M-CSF (expanded Maf-DKO cells obtained from blood and kept in cultivation for extended periods of time showed anti-tumor activity on par with Maf-DKOs derived from bone marrow did not reduce their anti-tumor activity.

Without wishing to be bound by any theory, the yet unknown mechanism of Maf-DKO tumor inhibition is probably both direct and indirect. The results with Rag2yc knock-out mice (that are devoid of T cells, B cells and NK cells) suggest that Maf-DKO macrophages - unlike WT macrophages - continuously cooperate with other immune cells even in the presence of a tumor in vivo in order to achieve tumor reduction. In addition, we showed that T cells, such as CD8 T cells, and to a lesser extent NK cells were recruited to the peritoneal cavity into mice bearing established tumor at an early time point after Maf-DKO adoptive transfer (figure 3a, b). Together, these results suggest that Maf-DKO macrophages may inhibit tumor growth also through T cell activation, such as CD4 T cells or CD8 T cells, or NK cells activation. Furthermore, we show that macrophages of Maf-DKO treated mice are MHC II (hi) as compared to peritoneal macrophages of WT treated mice. Importantly, it has been demonstrated that the transition from MHC II (hi) to MHC II (low) in TAMs mediates tumor progression in mice (Wang et al., 2011). Also, another study showed that CD169+ macrophages lead to anti-tumor immune response via their ability to phagocyte and to cross present tumor cell peptides to cytotoxic T cells (Asano et al., 2011). Taken together, these data and our result allow us to suggest that Maf-DKO anti-tumor activity may be triggered partially through tumor peptide presentation to T cells and their subsequent activation.

Preliminary in vitro results on the co-culture of Maf-DKO macrophages or WT macrophages with ID8 tumor cells show more lactate dehydrogenase (LDH) (data not shown) release while tumor cells are co-cultured with Maf-DKO macrophages which argued for more ID8 cells lysis in contact with Maf-DKO macrophages. This indicates that Maf-DKO macrophages also have a direct killing effect on tumor cells at least in vitro.

Previous literature has suggested that the co-culture with ID8 cells polarizes macrophages towards a M2-like phenotype by increasing IL-10 expression and decreasing IL-12 (Hagemann et al., 2006). While monitoring the profile of TAMs from Maf-DKO treated mice ex vivo, we showed that the Maf-DKO macrophages are stable M1-like macrophages in comparison to the WT macrophages., despite the exposure t a tumor environment In addition, we analyzed the Maf-DKO macrophages phenotype in vitro, by measuring IL-6, C2TA, NOS2, arginase and Il-10 mRNA expression levels. We showed that Maf-DKO macrophages are more sensitive to M1 stimuli (LPS and IFNγ), through the up-regulation of NOS2, C2TA and IL-6 and down-regulation of arginase and IL-10, whereas WT macrophages are more sensitive to M2 stimuli (IL-4) and display the opposite expression profile.

Furthermore, the in vitro treatment of Maf-DKO macrophages with tumor cells conditioned medium (ID8- SN) did not switch their phenotype from M1 to M2, further substantiating that Maf-DKO macrophages resist repolarization by the tumor secretome.

These experiments offer evidence for the role of Maf-DKO macrophages in driving an anti-tumor immune response. This opens new avenues in cancer cell therapy.

### HUMAN MAF/MAFB DKO MACROPHAGES

Methods for the generation of human induced pluripotent stem cells are well known in the art. A human iPS cell line was prepared from dermal fibroblasts obtained from the prepuce of the penis of a healthy human caucasian neonate male. Reprogramming was with non-integrating Sendai virus containing POU5F1, SOX2, KLF4 and MYC (Fusaki N., et al. (2009)). Virus screening for HIV1, HIV2, hepatitis virus B and C and mycoplasm was negative. Karyotypic showed a normal 46 (X,Y) karyotype. Into this cell line a doxycycline-inducible Cas9-expression cassette was introduced into the AAVS1 locus through TALEN-mediated gene targeting, essentially as described in Gonzalez et al. (2014), providing cell line BIHi001-A-1.

### Example 9

### Generation of double knock-out (DKO) cells from human iPS cells

### a) iPSC culture

iPSCs were cultured under feeder-free conditions in StemFlex Medium (Thermo Fisher, #A3349401) on vitronectin-coated (Thermo Fisher, # A14700) tissue-culture plates at 37°C, 5% CO2 under normoxic conditions. Cells were passaged with ReLeSR (Stem Cell Technologies, #05872) when reaching 60-80% confluency and maintained in the presence of 10 µM of the ROCK inhibitor Y-27632 (biomol, #Cay10005583) on the day after passaging. Single-cell suspensions of iPSC were obtained by incubation with Accutase (Merck Millipore, #SF006).

### b) Genome editing

We used the CRISPR/Cas9 system to delete the coding sequence of MAF (Gene ID: 4094, 373 amino acids) and MAFB (Gene ID: 9935, 323 amino acids) in human cells. Towards this end, we transfected a healthy-donor-derived iPSC line harbouring a doxycycline-inducible Cas9 expression cassette integrated into the AAVS1 locus (BIHi001-A-1, https://hpscreg.eu/cell-line/BIHi001-A-1; also called iBCRT Cas9v1-3G-Kl.16) with sgRNA-expressing plasmids targeting MAF and MAFB (pU6-(Bbsl)sgRNA_CAG-venus-bpA, Addgene ID 86985, https://www.addgene.org/86985/) according to a published protocol (Yumlu, (2017)).

To delete the complete CDS, we designed 2 sgRNAs per gene to target a sequence in the 5'UTR at least 20 nt upstream of the start codon and in the 3'UTR at least 20 nt downstream of the stop codon of each gene using the CrispRGold program for sequence design (https://crisprgold.mdc-berlin.de/ Chu et al. (2016)). The 2 sgRNAs for each gene were expressed together from the sgRNA-expression vector pU6-(Bbsl)sgRNA_CAG-venus-bpA, which also encodes a fluorescent reporter gene (Venus, derived from YFP) for positive selection of transfected cells by FACS.

**Table 1: sgRNA and protospacer sequences for CRISPR/Cas9-mediated deletion of MAF and MAFB CDS. The position of the target-specific, 20-nucleotide long protospacer is marked by "N" within the sgRNA sequence. The genomic target sequences of the sgRNAs expressed from pU6-(Bbsl)sgRNA_CAG-venus-bpA with the indicated protospacer sequences are located in the 5'UTR and 3'UTR, respectively, of MAF and MAFB. UTR, untranslated region.**

| **sgRNA** | | |
|---|---|---|
| NNNNNNNNNNNNNNNNNNNNGTTTTAGAGCTAGAAATAGCAAGTTAAAATAAGGCTAGTCCGTT ATCAACTTGAAAAAGTGGCACCGAGTCGGTGCTTT | | |
| | | |

| **Locus** | **Protospacer sequence for 5'UTR** | **Protospacer sequence for 3'UTR** |
|---|---|---|
| MAF | ATCTGGCGGAGCGGCGGCGG | ACCCTGATAAGTGCTCCGCG |
| MAFB | CGGCCGCAAAGTTTCCCGGG | TGACCTGTTTGACTTGAGCG |

SEQ ID No.1 (MAF-5'UTR): 
5'ATCTGGCGGAGCGGCGGCGGGTTTTAGAGCTAGAAATAGCAAGTTAAAATAAGGCTAGTCCGTTA
TCAACTTGAAAAAGTGGCACCGAGTCGGTGCTTT-3'
SEQ ID No.2 (MAF-3'UTR): 
5'ACCCTGATAAGTGCTCCGCGGTTTTAGAGCTAGAAATAGCAAGTTAAAATAAGGCTAGTCCGTTA
TCAACTTGAAAAAGTGGCACCGAGTCGGTGCTTT-3'
SEQ ID No.3 (MAFB-5'UTR): 
5'CGGCCGCAAAGTTTCCCGGGGTTTTAGAGCTAGAAATAGCAAGTTAAAATAAGGCTAGTCCGTTA
TCAACTTGAAAAAGTGGCACCGAGTCGGTGCTTT-3'
SEQ ID No.4 (MAFB-3'UTR):

5'TGACCTGTTTGACTTGAGCGGTTTTAGAGCTAGAAATAGCAAGTTAAAATAAGGCTAGTCCGTTA
TCAACTTGAAAAAGTGGCACCGAGTCGGTGCTTT-3'

Using Lipofectamine 3000 (Thermo Fisher, #L3000001) we transfected BIHi001-A-1 with sgRNA expression vectors together with pCAG-mTrex2-bpA (Addgene ID 86984, https://www.addgene.org/86984/) to enhance the propensity of indel formation. Control cells, labelled with "WT" or "wild-type" throughout the text, were treated the same way except for using the pU6-(Bbsl)sgRNA_CAG-venus-bpA without inserting any protospacer sequence. Transfected BIHi001-A-1 cells were treated with 1 µg/ml doxycycline hyclate (Sigma-Aldrich, #D9891) to induce Cas9 expression, followed by FACS-isolation of cells expressing the Venus reporter gene from the sgRNA-carrying vector. Sorted cells were seeded at low density to allow the isolation of single-cell-derived colonies. In the first round of transfection, sgRNAs targeting MAF were used and colonies were screened for full-length deletion of the MAF CDS using PCR and Sanger sequencing of the edited locus (figure 9). Cells were cultured under conditions for iPS cells, as described above.

MAF KO iPSC were subjected to a second round of transfection using MAFB-targeting sgRNAs, followed by PCR and sequencing analysis of the obtained colonies. Three MAF/MAFB DKO (MAF-DKO) iPSC clones were isolated (clone 1, 2, 3). The genomic target region and the edited MAF and MAFB loci are shown in Figure 10.

### Example 10

### Directed differentiation of iPSC into macrophages

Directed differentiation of iPSC into macrophages was based on previously published protocols (Buchrieser 2017). Embryoid bodies (EBs) were formed by seeding a single-cell suspension of wild-type or MAF-DKO iPSC at a density of 12,500 cells/well into an ultra-low attachment U-shaped-bottom 96-well plate (Nunclon Sphera, Thermo Fisher, #174925), resuspended in EB medium consisting of StemFlex Medium (Thermo Fisher, #A3349401) supplemented with 50 ng/ml BMP-4 (R&D Systems, #314-BP), 20 ng/ml SCF (R&D systems, 255-SC), 50 ng/ml VEGF (Peprotech, AF-100-20A) and 10 µM Y-27632. The 96-well plate was centrifuged for 3 minutes at 100 g to collect the cells at the bottom, and placed at 37°C, 5% CO2 for 4 days. After 1 and 2 days, half of the EB medium was replaced with freshly prepared EB medium.

Wild-type and MAF-DKO EBs were indistinguishable by size or morphology.

After 4 days, EBs were selected manually, resuspended in EB differentiation medium consisting of X-VIVO 15 (Lonza, #BE02-060F) supplemented with 2 mM GlutaMAX (Thermo Fisher, #35050038), 0.055 mM 2-mercaptoethanol (Sigma-Aldrich, #M6250), 100 ng/ml human M-CSF (Thermo Fisher, #PHC9504), 25 ng/ml human IL-3 (R&D systems, #203-IL), and seeded into ultra-low attachment 6-well tissue-culture plates (8 EBs/well) or 90 mm tissue-culture dishes (24 EBs/dish; Nunclon Sphera, Thermo Fisher, #174932 and #174945). Two thirds of the culture medium were exchanged with fresh differentiation medium every 5 days. Production of monocytes/macrophages from EBs started around day 15 post EB seeding, and suspension cells were harvested, counted and used for immunophenotyping by flow cytometry or EdU incorporation assays.

For further experiments the cells harvested from the supernatant of the EB cultures were replated for final macrophage differentiation in RPMI supplemented with 10% FBS (PAA-GE Healthcare, A15-101), supplemented with 100 units/ml penicillin, 100 ug/ml streptomycin (Thermo Fisher, #15140122), 2 mM GlutaMAX (Thermo Fisher, #35050038), 1 mM sodium pyruvate (Thermo Fisher, #11360-039), 50 ng/ml M-CSF (Thermo Fisher, #PHC9504), seeded into ultra-low attachment 12-well tissue-culture plates (Nunclon Sphera, Thermo Fisher #174931, #174932) and 50 ng/ml GM-CSF (Peprotech, #300-03); 37°C, 5% CO2. Cells were counted manually using a Neubauer hemocytometer or automatically with a CASY cell counter (OMNI Life Science).

Deletion of MAF and MAFB had no effect on myeloid differentiation capacity, and, similar to wild-type EBs, MAF-DKO EBs started releasing monocytes/macrophages into the EB differentiation medium around day 15 post EB seeding. MAF-DKO suspension cells were viable, and after plating in differentiation medium containing 50 ng/ml M-CSF and GM-CSF, both wild-type and MAF-DKO macrophages phagocytosed beads, produced ROS, and stained positive for lysosomes and cathepsin activity, indicative of a mature macrophage phenotype.

### Example 11

iPS-cell derived MAF-DKO macrophages and wt macrophages obtained from EB-differentiation cultures essentially as described in example 11 were harvested on day 20 post EB-seeding by collecting the cells in suspension. 500000 cells per well were replated in 6 well plates (3ml medium per well) and then kept for 5 days for final differentiation in the presence of M-CSF and GM-CSF (essentially as described in example 11; partial medium change for fresh medium every second day). On day 5, cells were collected and replated in complete medium (incl. M-CSF and GM-CSF, as described in example 11) in Nunclon 12-well plates at 100000 cells per well, and kept for two more days (2ml medium per well). On day 7 after harvest of the suspension cells the medium was changed for fresh complete medium (incl. M-CSF and GM-CSF, both at 50 ng/ml). Cells were then harvested 2 hours after medium change - 10000 DAPI-negative, CD45 positive, C11b positive cells were sorted directly into LRT plus lysis buffer and RNA was extracted with the RNeasy micro kit (Quiagen, Cat. No. 74034). Differences in gene expression patterns were then analyzed by RNAseq, as described in detail in Picelli et al. (2013) Nature Methods 10: 1096-1098 with RNA isolated from 10000 cells, sequencing depth of 38 million fragments per library. Alignment of the fragments to the human reference (hg38) was done with GSNAP (v2020-12-16; [Thomas D. et al. (2005) Bioinformatics 21:1859-1875], [Thomas D. et al. (2010) Bioinformatics 26:873-881]) and Ensembl gene annotation 98 ([Howe et al. (2021) NAR vol. 49(1):884-891]) was used to detect splice sites. The uniquely aligned fragments were counted with featureCounts (2.0.1; [Liao et al. (2014) "featureCounts: an efficient general purpose program for assigning sequence reads to genomic features."]) and the support of the same Ensembl annotation. Simultaneously,

sequenced libraries underwent a quality control with RNA-SeQC (1.1.8; [DeLuca et al. (2012) Bioinformatics 28.11: 1530-1532.]) which includes exonic, intronic and intergenic distribution of the reads and rRNA rate within each library. Normalization of the raw fragments counts based on the library size and testing for differential expression between the two conditions DKO 2h and WT 2h was performed with the DESeq2 (v1.24.0; [Love et al. (2014) Genome Biology, 15, 550]) and IHW (1.12.0; [Ignatiadis et al. (2016) Nature Methods. doi: 10.1038/nmeth.3885; and Ignatiadis N, Huber W (2017). "Covariate-powered weighted multiple testing with false discovery rate control." arXiv. doi: arXiv:1701.05179]) package in R (3.6.3; [R Core Team (2020). R: A language and environment for statistical computing. R Foundation for Statistical Computing, Vienna, Austria. URL https://www.R-project.org/]). Genes, which have an adjusted p value (padj) < 0.05 and/or log2FoldChange > 0.58 or < -0.58 were considered as differentially expressed.

The differences in the gene expression patterns between MAF-DKO macrophages and wt-macrophages are dramatic, as visualized in the volcano plot shown as figure 11. In comparison to wt macrophages the indicated M1-markers were strongly upregulated in DKO-macrophages, as shown in the below table, while the indicated M2-markers were strongly downregulated, even though M-CSF was present at a concentration of 50ng/ml in the final differentiation step. This shows that the human MAF-DKO macrophages have an essentially identical polarization phenotype as the mouse MAF-DKO macrophages and are therefore expected to be essentially as anti-tumorigenic as the mouse MAF-DKO macrophages.

| Gene | M1 marker | M2 marker | Upregulation in DKO vs. wt | Downregulation in DKO vs. wt |
|---|---|---|---|---|
| HLA-DPA1 | + | | >30-fold | |
| HLA-DPB1 | + | | >30-fold | |
| HLA-DPB2 | + | | >30-fold | |
| HLA-DQB1 | + | | >30-fold | |
| HLA-DQA1 | + | | >30-fold | |
| HLA-DRA | + | | >30-fold | |
| HLA-DRB5 | + | | >30-fold | |
| HLA-DOA | + | | >30-fold | |
| RXFP2 | + | | >30-fold | |
| CD74 | + | | >20-fold | |
| CD38 | + | | >15-fold | |
| IL18 | + | | >5-fold | |
| RNASE1 | | + | | >100-fold |
| LYVE1 | | + | | >100-fold |
| FCGBP | | + | | >100-fold |
| CD28 | | + | | >100-fold |
| F13A1 | | + | | >100-fold |
| QPCT | | | | >100-fold |
| CCL7 | | | | >50-fold |
| RNF128 | | | | >50-fold |

## Claims

1. An allogeneic human myeloid cell for use as therapeutic in a human patient, wherein the allogeneic myeloid cell comprises at least one MHC-II complex which is identical with any one MHC-II complex of said human patient.

2. The allogeneic human myeloid cell according to claim 1 for use in the treatment of cancer.

3. The allogeneic human myeloid cell for use according to any one of claims 1 or 2, wherein the myeloid cell is a macrophage or a monocyte.

4. The allogeneic human myeloid cell for use in the treatment of cancer according to claims 2 or 3, wherein the allogeneic myeloid cell comprises at least one protein complex selected from the group consisting of HLA-DR, HLA-DP and HLA-DQ, which protein complex is identical with any corresponding HLA complex of said human patient.

5. The allogeneic human macrophage for use in the treatment of cancer according to claim 2, wherein the allogeneic macrophage comprises two protein complexes selected from said group, preferably wherein the allogeneic macrophages comprises three complexes selected from said group.

6. The allogeneic human macrophage for use in the treatment of cancer according to any one of claims 1 to 3, wherein the allogeneic macrophage comprises at least one HLA-DR protein complex which is identical with any corresponding HLA-DR complex of said human patient and/or at least one HLA-DP protein complex which is identical with any corresponding HLA-DP complex of said human patient and/or at least one HLA-DQ protein complex which is identical with any corresponding HLA-DQ complex of said human patient.

7. An allogeneic human macrophage for use in the treatment of cancer in a patient, wherein the allogeneic macrophage and the patient to be treated have at least one pair of alleles in common selected from the group consisting of the following pairs: HLA-DRA / HLA-DRB1, HLA-DRA/ HLA-DRB3, HLA-DRA/ HLA-DRB4, HLA-DRA/ HLA-DRB5, HLA-DPA1/ HLA-DPB1 and HLA-DQA1/HLA-DQB1.

8. The allogeneic human macrophage for use in the treatment of cancer according to claim 7, wherein the allogeneic macrophage and the patient to be treated have two, three, four, five or even all six pairs of alleles in common.

9. The allogeneic human macrophage for use in the treatment of cancer according to any one of claims 7 to 8, further comprising the feature(s) of any one of claims 1 to 7.

10. The allogeneic human macrophage for use in the treatment of cancer according to any one of claims 1 to 9, wherein the human macrophage is resistant to M-CSF induced M2-polarization.

11. The allogeneic human macrophage for use in the treatment of cancer according to any one of claims 1 to 10, wherein the human macrophage is genetically modified.

12. The allogeneic human macrophage for use in the treatment of cancer according to claim 11, wherein the genetic modification is introduction of a gene encoding a surface protein, such as a chimeric antigen receptor.

13. The allogeneic human macrophage for use in the treatment of cancer according to any one of claims 1 to 12, wherein the human macrophage is derived from an iPS cell.

14. A collection of allogeneic human macrophages according to any one of claims 1 to 13, wherein the collection of human macrophages is capable of recruiting T cells and/or NK cells to a tumor in vivo.

15. The collection of human macrophages according to claim 14, wherein the number of human macrophages is at least 10000000.
